(19) Europäisches Patentamt
European Patent Office
Office européen des brevets

(11) EP 4 061 324 B1

(12) **EUROPEAN PATENT SPECIFICATION**

(45) Date of publication and mention of the grant of the patent:
**17.06.2026 Bulletin 2026/25**

(21) Application number: **20807427.8**

(22) Date of filing: **19.11.2020**

(51) International Patent Classification (IPC):
***A61K 8/81*** *(2006.01)* ***A61K 8/84*** *(2006.01)*
***A61K 8/87*** *(2006.01)* ***A61K 8/891*** *(2006.01)*
***A61K 8/898*** *(2006.01)* ***A61Q 5/06*** *(2006.01)*

(52) Cooperative Patent Classification (CPC):
**A61K 8/84; A61K 8/8152; A61K 8/87; A61K 8/891; A61K 8/898; A61Q 5/065;** A61K 2800/882; A61K 2800/884; A61K 2800/95

(86) International application number:
**PCT/EP2020/082775**

(87) International publication number:
**WO 2021/099515 (27.05.2021 Gazette 2021/21)**

(54) **PROCESS FOR TREATING KERATIN FIBERS, COMPRISING A (POLY)CARBODIIMIDE COMPOUND, AN AQUEOUS DISPERSION OF PARTICLES OF POLYMER(S) AND A COLORING AGENT**

VERFAHREN ZUM BEHANDELN VON KERATINFASERN, DIE EINE (POLY) CARBODIIMID-VERBINDUNG, EINE WÄSSRIGE DISPERSION VON POLYMERTEILCHEN UND EIN FÄRBEMITTEL UMFASSEN

PROCÉDÉ DE TRAITEMENT DE FIBRES DE KÉRATINE, COMPRENANT UN (POLY) CARBODIIMIDE, UNE DISPERSION AQUEUSE DE PARTICULES DE POLYMÈRE(S) ET UN AGENT COLORANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.11.2019 FR 1913057**

(43) Date of publication of application:
**28.09.2022 Bulletin 2022/39**

(73) Proprietor: **L'OREAL**
**75008 Paris (FR)**

(72) Inventors:
- **LIARD, Alexis**
**93400 SAINT-OUEN (FR)**
- **GRACIA, Marie**
**93400 Saint-Ouen (FR)**

(74) Representative: **Casalonga, Axel**
**Casalonga & Partners**
**Bayerstrasse 71-73**
**80335 München (DE)**

(56) References cited:
**WO-A1-2016/084971 WO-A1-2018/079774**
**WO-A1-2019/211050 US-A1- 2018 371 237**

- **A. J. DERKSEN: "Polycarbodiimides as classification-free and easy to use crosslinkers for water-based coatings", 8 July 2017 (2017-07-08), XP055719203, Retrieved from the Internet <URL:https://web.archive.org/web/20170708064027/https://www.pcimag.com/ext/resources/WhitePapers/2017/Stahl-Polymers-White-paper-PolyCarbodiimide-Crosslinkers.pdf> [retrieved on 20200730]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 061 324 B1

## Description

The present invention relates to a process for treating keratin fibers, comprising the application to the keratin fibers of a composition (C) comprising at least one (poly)carbodiimide compound, at least one aqueous dispersion of particles of polymer(s) chosen from polyurethanes, acrylic polymers, and mixtures thereof, at least one silicone, and at least one coloring agent chosen from pigments, direct dyes, and mixtures thereof.
The present invention also relates to a device for treating keratin fibers. References to keratin fibers are to be understood as meaning hair.

## Technical field

In the field of dyeing keratin fibers, in particular human keratin fibers, it is already known practice to dye keratin fibers via various techniques using direct dyes or pigments for non-permanent dyeing, or dye precursors for permanent dyeing.
There are essentially three types of process for dyeing the hair:

a) "permanent" dyeing, the function of which is to afford a substantial modification to the natural color and which uses oxidation dyes which penetrate into the hair fiber and forms the dye via an oxidative condensation process;
b) non-permanent, semi-permanent or direct dyeing, which does not use the oxidative condensation process and withstands four or five shampoo washes; it consists in dyeing keratin fibers with dye compositions containing direct dyes.
c) temporary dyeing, which gives rise to a modification of the natural color of the hair that remains from one shampoo washing to the next, and which serves to enhance or correct a shade that has already been obtained. It may also be likened to a "makeup" process.

For this last type of dyeing, it is known practice to use colored polymers formed by grafting one or more dyes of azo, triphenylmethane, azine, indoamine or anthraquinone nature onto a polymer chain. These colored polymers are not entirely satisfactory, notably as regards the homogeneity of the coloring obtained and its resistance, not to mention the problems associated with their manufacture and notably with their reproducibility.
Another dyeing method consists in using pigments. Specifically, the use of pigment on the surface of keratin fibers generally makes it possible to obtain visible colorings on dark hair, since the surface pigment masks the natural color of the fiber. However, the colorings obtained via this dyeing method have the drawback of having poor resistance to shampoo washing and also to external agents such as sebum, perspiration, blow-drying and/or rubbing.
In addition, compositions for temporarily dyeing the hair may also lead to a hair feel that is uncosmetic and/or not natural; the hair thus dyed may notably lack softness and/or suppleness and/or strand separation.
In addition, there are no effective makeup-removing compositions for removing this type of temporary dye composition when it is persistent with respect to shampoo washing.
Application WO 2019/211050 A1 relates to a multicomponent composition for coloring hair. The first component includes an organic polymer, the second component includes an in situ linking material and the third component includes a base compound, wherein one or more of the components includes pigment microparticles. An optional fourth component may include a catalyst. Formulation #16 in Example 1 comprises a hydrofunctional polyacrylic dispersion, a carbodiimide and pigments.
The need remains for a process for treating keratin fibers, notably the hair, which has the advantage of obtaining a homogeneous and smooth colored coating on the hair, and also hair with complete strand separation, while at the same time forming a coating that is persistent with respect to shampoo washing and to the various attacking factors to which the hair may be subjected, such as blow-drying and/or rubbing, without degrading the hair. There is also a need to be able to eliminate this colored coating when so desired.
Thus, the aim of the present invention is to develop a process for treating keratin fibers, notably the hair, which has the advantage of obtaining a homogeneous and smooth colored coating on the hair, and also hair with complete strand separation, while at the same time forming a coating that is persistent with respect to shampoo washing and to the various attacking factors to which the hair may be subjected, such as blow-drying and/or rubbing, without degrading the hair. Advantageously, the colored coating can be readily eliminated when so desired.

## Disclosure of the invention

One subject of the present invention is thus a process for treating keratin fibers, comprising the application to the keratin fibers of a composition (C) comprising:

a) at least one (poly)carbodiimide compound,
b) at least one aqueous dispersions of particles of polymer(s) chosen from polyurethanes, acrylic polymers, and mixtures thereof,
c) at least one silicone, and
d) at least one coloring agent chosen from pigments, direct dyes and mixtures thereof.

The present invention also relates to a device for treating keratin fibers, comprising one or more compartments containing:

- in a first compartment (E1), a composition (C) as defined previously; and
- optionally, in a second compartment (E2), a composition (D) as defined below.

In one variant of the invention, composition (C) is obtained by extemporaneous mixing at the time of use of at least one composition (C1) comprising a) at least one (poly)carbodiimide compound, at least one composition (C2) comprising b) at least one aqueous dispersion of particles of polymer(s) chosen from polyurethanes, acrylic polymers and mixtures thereof, one and/or the other of the compositions (C1) and (C2) comprising c) at least one silicone, and d) at least one coloring agent chosen from pigments, direct dyes, and mixtures thereof.

Thus, the present invention also relates to a device for treating keratin fibers, comprising several compartments containing:

- in a first compartment (F1), a composition (C1) comprising a) at least one (poly)carbodiimide compound;
- in a second compartment (F2), a composition (C2) comprising b) at least one aqueous dispersion of particles of polymer(s) chosen from polyurethanes, acrylic polymers and mixtures thereof, one and/or the other of the compositions (C1) and (C2) comprising c) at least one silicone, and d) at least one coloring agent chosen from pigments, direct dyes, and mixtures thereof; and
- optionally, in a third compartment (F3), a composition (D) as defined below.

Through the use of this treatment process on keratin fibers, colored coatings are obtained on the hair that make it possible to obtain a coloring that is visible on all types of hair in a manner that is persistent with respect to shampoo washing, while at the same time preserving the physical qualities of the keratin fibers. Such a coating may be resistant to the external attacking factors to which the hair may be subjected, such as blow-drying and perspiration. It makes it possible in particular to obtain a smooth and uniform deposit.

Moreover, this process makes it possible to obtain hair with perfect strand separation, which can be styled without problem and which has good cosmetic properties, notably in terms of softness and feel.

Advantageously, the colored coating thus obtained can be readily eliminated by means of a makeup-removing composition.

The term *"hair with strand separation"* means hair which, after application of the composition (C) and drying, is not stuck together (or of which all the strands are separated from each other) and thus does not form clumps of hair.

For the purposes of the present invention, the term *"coloring that is persistent with respect to shampoo washing"* means that the coloring obtained persists after one shampoo wash, preferably after three shampoo washes, more preferentially after five shampoo washes.

The term "*keratin fibers*" particularly means human keratin fibers such as head hair, eyelashes, eyebrows, and bodily hair, preferentially head hair, eyebrows and eyelashes, even more preferentially head hair.

The term "*at least one*" means one or more.

The invention is not limited to the illustrated examples. The features of the various examples may notably be combined within variants which are not illustrated.

For the purposes of the invention and unless otherwise indicated:

- an "*alkyl*" radical denotes a saturated linear or saturated branched hydrocarbon-based radical containing from 1 to 24 carbon atoms, particularly from 1 to 20 carbon atoms, more particularly from 1 to 12 carbon atoms, preferably of $C_1$-$C_6$, even more preferentially of $C_2$-$C_4$. For example, the alkyl group represents a methyl, an ethyl, a propyl, an isopropyl, a butyl, an isobutyl, a tert-butyl, a pentyl, an isopentyl, a hexyl, an isohexyl, a heptyl, an octyl, a nonyl or a decyl; preferably methyl, ethyl or propyl;
- an "*aminoalkyl*" radical denotes an alkyl radical as defined previously, said alkyl radical comprising an $NH_2$ group;
- a "*hydroxyalkyl*" radical denotes an alkyl radical as defined previously, said alkyl radical comprising an OH group;
- an "*alkylene*" radical denotes a divalent alkyl group with "*alkyl*" as defined previously, preferentially of $C_2$-$C_4$, linear or branched, such as methylene, ethylene or propylene;
- a "*cycloalkyl*" or "*alicycloalkyl*" radical denotes a cyclic saturated monocyclic or bicyclic, preferably monocyclic,

hydrocarbon-based group comprising from 1 to 3 rings, preferably 2 rings, and comprising from 3 to 24 carbon atoms, in particular comprising from 3 to 20 carbon atoms, more particularly from 3 to 13 carbon atoms, even more particularly from 3 to 12 carbon atoms, preferably between 5 and 10 carbon atoms, such as cyclopropyl, cyclopentyl, cyclohexyl, cycloheptyl or norbornyl, in particular cyclopropyl, cyclopentyl or cyclohexyl, it being understood that the cycloalkyl radical may be substituted with one or more $(C_1-C_4)$alkyl groups such as methyl; preferably, the cycloalkyl group is then an isobornyl group,

- a "*cycloalkylene*" radical denotes a divalent cycloalkyl group with "*cycloalkyl*" as defined previously, preferably of $C_3-C_{12}$;
- an "*aryl*" radical is a monocyclic, bicyclic or tricyclic, fused or non-fused, unsaturated and aromatic hydrocarbon-based cyclic radical, comprising from 6 to 14 carbon atoms, preferably between 6 and 12 carbon atoms; preferably, the aryl group comprises 1 ring of 6 carbon atoms such as phenyl, naphthyl, anthryl, phenanthryl and by phenyl, it being understood that the aryl radical may be substituted with one or more $(C_1-C_4)$alkyl groups such as methyl, preferably tolyl, xylyl, or methylnaphthyl; preferably, the aryl group represents phenyl;
- an "*arylene*" radical is a divalent aryl radical with "*aryl*" as defined previously; preferably, arylene represents phenylene;
- a "*heterocyclic*" radical denotes a saturated or unsaturated, non-aromatic or aromatic, monocyclic or polycyclic hydrocarbon-based radical, comprising one or more heteroatoms, preferably from 1 to 5 atoms chosen from O, S or N, including from 3 to 20 ring members, preferably between 5 and 10 ring members, such as imidazolyl, pyrrolyl and furanyl;
- a "*heterocycloalkylene*" radical is a divalent heterocyclic group with "*heterocyclic*" as defined previously;
- an "*aryloxy*" radical denotes an aryl-oxy or aryl-O radical with "*aryl*" as defined previously;
- an "*alkoxy*" radical denotes an alkyl-oxy or alkyl-O- radical with "*alkyl*" as defined previously;
- an "*acyloxy*" radical denotes an ester radical R-C(O)-O- with R being an alkyl group as defined previously.

The process for treating keratin fibers according to the invention is a process for dyeing keratin fibers such as the hair.

**(Poly)carbodiimide compound:**

Composition (C) according to the invention comprises at least one (poly)carbodiimide compound.
The composition may comprise at least two different (poly)carbodiimide compounds, present as a mixture in the composition.
The term "*(poly)carbodiimide compound"* means a compound comprising one or more carbodiimide groups, preferably at least two carbodiimide groups, more preferentially at least three carbodiimide groups; in particular, the number of carbodiimide groups does not exceed 200, preferably 150, more preferentially 100.
The term "*carbodiimide group"* means a divalent linear triatomic fraction of general formula -(N=C=N)-.
According to a particular embodiment, the (poly)carbodiimide compound is chosen from the compounds of formula (I) below:

[Chem. 1]

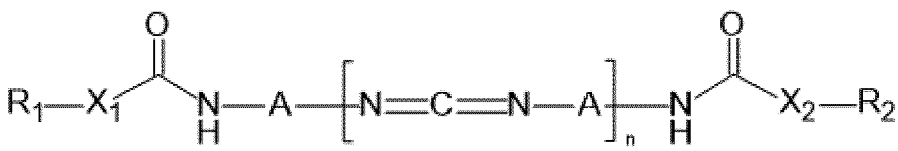

(I)

in which formula (I):

- **$X_1$** and **$X_2$** independently represent an oxygen atom O, a sulfur atom S or an NH group,
- **$R_1$** and **$R_2$** independently represent a hydrocarbon-based radical, preferably alkyl, optionally interrupted with one or more heteroatoms,
- **n** denotes an integer ranging from 1 to 100, and
- **A** is a monomer chosen from the compounds below:

[Chem. 2]

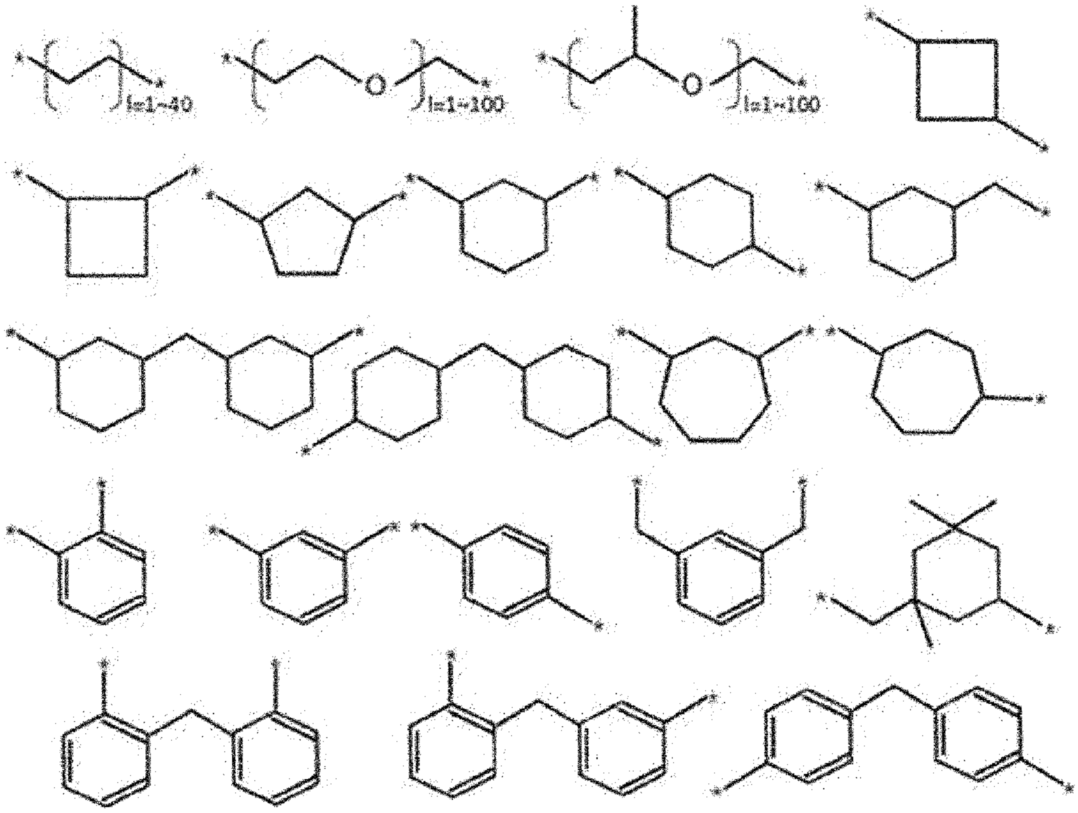

Preferably, the (poly)carbodiimide compound is chosen from the compounds of formula (II) below:

[Chem. 3]

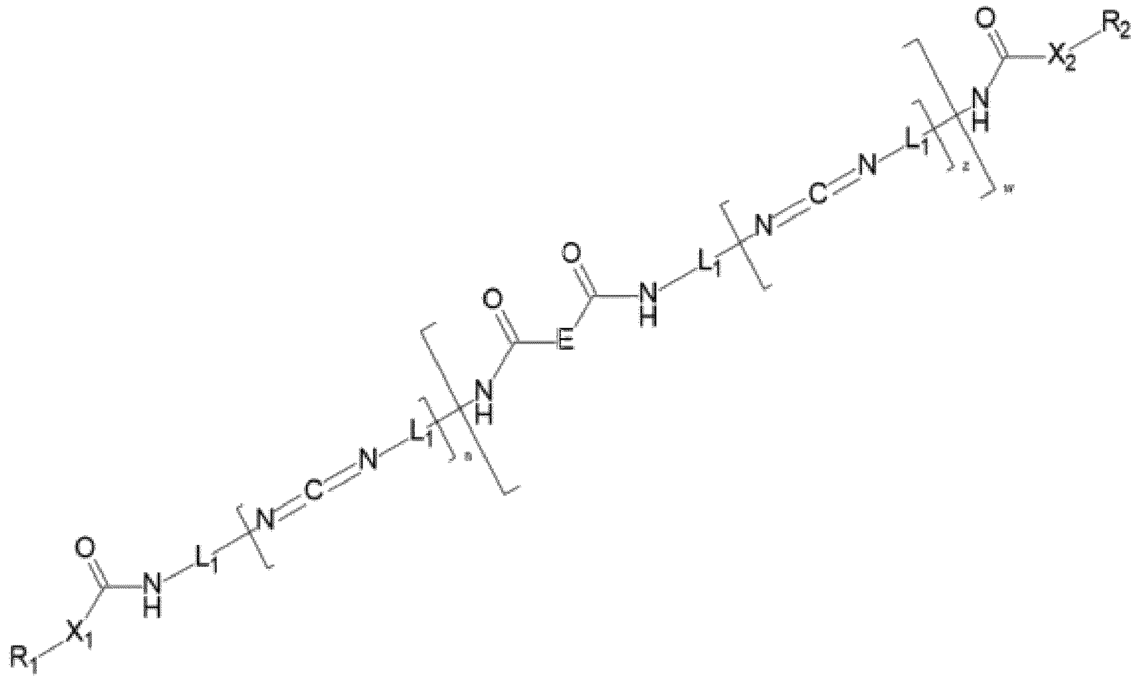

(II)

in which formula (II):

- $\mathbf{X_1}$ and $\mathbf{X_2}$ independently represent an oxygen atom O, a sulfur atom S or an NH group;
- $\mathbf{R_1}$ and $\mathbf{R_2}$ independently represent a hydrocarbon-based radical, optionally interrupted with one or more heteroatoms,
- **n and z** denote an integer ranging from 1 to 20, with $n+z \geq 2$ and **w** denotes an integer ranging from 1 to 3;
- $\mathbf{L_1}$ independently represents a $C_1$-$C_{18}$ divalent aliphatic hydrocarbon-based radical, a $C_3$-$C_{15}$ cycloalkylene radical, a $C_3$-$C_{12}$ heterocycloalkylene group or a $C_6$-$C_{14}$ arylene group, and mixtures thereof;
- **E** independently represents a group chosen from:

  - $-O-R_3-O-$; $-S-R_4-S-$; $-R_5-N(R_6)-R_4-N(R_6)-R_5-$,

  in which $\mathbf{R_3}$ and $\mathbf{R_4}$ independently represent a divalent hydrocarbon-based radical optionally interrupted with one or more heteroatoms,
- $\mathbf{R_5}$ independently represents a covalent bond or a saturated divalent hydrocarbon-based radical, optionally interrupted with one or more heteroatoms;
- $\mathbf{R_6}$ independently represents a hydrogen atom or a hydrocarbon-based radical, optionally interrupted with one or more heteroatoms.

The term *"hydrocarbon-based radical"* means a saturated or unsaturated, linear or branched radical containing from 1 to 300 carbon atoms, preferably from 1 to 250 carbon atoms, more preferentially from 1 to 200 carbon atoms. Preferably, the hydrocarbon-based radical is a saturated linear radical.

The hydrocarbon-based radical may comprise one or more cyclic groups.

The hydrocarbon-based radical may be interrupted with one or more heteroatoms, in particular chosen from O, S or N and/or substituted with one or more cations, anions or zwitterions or cationic groups such as ammonium, anionic groups such as carboxylate, or zwitterionic groups, and/or comprising a metal ion which may be incorporated in the form of a salt.

The term "heteroatom(s)" means an oxygen O, sulfur S or nitrogen N atom, and also halogen atoms such as Cl, F, Br and I. If the heteroatom is included in the chain of the hydrocarbon-based radical, the heteroatom is preferably chosen from oxygen O, sulfur S or nitrogen N atoms.

Preferably, $\mathbf{X_1}$ and $\mathbf{X_2}$ independently represent an oxygen atom.

Preferably, $\mathbf{R_1}$ and $\mathbf{R_2}$ are independently chosen from dialkylamino alcohols, alkyl esters of hydroxycarboxylic acid and monoalkyl ethers of (poly)alkylene glycol, in which a hydroxyl group has been removed, and mixtures thereof.

In a preferred embodiment, $\mathbf{R_1}$ and $\mathbf{R_2}$ are independently chosen from groups (i) to (iv) below:

(i) the compound of formula (III) below:
[Chem. 4]

$$R_7\text{-O-C(O)-C(}R_8\text{)(H)-} \qquad \text{(III)}$$

in which $R_7$ represents a $C_1$-$C_3$alkyl group and $R_8$ represents a hydrogen atom or a $C_1$-$C_3$ alkyl group; preferably, $R_7$ is a methyl and $R_8$ is a hydrogen atom or a methyl.

(ii) the compound of formula (IV) below:
[Chem. 5]

$$R_9\text{-[O-}CH_2\text{-C(H)(}R_{10}\text{)]}_p\text{-} \qquad \text{(IV)}$$

in which $R_9$ represents a $C_1$-$C_4$alkyl group, $R_{10}$ represents a hydrogen atom or a $C_1$-$C_4$ alkyl group and p denotes an integer ranging from 1 to 3; preferably, $R_9$ is a methyl, ethyl or butyl, $R_{10}$ is a hydrogen atom or a methyl and p is equal to 1.

(iii) the compound of formula (V) below:
[Chem. 6]

$$(R_{11})_2\text{N-}CH_2\text{-C(H)(}R_{12}\text{)-} \qquad \text{(V)}$$

in which $R_{11}$ represents a $C_1$-$C_4$alkyl group and $R_{12}$ represents a hydrogen atom or a $C_1$-$C_4$ alkyl group; preferably, $R_{11}$ is a methyl, ethyl or butyl and $R_{12}$ is a hydrogen atom or a methyl.

(iv) the compound of formula (VI) below:
[Chem. 7]

$$R_{13}\text{-[O-}CH_2\text{-C(H)(}R_{14}\text{)]}_q\text{-} \qquad \text{(VI)}$$

in which $R_{13}$ represents a $C_1$-$C_4$alkyl group or a phenyl, $R_{14}$ represents a hydrogen atom or a $C_1$-$C_4$ alkyl group and q denotes an integer ranging from 4 to 30; preferably, $R_{13}$ is a methyl, ethyl or butyl and $R_{14}$ is a hydrogen atom or a methyl.

Preferably, $\mathbf{R_1}$ and $\mathbf{R_2}$ independently represent a compound of formula (VI) in which $R_{13}$ represents a $C_1$-$C_4$ alkyl group or a phenyl, preferably a $C_1$-$C_4$ alkyl group, more preferentially a methyl, $R_{14}$ represents a hydrogen atom or a $C_1$-$C_4$ alkyl group, preferably a hydrogen atom and q denotes an integer ranging from 4 to 30.

According to an alternative embodiment, $\mathbf{R_1}$ and $\mathbf{R_2}$ are different and one of the radicals $\mathbf{R_1}$ or $\mathbf{R_2}$ represents a compound of formula (IV) as described above and the other radical $\mathbf{R_1}$ or $\mathbf{R_2}$ represents a compound of formula (VI) as described above.

Preferably, in formula (IV), $R_9$ is a methyl, ethyl or butyl and $R_{10}$ is a hydrogen atom or a methyl and p is equal to 1.

Preferably, in formula (VI), $R_{13}$ is a methyl, ethyl or butyl and $R_{14}$ is a hydrogen atom or a methyl and q denotes an integer ranging from 4 to 30.

According to another alternative embodiment, $\mathbf{R_1}$ and $\mathbf{R_2}$ are identical and represent a compound of formula (VI) in which $R_{13}$ represents a $C_1$-$C_4$ alkyl group or a phenyl, preferably a $C_1$-$C_4$ alkyl group, more preferentially a methyl, $R_{14}$ represents a hydrogen atom or a $C_1$-$C_4$ alkyl group, preferably a hydrogen atom and q denotes an integer ranging from 4 to 30.

Preferably, n is an integer ranging from 1 to 20, more preferentially from 2 to 20.

Preferably, z denotes an integer ranging from 1 to 20, more preferentially from 2 to 20.

Preferably, w is equal to 1.

Preferably, **w is** equal to 1, **n+z** denotes an integer ranging from 4 to 10.

Preferably, **Li** is chosen from a $C_1$-$C_{18}$ divalent aliphatic hydrocarbon-based radical such as methylene, ethylene and propylene, a $C_3$-$C_{15}$ cycloalkylene radical such as cyclopentylene, cycloheptylene and cyclohexylene, a $C_3$-$C_{12}$ heterocycloalkylene group such as imidazolene, pyrrolene and furanylene, or a $C_6$-$C_{14}$ arylene group such as phenylene, and mixtures thereof.

For example, $\mathbf{L_1}$ may be chosen from a radical derived from tolylene diisocyanate, hexamethylene diisocyanate,

xylylene diisocyanate, 2,2,4-trimethylhexamethylene diisocyanate, 1,12-dodecane diisocyanate, norbornane diisocyanate, 2,4-bis(8-isocyanatooctyl)-1,3-dioctylcyclobutane, 4,4'-dicylclohexylmethane diisocyanate, tetramethylxylylene diisocyanate, isophorone diisocyanate, 1,5-napththylene diisocyanate, 4,4'-diphenylmethane diisocyanate, 4,4'-diphenyldimethylmethane diisocyanate and phenylene diisocyanate, and mixtures thereof.

Preferably, $\mathbf{L_1}$ is chosen from a $C_3$-$C_{15}$ cycloalkylene radical or a $C_6$-$C_{14}$ arylene group, and mixtures thereof, such as the compounds of formula (VII) below:

[Chem. 8]

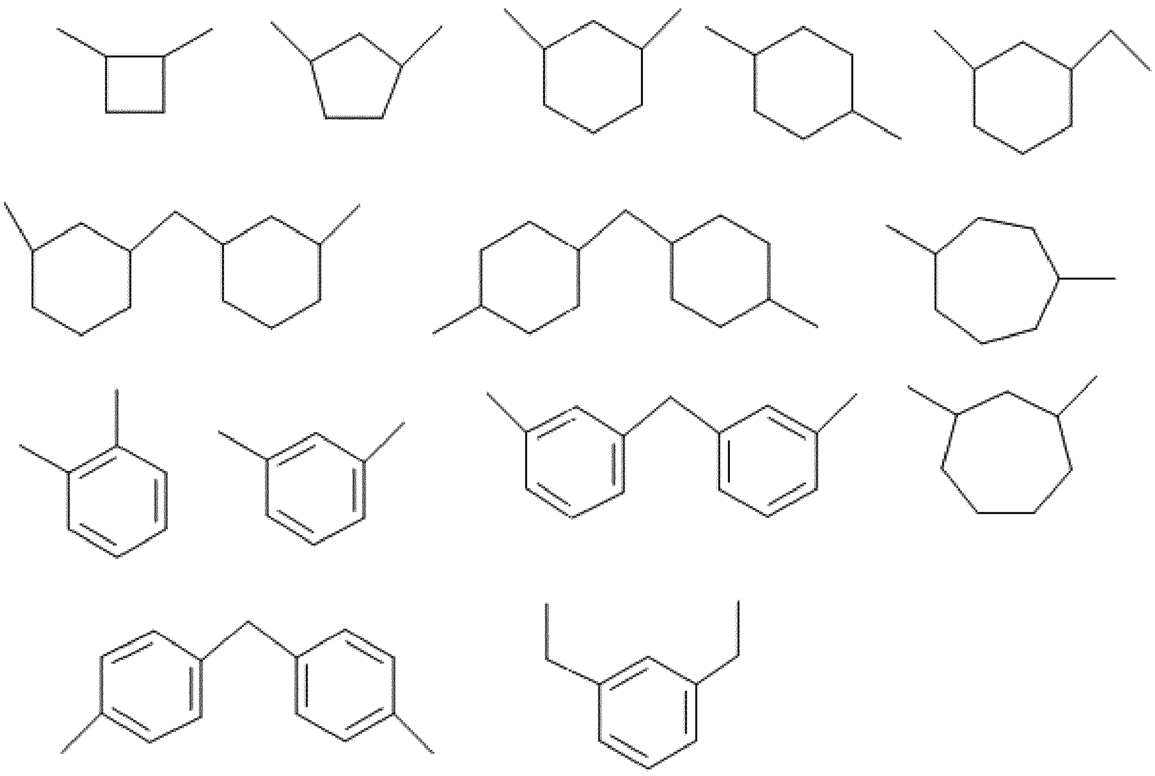

(VII)

Preferably, $\mathbf{L_1}$ is 4,4-dicyclohexylenemethane corresponding to formula (VIII) below:

[Chem. 9]

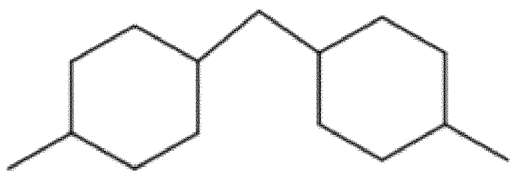

(VIII)

According to another embodiment, when Li is a $C_6$-$C_{14}$ arylene group, Li is not the m-tetramethylxylylene radical represented by formula (IX) below:

[Chem. 10]

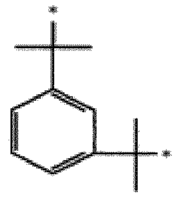

(IX)

As indicated previously, **E** independently represents a group chosen from:

- $-O-R_3-O-$; $-S-R_4-S-$; $-R_5-N(R_6)-R_4-N(R_6)-R_5-$;

in which $\mathbf{R_3}$ and $\mathbf{R_4}$ independently represent a divalent hydrocarbon-based radical optionally interrupted with one or more heteroatoms;

- $\mathbf{R_5}$ independently represents a covalent bond or a saturated divalent hydrocarbon-based radical, optionally interrupted with one or more heteroatoms; and
- $\mathbf{R_6}$ independently represents a hydrogen atom or a hydrocarbon-based radical, optionally interrupted with one or more heteroatoms.

Preferably, $\mathbf{R_3}$ and $\mathbf{R_4}$ are independently chosen from a $C_6$-$C_{14}$ arylene radical such as phenylene, a $C_3$-$C_{12}$ cycloalkylene radical such as cyclopropylene and cyclobutylene, a linear or branched $C_1$-$C_{18}$ alkylene radical such as methylene and ethylene, optionally interrupted with one or more heteroatoms, and mixtures thereof.

More preferentially, $\mathbf{R_3}$ and $\mathbf{R_4}$ are independently chosen from a linear or branched $C_1$-$C_{18}$ alkylene radical such as methylene, butylene, propylene or ethylene, optionally interrupted with one or more heteroatoms.

Preferably, when $\mathbf{R_5}$ is not a covalent bond, $\mathbf{R_5}$ is chosen from a $C_6$-$C_{14}$ arylene radical such as phenylene, a $C_3$-$C_{12}$ cycloalkylene radical such as cyclopropylene and cyclobutylene, a linear or branched $C_1$-$C_{18}$ alkylene radical such as methylene and ethylene, optionally interrupted with one or more heteroatoms, and mixtures thereof.

Preferably, $\mathbf{R_6}$ is chosen from a $C_6$-$C_{14}$ arylene radical such as phenylene, a $C_3$-$C_{12}$ cycloalkylene radical such as cyclopropylene and cyclobutylene, a linear or branched $C_1$-$C_{18}$ alkylene radical such as methylene and ethylene, optionally interrupted with one or more heteroatoms, and mixtures thereof.

Preferably, **E** represents a group $-O-R_3-O-$ in which $\mathbf{R_3}$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof.

More preferentially, **E** represents a group $-O-R_3-O-$ in which $\mathbf{R_3}$ represents a linear or branched $C_1$-$C_{18}$ alkylene radical such as methylene, butylene, propylene or ethylene, optionally interrupted with one or more heteroatoms.

According to a particular embodiment, the (poly)carbodiimide compound is a copolymer derived from $\alpha$-methylstyryl isocyanates of formula (X) below:

[Chem. 11]

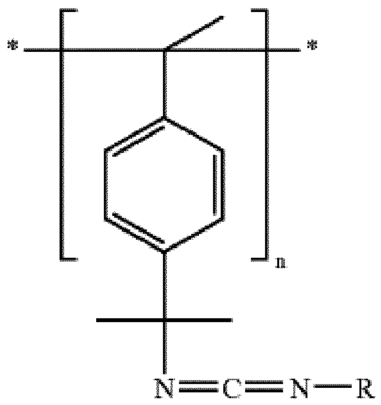

(X)

in which R independently represents an alkyl group containing from 1 to 24 carbon atoms, a cycloalkyl group containing from 3 to 24 carbon atoms or an aryl group containing from 6 to 24 carbon atoms, and

- n denotes an integer ranging from 2 to 100.

In this embodiment, the term "alkyl group" is as defined previously.

In this embodiment, the term "cycloalkyl group" is as defined previously.

In this embodiment, n may denote an integer ranging from 2 to 50, preferably from 3 to 30 and even more preferentially from 5 to 10.

According to another particular embodiment, the (poly)carbodiimide compound is a compound of formula (XI) below:

[Chem. 12]

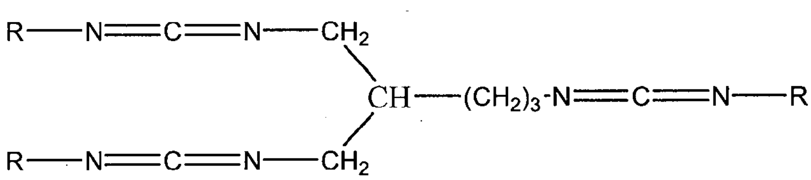

(XI)

in which R independently represents an alkyl group containing from 1 to 24 carbon atoms, a cycloalkyl group containing from 3 to 24 carbon atoms or an aryl group containing from 6 to 24 carbon atoms.

The "alkyl group", the "cycloalkyl group" and the "aryl group" are as defined previously.

According to a preferred embodiment, the (poly)carbodiimide compound is chosen from the compounds of formula (I) or of formula (II) in which:

- **$X_1$** and **$X_2$** independently represent an oxygen atom;
- **$R_1$** and **$R_2$**, are independently chosen from dialkylamino alcohols, alkyl esters of hydroxycarboxylic acid and monoalkyl ethers of (poly)alkylene glycol, in which a hydroxyl group has been removed, and mixtures thereof, preferably monoalkyl ethers of (poly)alkylene glycol, in which a hydroxyl group has been removed, more preferentially the compound of formula (VI) as described previously in which $R_{13}$ represents a $C_1$-$C_4$ alkyl group or a phenyl, preferably a $C_1$-$C_4$ alkyl group, more preferentially a methyl, $R_{14}$ represents a hydrogen atom or a $C_1$-$C_4$ alkyl group, preferably a hydrogen atom, and q denotes an integer ranging from 4 to 30;
- **n and z,** when they are present, denote an integer ranging from 1 to 20, with $n+z \geq 2$ and **w** is equal to 1;
- **$L_1$**, when it is present, is chosen from a $C_1$-$C_{18}$ divalent aliphatic hydrocarbon-based radical, a $C_3$-$C_{15}$ cycloalkylene radical, a $C_3$-$C_{12}$ heterocycloalkylene group or a $C_6$-$C_{14}$ arylene group, and mixtures thereof, preferably a $C_3$-$C_{15}$ cycloalkylene radical;

- **A,** when it is present, is chosen from a $C_1$-$C_{18}$ divalent aliphatic hydrocarbon-based radical, a $C_3$-$C_{15}$ cycloalkylene radical, a $C_3$-$C_{12}$ heterocycloalkylene group or a $C_6$-$C_{14}$ arylene group, and mixtures thereof, preferably a $C_3$-$C_{15}$ cycloalkylene radical;
- **E,** when it is present, independently represents a group chosen from:

- -O-$R_3$-O-; -S-$R_4$-S-; -$R_5$-N($R_6$)-$R_4$-N($R_6$)-$R_5$-;

in which $\mathbf{R_3}$ and $\mathbf{R_4}$ are independently chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof;
- when $\mathbf{R_5}$ is not a covalent bond, $\mathbf{R_5}$, when it is present, is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof; and
- $\mathbf{R_6}$, when it is present, is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof.

Preferably, the (poly)carbodiimide compound is chosen from the compounds of formula (II) in which:

- $\mathbf{X_1}$ and $\mathbf{X_2}$ independently represent an oxygen atom;
- $\mathbf{R_1}$ and $\mathbf{R_2}$ are independently chosen from dialkylamino alcohols, alkyl esters of hydroxycarboxylic acid and monoalkyl ethers of (poly)alkylene glycol, in which a hydroxyl group has been removed, and mixtures thereof;
- **n and z** denote an integer ranging from 1 to 20, with $n+z \geq 2$ and w is equal to 1;
- $\mathbf{L_1}$ is chosen from a $C_1$-$C_{18}$ divalent aliphatic hydrocarbon-based radical, a $C_3$-$C_{15}$ cycloalkylene radical, a $C_3$-$C_{12}$ heterocycloalkylene group or a $C_6$-$C_{14}$ arylene group, and mixtures thereof;
- **E** independently represents a group chosen from:

- -O-$R_3$-O-; -S-$R_4$-S-; -$R_5$-N($R_6$)-$R_4$-N($R_6$)-$R_5$-,

in which $\mathbf{R_3}$ and $\mathbf{R_4}$ are independently chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof;
- when $\mathbf{R_5}$ is not a covalent bond, $\mathbf{R_5}$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof; and
- $\mathbf{R_6}$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof.

More preferentially, the (poly)carbodiimide compound is chosen from the compounds of formula (II) in which:

- $\mathbf{X_1}$ and $\mathbf{X_2}$ independently represent an oxygen atom;
- $\mathbf{R_1}$ and $\mathbf{R_2}$ are, independently, monoalkyl ethers of (poly)alkylene glycol, in which a hydroxyl group has been removed;
- **n and z** denote an integer ranging from 1 to 20, with $n+z \geq 2$ and w is equal to 1;
- $\mathbf{L_1}$ is a $C_3$-$C_{15}$ cycloalkylene radical;
- **E** independently represents a group chosen from:

- -O-$R_3$-O-; -S-$R_4$-S-; -$R_5$-N($R_6$)-$R_4$-N($R_6$)-$R_5$-,

in which $\mathbf{R_3}$ and $\mathbf{R_4}$ are independently chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof;
- when $\mathbf{R_5}$ is not a covalent bond, $\mathbf{R_5}$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof; and
- $\mathbf{R_6}$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof.

Even more preferentially, the (poly)carbodiimide compound is chosen from the compounds of formula (II) in which:

- $\mathbf{X_1}$ and $\mathbf{X_2}$ independently represent an oxygen atom;
- $\mathbf{R_1}$ and $\mathbf{R_2}$ independently represent the compound of formula (VI) below:
[Chem. 13]

$R_{13}$-[O-$CH_2$-C(H)($R_{14}$)]$_q$-     (VI)

in which $\mathbf{R_{13}}$ represents a $C_1$-$C_4$ alkyl group or a phenyl, preferably a $C_1$-$C_4$ alkyl group, more preferentially a methyl, $\mathbf{R_{14}}$ represents a hydrogen atom or a $C_1$-$C_4$ alkyl group, preferably a hydrogen atom and **q** denotes an integer ranging from 4 to 30;

- **n and z** denote an integer ranging from 2 to 20, with **n+z** ranging from 4 to 10 and w is equal to 1;
- $\mathbf{L_1}$ is an $C_3$-$C_{15}$ cycloalkylene radical such as cyclopentylene, cycloheptylene, cyclohexylene and 4,4-dicyclohexylenemethane; and
- **E** represents a group -O-$R_3$-O- in which $\mathbf{R_3}$ is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof.

Even more preferentially, the (poly)carbodiimide compound is chosen from the compounds of formula (I) in which:

- $\mathbf{X_1}$ and $\mathbf{X_2}$ independently represent an oxygen atom;
- $\mathbf{R_1}$ and $\mathbf{R_2}$ independently represent the compound of formula (VI) below:
[Chem. 14]

$R_{13}$-[O-$CH_2$-C(H)($R_{14}$)]$_q$-     (VI)

in which $R_{13}$ represents a $C_1$-$C_4$ alkyl group or a phenyl, preferably a $C_1$-$C_4$ alkyl group, more preferentially a methyl, $R_{14}$ represents a hydrogen atom or a $C_1$-$C_4$ alkyl group, preferably a hydrogen atom and q denotes an integer ranging from 4 to 30;

- **n and z** denote an integer ranging from 1 to 20, with n+z ranging from 4 to 10 and w is equal to 1;
- $\mathbf{L_1}$ is an $C_3$-$C_{15}$ cycloalkylene radical such as cyclopentylene, cycloheptylene, cyclohexylene and 4,4-dicyclohexylenemethane, preferably 4,4-dicyclohexylenemethane; and
- **E** represents a group -O-$R_3$-O- in which $\mathbf{R_3}$ represents a linear or branched $C_1$-$C_{18}$ alkylene radical such as methylene, butylene, propylene or ethylene, optionally interrupted with one or more heteroatoms.

According to a preferred embodiment, the (poly)carbodiimide compound is a compound of formula (XII) below:

[Chem. 15]

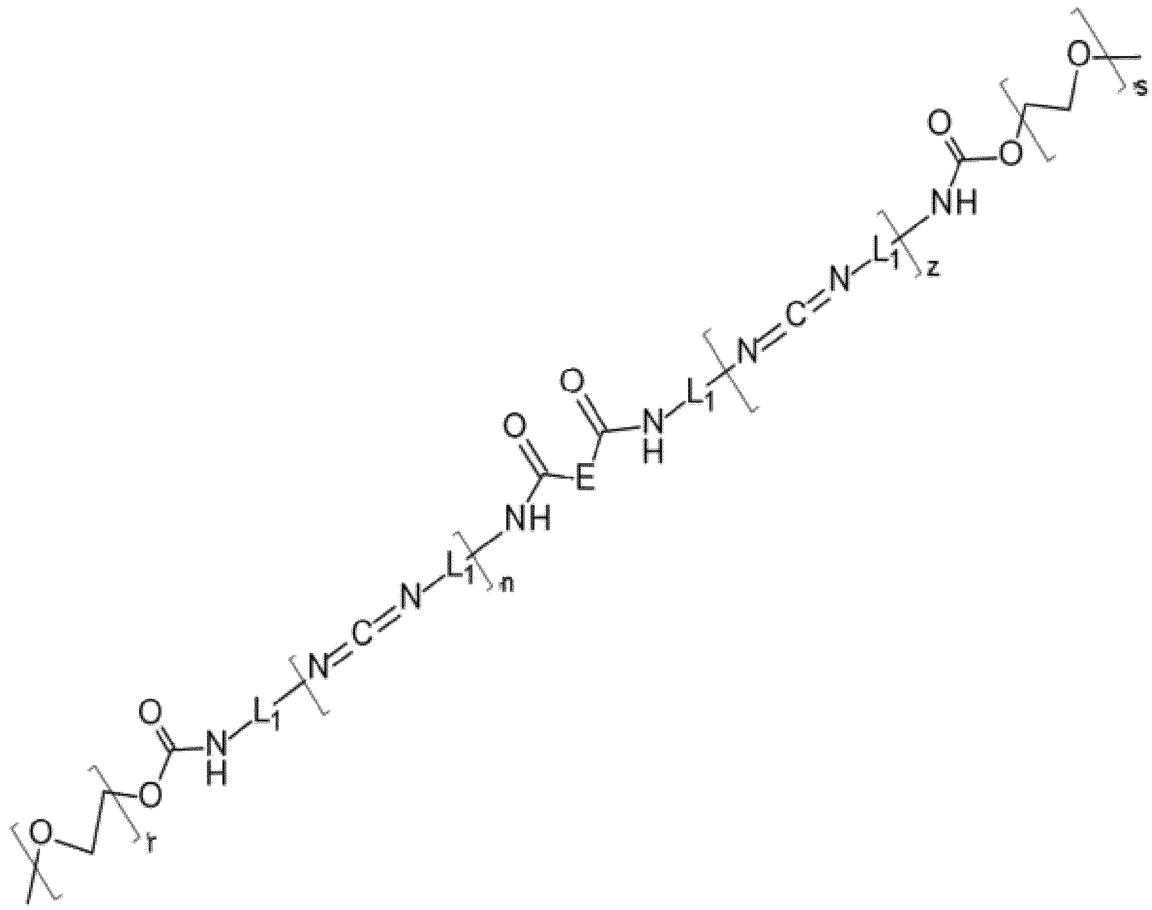

(XII)

in which $L_1$ is 4,4-dicyclohexylenemethane, n and z denote an integer ranging from 1 to 20, with n+z ranging from 4 to 10, E represents a group -O-$R_3$-O- in which $R_3$ represents a linear or branched $C_1$-$C_{18}$ alkylene radical such as methylene, propylene, butylene or ethylene, optionally interrupted with one or more heteroatoms, and r and s denote an integer ranging from 4 to 30.

The total amount of the (poly)carbodiimide compound(s), present in the composition according to the invention, preferably ranges from 0.01% to 40% by weight, more preferentially from 0.1% to 30% by weight, better still from 0.5% to 25% by weight and even better still from 1% to 10% by weight relative to the total weight of composition (C).

**Aqueous dispersion of particles of polymers:**

The composition according to the invention comprises at least one aqueous dispersion of particles of polymer(s) chosen from polyurethanes, acrylic polymers, and mixtures thereof.

The dispersion(s) may be simple dispersions in the aqueous medium of the cosmetic composition. As a particular case of dispersions, mention may be made of latexes.

The aqueous dispersion(s) of polymer particles may be chosen from aqueous dispersions of polyurethane particles.

More particularly, the polyurethane(s) present in the aqueous dispersions used in the present invention are derived from the reaction of:

- a prepolymer of formula (A) below:

[Chem.16]

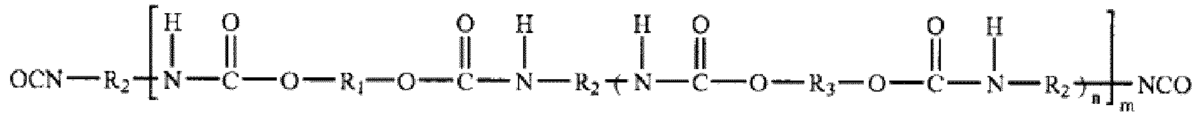

(A)

in which:

- **$R_1$** represents a divalent radical of a dihydroxylated compound,
- **$R_2$** represents a radical of an aliphatic or cycloaliphatic polyisocyanate,
- **$R_3$** represents a radical of a low molecular weight diol, optionally substituted with one or more ionic groups,
- **n** represents an integer ranging from 1 to 5, and
- **m** is greater than 1;

- at least one chain extender according to formula (B) below:
  [Chem.17]

  $H_2N-R_4-NH_2$ (B)

  in which **$R_4$** represents an alkylene or alkylene oxide radical that is not substituted with one or more ionic or potentially ionic groups; and

- at least one chain extender according to formula (C) below:
  [Chem.18]

  $H_2N-R_5-NH_2$ (C),

  in which **$R_5$** represents an alkylene radical substituted with one or more ionic or potentially ionic groups.

Among the dihydroxylated compounds that may be used according to the present invention, mention may be made notably of the compounds containing two hydroxyl groups and having a number-average molecular weight from about 700 to about 16 000, and preferably from about 750 to about 5000. As examples of dihydroxylated compounds of high molecular weight, mention may be made of polyol polyesters, polyol polyethers, polyhydroxylated polycarbonates, polyhydroxylated polyacetates, polyhydroxylated polyacrylates, polyhydroxylated amide polyesters, polyhydroxylated polyalkadienes, polyhydroxylated polythioethers, and mixtures thereof. Preferably, the hydroxylated compounds are chosen from polyol polyesters, polyol polyethers, polyhydroxylated polycarbonates, and mixtures thereof.

The polyisocyanates that may be used according to the present invention are notably chosen from organic diisocyanates with a molecular weight of about 112 to 1000, and preferably about 140 to 400.

Preferably, the polyisocyanates are chosen from diisocyanates and more particularly from those represented by the general formula $R_2(NCO)_2$, in which **$R_2$** represents a divalent aliphatic hydrocarbon-based group containing from 4 to 18 carbon atoms, a divalent cycloaliphatic hydrocarbon-based group containing from 5 to 15 carbon atoms, a divalent araliphatic hydrocarbon-based group containing from 7 to 15 carbon atoms or a divalent aromatic hydrocarbon-based group containing from 6 to 15 carbon atoms.

Preferably, **$R_2$** represents an organic diisocyanate. As examples of organic diisocyanates, the following may notably be chosen: tetramethylene diisocyanate, 1,6-hexamethylene diisocyanate, dodecamethylene diisocyanate, 1,3-diisocyanatocyclohexane, 1,4-diisocyanatocyclohexane, 3-isocyanatomethyl-3,5,5-trimethylcyclohexane isocyanate (isophorone diisocyanate or IPDI), bis(4-isocyanatocyclohexyl)methane, 1,3-bis(isocyanatomethyl)cyclohexane, 1,4-bis(isocyanatomethyl)cyclohexane, bis(4-isocyanato-3-methyl-cyclohexyl)methane, isomers of toluene diisocyanate (TDI) such as toluene 2,4-diisocyanate, toluene 2,6-diisocyanate and mixtures thereof, hydrogenated toluene diisocyanate, diphenylmethane 4,4'-diisocyanate and mixtures with its diphenylmethane 2,4-diisocyanate isomers and optionally diphenylmethane 2,2'-diisocyanate isomers, naphthalene 1,5-diisocyanate, and mixtures thereof.

Preferably, the diisocyanates are aliphatic and cycloaliphatic diisocyanates, and are more preferentially chosen from 1,6-hexamethylene diisocyanate, 3-isocyanatomethyl-3,5,5-trimethylcyclohexane isocyanate, and mixtures thereof.

According to the present invention, the term *"low molecular weight diol"* refers to a diol with a molecular weight from about 62 to 700, and preferably from 62 to 200. These diols may comprise aliphatic, alicyclic or aromatic groups. Preferably, they comprise only aliphatic groups.

Preferably, $\mathbf{R_3}$ represents a low molecular weight diol containing more than 20 carbon atoms, more preferentially chosen from ethylene glycol, diethylene glycol, 1,2-propanediol, 1,3-propanediol, 1,4-butanediol, 1,3-butylene glycol, neopentyl glycol, butylethylpropanediol, cyclohexanediol, 1,4-cyclohexanedimethanol, 1,6-hexanediol, bisphenol A (2,2-bis(4-hydroxyphenyl)propane), hydrogenated bisphenol A (2,2-bis(4-hydroxycyclohexyl)propane), and mixtures thereof.

The low molecular weight diols may optionally comprise ionic or potentially ionic groups. Examples of low molecular weight diols containing ionic or potentially ionic groups are notably described in patent US 3 412 054. Such compounds are preferably chosen from dimethylolbutanoic acid, dimethylolpropionic acid, polycaprolactone diols containing a carboxyl group, and mixtures thereof.

If low molecular weight diols containing ionic or potentially ionic groups are used, they are preferably used in an amount such that less than 0.30 meq of COOH per gram of polyurethane is present in the polyurethane dispersion.

The prepolymer is extended by means of two families of chain extenders. The first family of chain extenders corresponds to the compounds of general formula (B).

The chain extenders of formula (B) are preferably chosen from alkylenediamines, such as hydrazine, ethylenediamine, propylenediamine, 1,4-butylenediamine, piperazine; alkylene oxide diamines, such as 3-{2-[2-(3-aminopropoxy)ethoxy]ethoxy}propylamine (also known as dipropylamine diethylene glycol or DPA-DEG available from Tomah Products, Milton, Wis.), 2-methyl-1,5-pentanediamine (Dytec A from DuPont), hexanediamine, isophorone diamine, 4,4-methylenedi(cyclohexylamine), ether-amines of the DPA series, available from Tomah Products, Milton, Wis., such as dipropylamine propylene glycol, dipropylamine dipropylene glycol, dipropylamine tripropylene glycol, dipropylamine poly(propylene glycol), dipropylamine ethylene glycol, dipropylamine poly(ethylene glycol), dipropylamine 1,3-propanediol, dipropylamine 2-methyl-1,3-propanediol, dipropylamine 1,4-butanediol, dipropylamine 1,3-butanediol, dipropylamine 1,6-hexanediol and dipropylamine cyclohexane-1,4-dimethanol; and mixtures thereof.

The second family of chain extenders corresponds to the compounds of general formula (C). Such compounds preferably have an ionic or potentially ionic group and two groups that can react with isocyanate groups. Such compounds may optionally comprise two groups that react with isocyanate groups and one group which is ionic or capable of forming an ionic group.

The ionic or potentially ionic group may preferably be chosen from ternary or quaternary ammonium groups or groups that can be converted into such groups, a carboxyl group, a carboxylate group, a sulfonic acid group and a sulfonate group. The at least partial conversion of groups that can be converted into a ternary or quaternary ammonium group salt may be performed before or during the mixing with water.

The chain extenders of formula (C) are preferably chosen from diaminosulfonates, for instance the sodium salt of N-(2-aminoethyl)-2-aminoethanesulfonic acid (ASA), the sodium salt of N-(2-aminoethyl)-2-aminopropionic acid, and mixtures thereof.

The polyurethane that may be used according to the present invention may optionally also comprise compounds which are located, respectively, at the chain ends and terminate said chains (chain terminators). Such compounds are notably described in patents US 7 445 770 and/or US 7 452 770.

Preferably, the aqueous dispersion of polyurethane particles has a viscosity of less than 2000 mPa.s at 23°C, more preferentially less than 1500, and even better still less than 1000. Even more preferably, the aqueous polyurethane dispersion has a glass transition temperature of less than 0°C.

Preferably also, the aqueous polyurethane dispersion has a polyurethane (or active material, or solids) content, on the basis of the weight of the dispersion, of from 20% to 60% by weight, more preferentially from 25% to 55% by weight and even better still from 30% to 50% by weight. This means that the polyurethane content (solids) of the aqueous dispersion is preferably from 20% to 60% by weight, more preferentially from 25% to 55% by weight and even better still from 30% to 50% by weight, relative to the total weight of the dispersion.

Preferably also, the aqueous dispersion of polyurethane particles has a glass transition temperature (Tg) of less than or equal to -25°C, preferably less than -35°C and more preferentially less than -40°C.

The polyurethane particles may have a mean diameter ranging up to about 1000 nm, for example from about 50 nm to about 800 nm, better still from about 100 nm to about 500 nm. These particle sizes may be measured with a laser particle size analyzer (for example Brookhaven BI90).

As nonlimiting examples of aqueous polyurethane dispersions, mention may be made of those sold under the name Baycusan® by Bayer, for instance Baycusan® C1000 (INCI name: polyurethane-34), Baycusan® C1001 (INCI name: polyurethane-34), Baycusan® C1003 (INCI name: polyurethane-32), Baycusan® C1004 (INCI name: polyurethane-35) and Baycusan® C1008 (INCI name: polyurethane-48).

Mention may also be made of the aqueous polyurethane dispersions of isophthalic acid/adipic acid copolymer/hexylene glycol/neopentyl glycol/dimethylol acid/isophorone diisocyanate (INCI name: Polyurethane-1, such as

Luviset® PUR, BASF), the polyurethane of polycarbonate, polyurethane and aliphatic polyurethane of aliphatic polyester (such as the Neorez® series, DSM, such as Neorez® R989, Neorez® and R-2202).

According to a preferred embodiment, the aqueous dispersion of polyurethane particles may be chosen from aqueous dispersions of particles of compounds having the INCI name polyurethane-35 or compounds having the INCI name polyurethane-34.

Preferably, the aqueous dispersion(s) of polymers according to the invention are chosen from aqueous dispersions of acrylic polymer particles, and more preferentially from aqueous dispersions of film-forming acrylic polymer particles.

For the purposes of the invention, the term *"polymer"* means a compound corresponding to the repetition of one or more units (these units being derived from compounds known as monomers). This or these unit(s) are repeated at least twice and preferably at least three times.

The term *"film-forming polymer"* refers to a polymer that is capable of forming, by itself or in the presence of an auxiliary film-forming agent, a macroscopically continuous film on a support, notably on keratin materials, and preferably a cohesive film.

For the purposes of the present invention, the term *"acrylic polymer"* means a polymer synthesized from at least one monomer chosen from (meth)acrylic acid and/or (meth)acrylic acid ester and/or (meth)acrylic acid amide.

The unit(s) derived from the (meth)acrylic acid monomers of the polymer may optionally be in the form of salt(s), notably of alkali metal, alkaline-earth metal or ammonium salt(s), or organic base salt(s).

The (meth)acrylic acid esters (also known as (meth)acrylates) are advantageously chosen from alkyl (meth) acrylates, in particular $C_1$ to $C_{30}$, preferably $C_1$ to $C_{20}$ and better still $C_1$ to $C_{10}$ alkyl (meth)acrylates, aryl (meth)acrylates, in particular $C_6$ to $C_{10}$ aryl (meth)acrylates, and hydroxyalkyl (meth)acrylates, in particular $C_2$ to $C_6$ hydroxyalkyl (meth) acrylates.

Among the alkyl (meth)acrylates that may be mentioned are methyl (meth)acrylate, ethyl (meth)acrylate, butyl (meth)acrylate, isobutyl (meth)acrylate, 2-ethylhexyl (meth)acrylate, lauryl (meth)acrylate and cyclohexyl (meth)acrylate.

Among the hydroxyalkyl (meth)acrylates that may be mentioned are hydroxyethyl acrylate, 2-hydroxypropyl acrylate, hydroxyethyl methacrylate and 2-hydroxypropyl methacrylate.

Among the aryl (meth)acrylates that may be mentioned are benzyl acrylate and phenyl acrylate.

The (meth)acrylic acid esters that are particularly preferred are alkyl, preferably $C_1$ to $C_{30}$, more preferentially $C_1$ to $C_{20}$, even better still $C_1$ to $C_{10}$, and even more particularly $C_1$ to $C_4$, alkyl (meth)acrylates.

According to the present invention, the alkyl group of the esters may be fluorinated, or even perfluorinated, i.e. some or all of the hydrogen atoms of the alkyl group are substituted with fluorine atoms.

As (meth)acrylic acid amides, examples that may be mentioned include (meth)acrylamides and also N-alkyl(meth)acrylamides, in particular N-($C_2$ to $C_{12}$ alkyl)(meth)acrylamides. Among the N-alkyl(meth)acrylamides that may be mentioned are N-ethylacrylamide, N-t-butylacrylamide, N-t-octylacrylamide and N-undecylacrylamide.

The acrylic polymer according to the invention may be a homopolymer or a copolymer, advantageously a copolymer, better still a copolymer of (meth)acrylic acid and of (meth)acrylic acid esters.

Preferably, the acrylic polymer(s) according to the invention comprise one or more units derived from the following monomers:

a) (meth)acrylic acid; and
b) $C_1$ to $C_{30}$, more preferentially $C_1$ to $C_{20}$, better still $C_1$ to $C_{10}$, and even more particularly $C_1$ to $C_4$, alkyl (meth) acrylate.

Preferably, the aqueous dispersion of acrylic polymer particles does not comprise any surfactant.

The term "surfactant" refers to any agent that is capable of modifying the surface tension between two surfaces.

Among the acrylic polymers according to the invention, mention may be made of copolymers of (meth)acrylic acid and of methyl or ethyl (meth)acrylate, in particular copolymers of methacrylic acid and of ethyl acrylate such as the compound sold under the trade name Luvimer MAE by the company BASF, or the compound Polyacrylate-2 Crosspolymer sold under the trade name Fixate Superhold Polymer by the company Lubrizol, or the compound Acrylate Copolymer sold under the trade name Daitosol 3000VP3 by the company Daito Kasei Kogyo, or the compound Acrylate Polymer sold under the trade name Daitosol 3000 SLPN-PE1 by the company Daito Kasei Kogyo.

The acrylic polymer may optionally comprise one or more additional monomers, other than the (meth)acrylic acid and/or (meth)acrylic acid ester and/or (meth)acrylic acid amide monomers.

By way of additional monomer, mention will be made, for example, of styrene monomers, in particular styrene and $\alpha$-methylstyrene, and preferably styrene.

In particular, the acrylic polymer may be a styrene/(meth)acrylate copolymer and notably a polymer chosen from copolymers resulting from the polymerization of at least one styrene monomer and at least one $C_1$ to $C_{20}$, preferably $C_1$ to $C_{10}$, alkyl (meth)acrylate monomer.

The $C_1$ to $C_{10}$ alkyl (meth)acrylate monomer may be chosen from methyl acrylate, ethyl acrylate, propyl acrylate, butyl acrylate, hexyl acrylate, octyl acrylate and 2-ethylhexyl acrylate.

As acrylic polymer, mention may be made of the styrene/(meth)acrylate copolymers sold under the name Joncryl 77 by the company BASF, under the name Yodosol GH41F by the company Akzo Nobel and under the name Syntran 5760 CG by the company Interpolymer.

Preferably, composition (C) comprises at least one aqueous dispersion of acrylic polymer particles.

More preferentially, composition (C) comprises at least one aqueous dispersion of acrylic polymer particles comprising one or more units derived from the following monomers:

a) (meth)acrylic acid; and

b) $C_1$ to $C_{30}$, more preferentially $C_1$ to $C_{20}$, better still $C_1$ to $C_{10}$, and even more particularly $C_1$ to $C_4$, alkyl (meth) acrylate.

Preferably, the aqueous dispersion of acrylic polymer particles has an acrylic polymer (or active material, or solids) content, on the basis of the weight of the dispersion, of from 20% to 60% by weight, more preferentially from 22% to 55% by weight and better still from 25% to 50% by weight.

The total amount of the aqueous dispersion(s) of polymer particles, present in the composition according to the invention, preferably ranges from 0.1% to 40% by weight, more preferentially from 0.1% to 35% by weight and better still from 0.2% to 30% by weight, relative to the total weight of composition (C).

According to a preferred embodiment, the total amount of the aqueous dispersion(s) of acrylic polymer particles, present in the composition according to the invention, preferably ranges from 0.1% to 40% by weight, more preferentially from 0.1% to 35% by weight, and better still from 0.2% to 30% by weight, relative to the total weight of composition (C).

**Silicone:**

Composition (C) also comprises at least one silicone.

Preferably, composition (C) comprises at least one silicone chosen from non-amino silicones, amino silicones and mixtures thereof.

The silicones may be solid or liquid at 25°C and atmospheric pressure ($1.013 \times 10^5$ Pa), and volatile or nonvolatile.

The silicones that may be used may be soluble or insoluble in the composition according to the invention; they may be in the form of oil, wax, resin or gum; silicone oils are preferred.

Silicones are notably described in detail in Walter Noll's Chemistry and Technology of Silicones (1968), Academic Press.

Preferably, the composition contains one or more silicones that are liquid at 25°C and atmospheric pressure ($1.013\times10^5$ Pa).

The volatile silicones may be chosen from those with a boiling point of between 60°C and 260°C (at atmospheric pressure) and more particularly from:

i) cyclic polydialkylsiloxanes including from 3 to 7 and preferably 4 to 5 silicon atoms, such as

- octamethylcyclotetrasiloxane and decamethylcyclopentasiloxane.

Mention may be made of the products sold under the name Volatile Silicone 7207 by Union Carbide or Silbione 70045 V 2 by Rhodia, Volatile Silicone 7158 by Union Carbide or Silbione 70045 V 5 by Rhodia.

- cyclocopolymers of the dimethylsiloxane/methylalkylsiloxane type having the chemical structure:

[Chem.19]

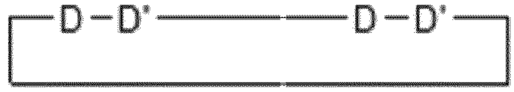

with D : 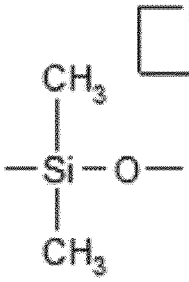 with D' : 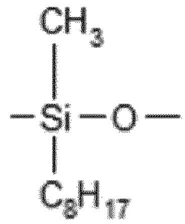

Preferably cyclomethylsiloxane.

Mention may be made of Volatile Silicone FZ 3109 sold by the company Union Carbide.

- mixtures of cyclic silicones with silicon-derived organic compounds, such as the mixture of octamethylcyclotetrasiloxane and of tetratrimethylsilylpentaerythritol (50/50) and the mixture of octamethylcyclotetrasiloxane and of 1,1'-oxy(2,2,2',2',3,3'-hexatrimethylsilyloxy)bisneopentane;

ii) linear polydialkylsiloxanes containing 2 to 9 silicon atoms, which generally have a viscosity of less than or equal to $5\times10^{-6}$ $m^2/s$ at 25°C, such as decamethyltetrasiloxane.

Other silicones belonging to this category are described in the article published in Cosmetics and Toiletries, Vol. 91, Jan. 76, pages 27-32, Todd & Byers Volatile Silicone Fluids for Cosmetics; mention may be made of the product sold under the name SH 200 by the company Toray Silicone.

Among the nonvolatile silicones, mention may be made, alone or as a mixture, of polydialkylsiloxanes and notably polydimethylsiloxanes (PDMS), polydiarylsiloxanes, polyalkylarylsiloxanes, silicone gums and resins, and also organopolysiloxanes (or organomodified polysiloxanes, or alternatively organomodified silicones) which are polysiloxanes including in their structure one or more organofunctional groups, generally attached via a hydrocarbon-based group, and preferably chosen from aryl groups, amine groups, alkoxy groups and polyoxyethylene or polyoxypropylene groups. Preferably, the nonvolatile silicones are chosen from poly dimethyl/methylsiloxanes which are optionally oxyethylenated and oxypropylenated.

The organomodified silicones may be polydiarylsiloxanes, notably polydiphenylsiloxanes, and polyalkylarylsiloxanes, functionalized with the organofunctional groups mentioned previously. The polyalkylarylsiloxanes are particularly chosen from linear and/or branched polydimethyl/methylphenylsiloxanes and polydimethyl/diphenylsiloxanes.

Among the organomodified silicones, mention may be made of organopolysiloxanes including:

- polyoxyethylene and/or polyoxypropylene groups optionally including C6-C24 alkyl groups, such as dimethicone copolyols, and notably those sold by the company Dow Corning under the name DC 1248 or the oils Silwet® L 722, L 7500, L 77 and L 711 from the company Union Carbide; or alternatively (C12)alkylmethicone copolyols, and notably those sold by the company Dow Corning under the name Q2 5200;
- substituted or unsubstituted amine groups, in particular C1-C4 aminoalkyl groups; mention may be made of the products sold under the name GP4 Silicone Fluid and GP7100 by the company Genesee, or under the names Q2-8220 and DC929 or DC939 by the company Dow Corning;
- thiol groups, such as the products sold under the names GP 72 A and GP 71 from Genesee;
- alkoxylated groups, such as the product sold under the name Silicone Copolymer F-755 by SWS Silicones and Abil Wax® 2428, 2434 and 2440 by the company Goldschmidt;
- hydroxylated groups, for instance polyorganosiloxanes bearing a hydroxyalkyl function;
- acyloxyalkyl groups, such as the polyorganosiloxanes described in patent US-A-4 957 732;
- anionic groups of the carboxylic acid type, as described, for example, in EP 186 507, or of the alkylcarboxylic type, such as the product X-22-3701E from the company Shin-Etsu; or alternatively of the 2-hydroxyalkylsulfonate or 2-hydroxyalkylthiosulfate type, such as the products sold by the company Goldschmidt under the names Abil® S201 and Abil® S255;
- hydroxyacylamino groups, such as the polyorganosiloxanes described in patent application EP 342 834; mention may be made, for example, of the product Q2-8413 from the company Dow Corning.

The silicones may also be chosen from polydialkylsiloxanes, among which mention may be made mainly of polydimethylsiloxanes bearing trimethylsilyl end groups. Among these polydialkylsiloxanes, mention may be made of the following commercial products:

- the Silbione® oils of the 47 and 70 047 series or the Mirasil® oils sold by Rhodia, for instance the oil 70 047 V 500 000;
- the oils of the Mirasil® series sold by the company Rhodia;
- the oils of the 200 series from the company Dow Corning, such as DC200, with a viscosity of 60 000 $mm^2/s$;
- the Viscasil® oils from General Electric and certain oils of the SF series (SF 96, SF 18) from General Electric.

Mention may also be made of polydimethylsiloxanes bearing dimethylsilanol end groups, known under the name dimethiconol (CTFA), such as the oils of the 48 series from the company Rhodia.
In this category of polydialkylsiloxanes, mention may also be made of the products sold under the names Abil Wax® 9800 and 9801 by the company Goldschmidt, which are poly(C1-C20)dialkylsiloxanes.
Products that may be used more particularly in accordance with the invention are mixtures such as:

- mixtures formed from a polydimethylsiloxane with a hydroxy-terminated chain, or dimethiconol (CTFA), and from a cyclic polydimethylsiloxane, also known as cyclomethicone (CTFA), such as the product Q2-1401 sold by the company Dow Corning,
- mixtures formed from a polydimethylsiloxane with a hydroxy-terminated chain, or dimethiconol (CTFA), and from a cyclic polydimethylsiloxane, also known as dimethicone (CTFA), such as the product PMX-1503 Fluid sold by the company Dow Corning.

The polyalkylarylsiloxanes are particularly chosen from linear and/or branched polydimethyl/methylphenylsiloxanes and polydimethyl/diphenylsiloxanes with a viscosity ranging from $1\times 10^{-5}$ to $5\times10^{-2}$ $m^2/s$ at 25°C.
Among these polyalkylarylsiloxanes, mention may be made of the products sold under the following names:

- the Silbione® oils of the 70 641 series from Rhodia;
- the oils of the Rhodorsil® 70 633 and 763 series from Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

Preferably, composition (C) comprises at least one amino silicone. The term *"amino silicone"* denotes any silicone including at least one primary, secondary or tertiary amine or a quaternary ammonium group.
The weight-average molecular masses of these amino silicones may be measured by gel permeation chromatography (GPC) at room temperature (25°C), as polystyrene equivalent. The columns used are μ styragel columns. The eluent is THF and the flow rate is 1 ml/minute. 200 μl of a 0.5% by weight solution of silicone in THF are injected. Detection is performed by refractometry and UV-metry.
Preferably, the amino silicone(s) that may be used in the context of the invention are chosen from:

a) the polysiloxanes corresponding to formula (A):

[Chem. 20]

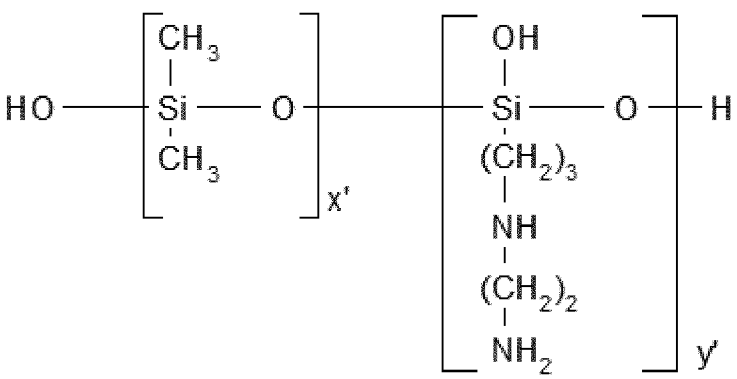

(A)

in which x' and y' are integers such that the weight-average molecular weight (Mw) is between 5000 and 500 000 approximately.

b) the amino silicones corresponding to formula (B):

R'aG3-a-Si(OSiG2)n-(OSiGbR'2-b)m-O-SiG3-a-R'a (B)

in which:

- G, which may be identical or different, denotes a hydrogen atom or a phenyl, OH, $C_1$-$C_8$ alkyl, for example methyl, or $C_1$-$C_8$ alkoxy, for example methoxy, group,
- a, which may be identical or different, denotes 0 or an integer from 1 to 3, in particular 0,
- b denotes 0 or 1, in particular 1,
- m and n are numbers such that the sum (n + m) varies from 1 to 2000 and notably from 50 to 150, it being possible for n to denote a number from 0 to 1999 and in particular from 49 to 149 and it being possible for m to denote a number from 1 to 2000 and in particular from 1 to 10;
- R', which may be identical or different, denotes a monovalent radical of formula -CqH2qL in which q is a number ranging from 2 to 8 and L is an optionally quaternized amine group chosen from the following groups: -N(R")2; -N+(R")3 A-; -NR"-Q-N(R")2 and -NR"-Q-N+(R")3 A-, in which R", which may be identical or different, denotes hydrogen, phenyl, benzyl, or a saturated monovalent hydrocarbon-based radical, for example a $C_1$-$C_{20}$ alkyl radical; Q denotes a linear or branched group of formula CrH2r, r being an integer ranging from 2 to 6, preferably from 2 to 4; and A- represents a cosmetically acceptable anion, notably a halide anion such as a fluoride, chloride, bromide or iodide anion.

Preferably, the amino silicone(s) are chosen from the amino silicones of formula (B). Preferably, the amino silicones of formula (B) are chosen from the amino silicones corresponding to formulae (C), (D), (E), (F) and/or (G) below.

According to a first embodiment, the amino silicones corresponding to formula (B) are chosen from the silicones known as "trimethylsilyl amodimethicone" corresponding to formula (C):

[Chem. 21]

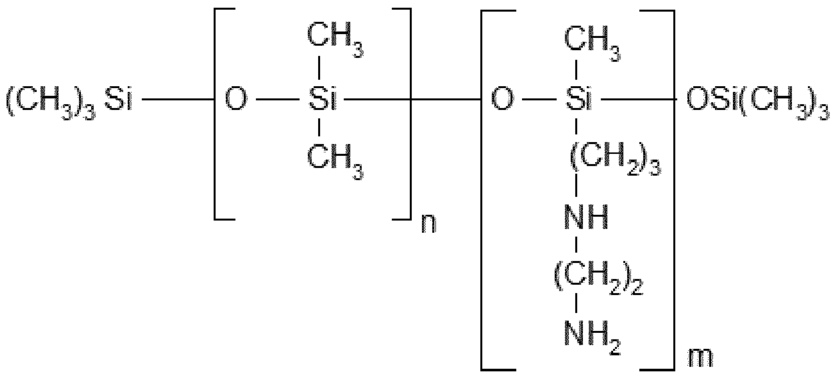

(C)

in which m and n are numbers such that the sum (n + m) ranges from 1 to 2000 and in particular from 50 to 150, it being possible for n to denote a number from 0 to 1999 and in particular from 49 to 149, and for m to denote a number from 1 to 2000 and in particular from 1 to 10.

According to a second embodiment, the amino silicones corresponding to formula (B) are chosen from the silicones of formula (D) below:

[Chem. 22]

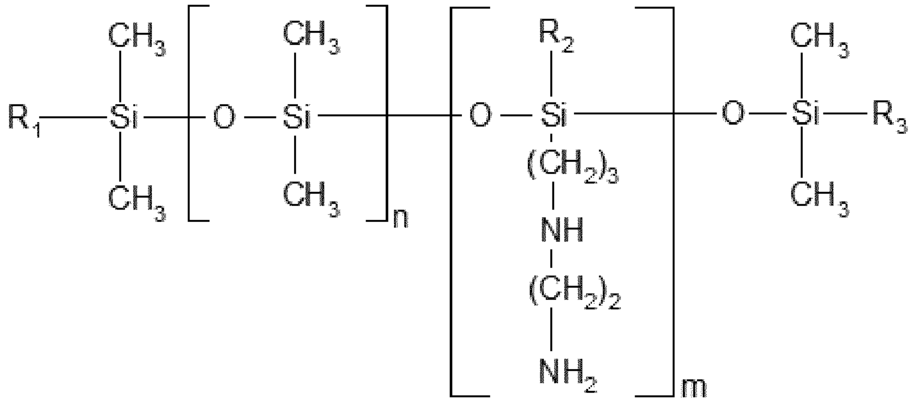

(D)

in which:

- m and n are numbers such that the sum (n + m) varies from 1 to 1000, notably from 50 to 250 and more particularly from 100 to 200, it being possible for n to denote a number from 0 to 999, in particular from 49 to 249 and more particularly from 125 to 175 and it being possible for m to denote a number from 1 to 1000, in particular from 1 to 10 and more particularly from 1 to 5;
- $R_1$, $R_2$ and $R_3$, which may be identical or different, represent a hydroxyl or $C_1$-$C_4$ alkoxy radical, at least one of the radicals $R_1$ to $R_3$ denoting an alkoxy radical.

Preferably, the alkoxy radical is a methoxy radical.

The hydroxy/alkoxy mole ratio preferably ranges from 0.2:1 to 0.4:1 and preferably from 0.25:1 to 0.35:1 and more particularly is equal to 0.3:1.

The weight-average molecular weight (Mw) of these silicones preferably ranges from 2000 to 1 000 000 and more particularly from 3500 to 200 000.

According to a third embodiment, the amino silicones corresponding to formula (B) are chosen from the silicones of formula (E) below:

[Chem. 23]

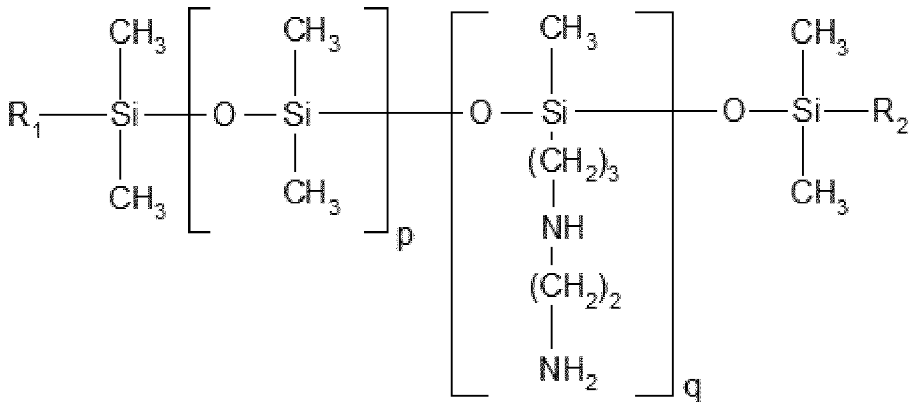

(E)

in which:

- m and n are numbers such that the sum (p + q) ranges from 1 to 1000, notably from 50 to 350 and more particularly from 150 to 250; p possibly denoting a number from 0 to 999 and in particular from 49 to 349 and more particularly from 159 to 239, and q possibly denoting a number from 1 to 1000, notably from 1 to 10 and more particularly from 1 to 5;
- $R_1$ and $R_2$, which may be different, represent a hydroxyl or $C_1$-$C_4$ alkoxy radical, at least one of the radicals $R_1$ or $R_2$ denoting an alkoxy radical.

Preferably, the alkoxy radical is a methoxy radical.

The hydroxy/alkoxy mole ratio generally ranges from 1:0.8 to 1:1.1 and preferably from 1:0.9 to 1:1 and more particularly is equal to 1:0.95.

The weight-average molecular mass (Mw) of the silicone preferably ranges from 2000 to 200 000, even more particularly from 5000 to 100 000 and more particularly from 10 000 to 50 000.

The commercial products comprising silicones of structure (D) or (E) may include in their composition one or more other amino silicones whose structure is different from formula (D) or (E).

A product containing amino silicones of structure (D) is sold by the company Wacker under the name Belsil® ADM 652.

A product containing amino silicones of structure (E) is sold by Wacker under the name Fluid WR 1300® or Belsil® ADM LOG 1.

When these amino silicones are used, one particularly advantageous embodiment consists in using them in the form of an oil-in-water emulsion. The oil-in-water emulsion may comprise one or more surfactants. The surfactants may be of any nature but are preferably cationic and/or nonionic. The number-mean size of the silicone particles in the emulsion generally ranges from 3 nm to 500 nanometers. Preferably, notably as amino silicones of formula (E), use is made of microemulsions with a mean particle size ranging from 5 nm to 60 nanometers (limits included) and more particularly from 10 nm to 50 nanometers (limits included). Thus, use may be made according to the invention of the amino silicone microemulsions of formula (E) sold under the names Finish CT 96 E® or SLM 28020® by the company Wacker.

According to a fourth embodiment, the amino silicones corresponding to formula (B) are chosen from the silicones of formula (F) below:

[Chem. 24]

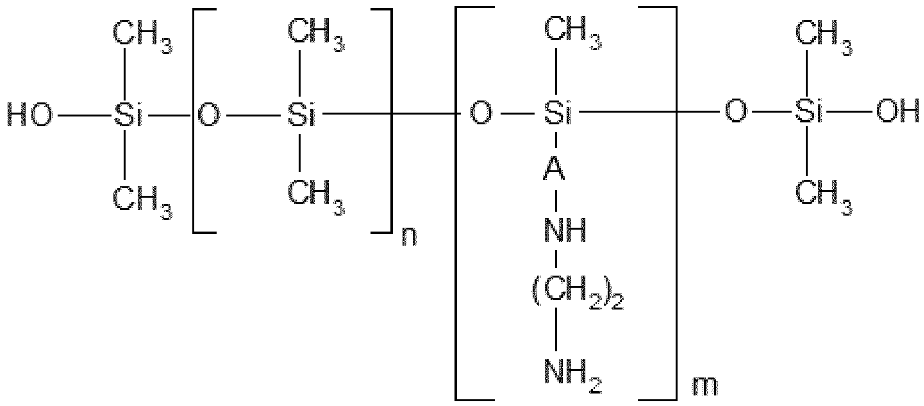

(F)

in which:

- m and n are numbers such that the sum (n + m) ranges from 1 to 2000 and in particular from 50 to 150, n possibly denoting a number from 0 to 1999 and notably from 49 to 149, and m possibly denoting a number from 1 to 2000 and notably from 1 to 10;
- A denotes a linear or branched alkylene radical containing from 4 to 8 carbon atoms and preferably 4 carbon atoms. This radical is preferably linear.

The weight-average molecular mass (Mw) of these amino silicones preferably ranges from 2000 to 1 000 000 and even more particularly from 3500 to 200 000.

Another silicone corresponding to formula (B) is, for example, the Xiameter MEM 8299 Emulsion from Dow Corning (INCI name: amodimethicone and trideceth-6 and cetrimonium chloride).

According to a fifth embodiment, the amino silicones corresponding to formula (B) are chosen from the silicones of formula (G) below:

[Chem. 25]

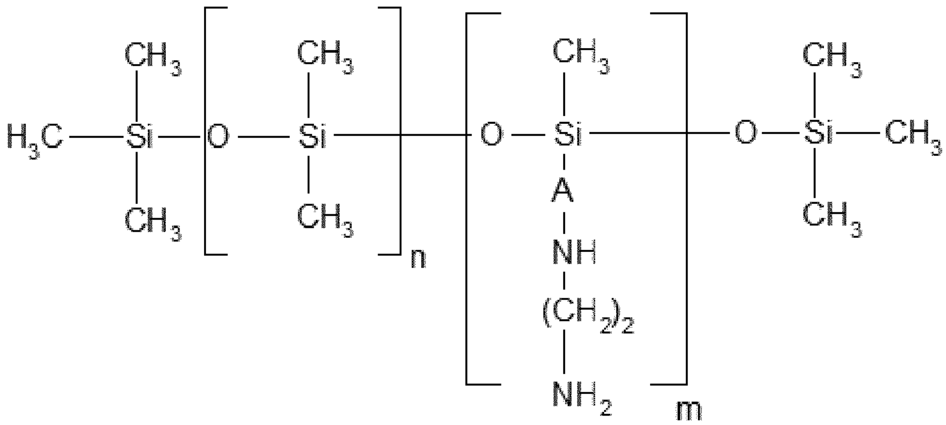

(G)

in which:

- m and n are numbers such that the sum (n + m) ranges from 1 to 2000 and in particular from 50 to 150, n possibly denoting a number from 0 to 1,999 and notably from 49 to 149, and m possibly denoting a number from 1 to 2000 and notably from 1 to 10;
- A denotes a linear or branched alkylene radical containing from 4 to 8 carbon atoms and preferably 4 carbon atoms. This radical is preferably branched.

The weight-average molecular mass (Mw) of these amino silicones preferably ranges from 500 to 1 000 000 and even more particularly from 1000 to 200 000.

A silicone corresponding to this formula is, for example, DC2-8566 Amino Fluid from Dow Corning;
c) the amino silicones corresponding to the formula (H):

[Chem. 26]

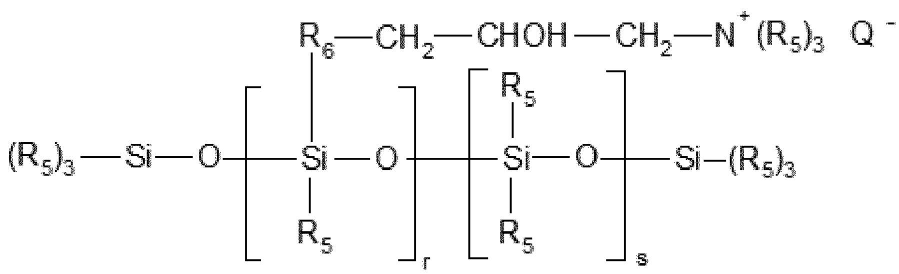

(H)

in which:

- R5 represents a monovalent hydrocarbon-based radical containing from 1 to 18 carbon atoms, and in particular a C1-C18 alkyl or C2-C18 alkenyl radical, for example methyl;
- R6 represents a divalent hydrocarbon-based radical, notably a C1-C18 alkylene radical or a divalent C1-C18, and for example C1-C8, alkylenoxy radical linked to the Si via an SiC bond;
- Q- is an anion, such as a halide ion, in particular a chloride ion, or an organic acid salt, in particular an acetate;
- r represents a mean statistical value ranging from 2 to 20 and in particular from 2 to 8;
- s represents a mean statistical value ranging from 20 to 200 and in particular from 20 to 50.

Such amino silicones are notably described in patent US 4 185 087.

- d) the quaternary ammonium silicones of formula (I):

[Chem. 27]

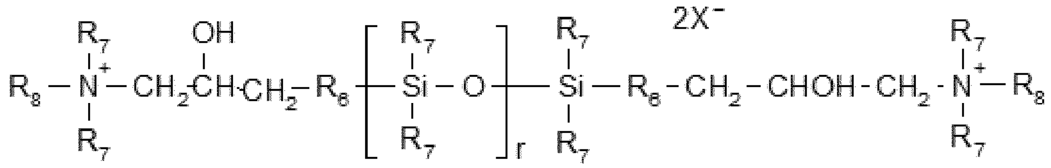

(I)

in which:

- $R_7$, which may be identical or different, represents a monovalent hydrocarbon-based radical containing from 1 to 18 carbon atoms, and in particular a $C_1$-$C_{18}$ alkyl radical, a C2-C18 alkenyl radical or a ring containing 5 or 6 carbon atoms, for example methyl;
- $R_6$ represents a divalent hydrocarbon-based radical, notably a $C_1$-$C_{18}$ alkylene radical or a divalent $C_1$-$C_{18}$, for example $C_1$-$C_8$, alkyleneoxy radical linked to the Si via an SiC bond;
- R8, which may be identical or different, represent a hydrogen atom, a monovalent hydrocarbon-based radical containing from 1 to 18 carbon atoms, and in particular a $C_1$-$C_{18}$ alkyl radical, a C2-C18 alkenyl radical or a -$R_6$-NHCOR$_7$ radical;
- X- is an anion, such as a halide ion, in particular a chloride iron, or an organic acid salt, in particular an acetate;
- r represents a mean statistical value ranging from 2 to 200 and in particular from 5 to 100.

These silicones are described, for example, in patent application EP-A 0 530 974.
e) the amino silicones of formula (J):

[Chem. 28]

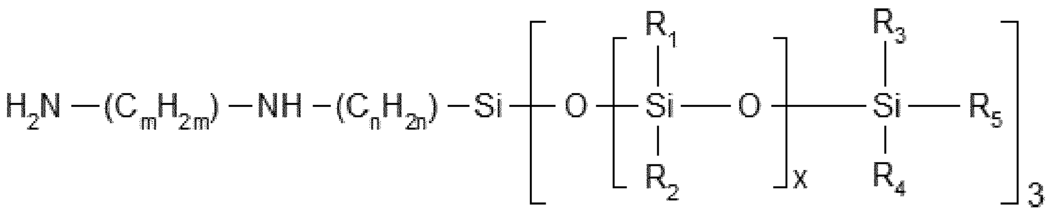

(J)

in which:

- $R_1$, $R_2$, $R_3$ and $R_4$, which may be identical or different, denote a $C_1$-$C_4$ alkyl radical or a phenyl group,
- $R_5$ denotes a $C_1$-$C_4$ alkyl radical or a hydroxyl group,
- n is an integer ranging from 1 to 5,
- m is an integer ranging from 1 to 5, and
- x is chosen such that the amine number ranges from 0.01 to 1 meq/g.

f) the multiblock polyoxyalkylenated amino silicones of type (AB)n, A being a polysiloxane block and B being a polyoxyalkylene block including at least one amine group.
Said silicones are preferably formed from repeating units having the following general formulae:

[Chem. 29] $[-(SiMe_2O)_xSiMe_2 - R -N(R'')- R'-O(C_2H_4O)_a(C_3H_6O)_b -R'-N(H)-R-]$

or alternatively

[Chem. 30] $[-(SiMe_2O)xSiMe_2 - R -N(R'')- R' - O(C_2H_4O)_a(C_3H_6O)_b -]$

in which:

- a is an integer greater than or equal to 1, preferably ranging from 5 to 200 and more particularly ranging from 10 to 100;
- b is an integer of between 0 and 200, preferably ranging from 4 to 100 and more particularly between 5 and 30;

- x is an integer ranging from 1 to 10 000 and more particularly from 10 to 5000;
- R" is a hydrogen atom or a methyl;
- R, which may be identical different, represent a linear or branched divalent $C_2$-$C_{12}$ hydrocarbon-based radical, optionally including one or more heteroatoms such as oxygen; preferably, R denotes an ethylene radical, a linear or branched propylene radical, a linear or branched butylene radical or a radical $CH_2CH_2CH_2OCH_2CH(OH)CH_2$-; preferentially, R denotes a radical $CH_2CH_2CH_2OCH_2CH(OH)CH_2$-;
- R', which may be identical or different, represent a linear or branched divalent $C_2$-$C_{12}$ hydrocarbon-based radical, optionally including one or more heteroatoms such as oxygen; preferably, R' denotes an ethylene radical, a linear or branched propylene radical, a linear or branched butylene radical or a radical $CH_2CH_2CH_2OCH_2CH(OH)CH_2$-; preferentially, R' denotes -$CH(CH_3)$-$CH_2$-.

The siloxane blocks preferably represent from 50 mol% to 95 mol% of the total weight of the silicone, more particularly from 70 mol% to 85 mol%.

The amine content is preferably between 0.02 and 0.5 meq/g of copolymer in a 30% solution in dipropylene glycol, more particularly between 0.05 and 0.2.

The weight-average molecular mass (Mw) of the silicone is preferably between 5000 and 1 000 000 and more particularly between 10 000 and 200 000.

Mention may in particular be made of the silicones sold under the name Silsoft A-843 or Silsoft A+ by Momentive.

g) and mixtures thereof.

Preferably, the amino silicones of formula (B) are chosen from the amino silicones corresponding to formula (E).

Preferably, the composition according to the invention comprises at least one amino silicone having the INCI name amodimethicone, preferably introduced in the form of an emulsion or microemulsion with surfactants.

Preferably, the composition according to the invention comprises at least one amino silicone having the INCI name amodimethicone as an emulsion or microemulsion with surfactants, having the INCI names trideceth-5 and trideceth-10.

The silicone(s) may be present in a total amount ranging from 0.01% to 20% by weight relative to the total weight of the composition, preferably from 0.05% to 15% by weight, more preferentially from 0.1% to 10% by weight, more preferentially from 0.1% to 5% by weight relative to the total weight of composition (C).

When composition (C) comprises one or more amino silicones, the total amount of amino silicone(s) may range from 0.01% to 20% by weight, preferably from 0.05% to 15% by weight, better still from 0.1% to 10% by weight and more preferentially from 0.1% to 5% by weight relative to the total weight of composition (C).

**Coloring agent:**

Composition (C) according to the invention comprises at least one coloring agent chosen from pigments, direct dyes and mixtures thereof.

Preferably, composition (C) according to the invention comprises one or more pigments.

The term "pigment" means any pigment that gives color to keratin materials. Their solubility in water at 25°C and at atmospheric pressure (760 mmHg) is less than 0.05% by weight, and preferably less than 0.01%.

The pigments that may be used are notably chosen from the organic and/or mineral pigments known in the art, notably those described in Kirk-Othmer's Encyclopedia of Chemical Technology and in Ullmann's Encyclopedia of Industrial Chemistry.

They may be natural, of natural origin, or non-natural.

These pigments may be in pigment powder or paste form. They may be coated or uncoated.

The pigments may be chosen, for example, from mineral pigments, organic pigments, lacquers, pigments with special effects such as nacres or glitter flakes, and mixtures thereof.

The pigment may be a mineral pigment. The term "mineral pigment" refers to any pigment that satisfies the definition in Ullmann's encyclopedia in the chapter on inorganic pigments. Among the mineral pigments that are useful in the present invention, mention may be made of iron oxides, chromium oxides, manganese violet, ultramarine blue, chromium hydrate, ferric blue and titanium oxide.

The pigment may be an organic pigment. The term "organic pigment" refers to any pigment that satisfies the definition in Ullmann's encyclopedia in the chapter on organic pigments.

The organic pigment may notably be chosen from nitroso, nitro, azo, xanthene, pyrene, quinoline, anthraquinone, triphenylmethane, fluorane, phthalocyanine, metal-complex, isoindolinone, isoindoline, quinacridone, perinone, perylene, diketopyrrolopyrrole, indigo, thioindigo, dioxazine, triphenylmethane and quinophthalone compounds.

In particular, the white or colored organic pigments may be chosen from carmine, carbon black, aniline black, azo

yellow, quinacridone, phthalocyanine blue, the blue pigments codified in the Color Index under the references CI 42090, 69800, 69825, 74100, 74160, the yellow pigments codified in the Color Index under the references CI 11680, 11710, 19140, 20040, 21100, 21108, 47000, 47005, the green pigments codified in the Color Index under the references CI 61565, 61570, 74260, the orange pigments codified in the Color Index under the references CI 11725, 45370, 71105, the red pigments codified in the Color Index under the references CI 12085, 12120, 12370, 12420, 12490, 14700, 15525, 15580, 15620, 15630, 15800, 15850, 15865, 15880, 26100, 45380, 45410, 58000, 73360, 73915, 75470, the pigments obtained by oxidative polymerization of indole or phenol derivatives as described in patent FR 2 679 771.

Examples that may also be mentioned include pigment pastes of organic pigments, such as the products sold by the company Hoechst under the names:

- Cosmenyl Yellow IOG: Yellow 3 pigment (CI 11710);
- Cosmenyl Yellow G: Yellow 1 pigment (CI 11680);
- Cosmenyl Orange GR: Orange 43 pigment (CI 71105);
- Cosmenyl Red R: Red 4 pigment (CI 12085);
- Cosmenyl Carmine FB: Red 5 pigment (CI 12490);
- Cosmenyl Violet RL: Violet 23 pigment (CI 51319);
- Cosmenyl Blue A2R: Blue 15.1 pigment (CI 74160);
- Cosmenyl Green GG: Green 7 pigment (CI 74260);
- Cosmenyl Black R: Black 7 pigment (CI 77266).

The pigments in accordance with the invention may also be in the form of composite pigments, as described in patent EP 1 184 426. These composite pigments may be composed notably of particles including a mineral core, at least one binder, for attaching the organic pigments to the core, and at least one organic pigment which at least partially covers the core.

The organic pigment may also be a lake. The term "lake" refers to dyes adsorbed onto insoluble particles, the assembly thus obtained remaining insoluble during use.

The inorganic substrates onto which the dyes are adsorbed are, for example, alumina, silica, calcium sodium borosilicate, calcium aluminum borosilicate and aluminum.

Among the dyes, mention may be made of carminic acid. Mention may also be made of the dyes known under the following names: D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 1 O (CI 77 002), D&C Green 3 (CI 42 053), D&C Blue 1 (CI 42 090).

An example of a lake that may be mentioned is the product known under the following name: D&C Red 7 (CI 15 850: 1).

The pigment may also be a pigment with special effects. The term "pigments with special effects" means pigments that generally create a colored appearance (characterized by a certain shade, a certain vivacity and a certain level of luminance) that is non-uniform and that changes as a function of the conditions of observation (light, temperature, angles of observation, etc.). They thereby differ from colored pigments, which afford a standard uniform opaque, semi-transparent or transparent shade.

Several types of pigments with special effects exist: those with a low refractive index, such as fluorescent or photochromic pigments, and those with a higher refractive index, such as nacres, interference pigments or glitter flakes.

Examples of pigments with special effects that may be mentioned include nacreous pigments such as mica coated with titanium or with bismuth oxychloride, colored nacreous pigments such as titanium mica with iron oxides, mica covered with iron oxide, titanium mica notably with ferric blue or with chromium oxide, titanium mica with an organic pigment of the abovementioned type, and also nacreous pigments based on bismuth oxychloride. Nacreous pigments that may be mentioned include the Cellini nacres sold by BASF (mica-TiO2-lake), Prestige sold by Eckart (mica-TiO2), Prestige Bronze sold by Eckart (mica-Fe2O3), and Colorona sold by Merck (mica-TiO2-Fe2O3).

Mention may also be made of the gold-colored nacres sold notably by the company BASF under the name Brilliant gold 212G (Timica), Gold 222C (Cloisonne), Sparkle gold (Timica), Gold 4504 (Chromalite) and Monarch gold 233X (Cloisonne); the bronze nacres sold notably by the company Merck under the name Bronze fine (17384) (Colorona) and Bronze (17353) (Colorona) and by the company BASF under the name Super bronze (Cloisonne); the orange nacres sold notably by the company BASF under the name Orange 363C (Cloisonne) and Orange MCR 101 (Cosmica) and by the company Merck under the name Passion orange (Colorona) and Matte orange (17449) (Microna); the brown nacres sold notably by the company BASF under the name Nu-antique copper 340XB (Cloisonne) and Brown CL4509 (Chromalite); the nacres with a copper tint sold notably by the company BASF under the name Copper 340A (Timica); the nacres with a red tint sold notably by the company Merck under the name Sienna fine (17386) (Colorona); the nacres with a yellow tint sold notably by the company BASF under the name Yellow (4502) (Chromalite); the red nacres with a gold tint sold notably by the company BASF under the name Sunstone G012 (Gemtone); the pink nacres sold notably by the company BASF

under the name Tan opale G005 (Gemtone); the black nacres with a gold tint sold notably by the company BASF under the name Nu antique bronze 240 AB (Timica), the blue nacres sold notably by the company Merck under the name Matte blue (17433) (Microna), the white nacres with a silvery tint sold notably by the company Merck under the name Xirona Silver, and the golden-green pink-orange nacres sold notably by the company Merck under the name Indian summer (Xirona), and mixtures thereof.

Still as examples of nacres, mention may also be made of particles including a borosilicate substrate coated with titanium oxide.

Particles comprising a glass substrate coated with titanium oxide are notably sold under the name Metashine MC1080RY by the company Toyal.

Finally, examples of nacres that may also be mentioned include polyethylene terephthalate flakes, notably those sold by the company Meadowbrook Inventions under the name Silver 1P 0.004X0.004 (silver flakes). Multilayer pigments based on synthetic substrates such as alumina, silica, sodium calcium borosilicate or calcium aluminum borosilicate, and aluminum, may also be envisaged.

The pigments with special effects may also be chosen from reflective particles, i.e. notably from particles whose size, structure, notably the thickness of the layer(s) of which they are made and their physical and chemical nature, and surface state, allow them to reflect incident light. This reflection may, where appropriate, have an intensity sufficient to create at the surface of the composition or of the mixture, when it is applied to the support to be made up, highlight points that are visible to the naked eye, i.e. more luminous points that contrast with their environment by appearing to sparkle.

The reflective particles may be selected so as not to significantly alter the coloring effect generated by the coloring agents with which they are combined, and more particularly so as to optimize this effect in terms of color rendition. They may more particularly have a yellow, pink, red, bronze, orange, brown, gold and/or coppery color or tint.

These particles may have varied forms and may notably be in platelet or globular form, in particular in spherical form.

Irrespective of their form, the reflective particles may or may not have a multilayer structure, and, in the case of a multilayer structure, may have, for example, at least one layer of uniform thickness, notably of a reflective material.

When the reflective particles do not have a multilayer structure, they may be composed, for example, of metal oxides, notably titanium or iron oxides obtained synthetically.

When the reflective particles have a multilayer structure, they may include, for example, a natural or synthetic substrate, notably a synthetic substrate at least partially coated with at least one layer of a reflective material, notably of at least one metal or metallic material. The substrate may be made of one or more organic and/or mineral materials.

More particularly, it may be chosen from glasses, ceramics, graphite, metal oxides, aluminas, silicas, silicates, notably aluminosilicates and borosilicates, and synthetic mica, and mixtures thereof, this list not being limiting.

The reflective material may include a layer of metal or of a metallic material.

Reflective particles are notably described in JP-A-09188830, JP-A-10158450, JP-A-10158541, JP-A-07258460 and JP-A-05017710.

Still as an example of reflective particles including a mineral substrate coated with a layer of metal, mention may also be made of particles including a silver-coated borosilicate substrate.

Particles with a silver-coated glass substrate, in the form of platelets, are sold under the name Microglass Metashine REFSX 2025 PS by the company Toyal. Particles with a glass substrate coated with nickel/chromium/molybdenum alloy are sold under the names Crystal Star GF 550 and GF 2525 by this same company.

Use may also be made of particles comprising a metallic substrate such as silver, aluminum, iron, chromium, nickel, molybdenum, gold, copper, zinc, tin, manganese, steel, bronze or titanium, said substrate being coated with at least one layer of at least one metal oxide such as titanium oxide, aluminum oxide, iron oxide, cerium oxide, chromium oxide or silicon oxides, and mixtures thereof.

Examples that may be mentioned include aluminum powder, bronze powder or copper powder coated with $SiO_2$ sold under the name Visionaire by the company Eckart.

Mention may also be made of pigments with an interference effect which are not attached to a substrate, such as liquid crystals (Helicones HC from Wacker) or interference holographic glitter flakes (Geometric Pigments or Spectra f/x from Spectratek). Pigments with special effects also comprise fluorescent pigments, whether these are substances that are fluorescent in daylight or that produce an ultraviolet fluorescence, phosphorescent pigments, photochromic pigments, thermochromic pigments and quantum dots, sold, for example, by the company Quantum Dots Corporation.

The variety of pigments that may be used in the present invention makes it possible to obtain a wide range of colors, and also particular optical effects such as metallic effects or interference effects.

The size of the pigment used in the composition according to the present invention is generally between 10 nm and 200 $\mu$m, preferably between 20 nm and 80 $\mu$m and more preferentially between 30 nm and 50 $\mu$m.

The pigments may be dispersed in the composition by means of a dispersant.

The dispersant serves to protect the dispersed particles against their agglomeration or flocculation. This dispersant may be a surfactant, an oligomer, a polymer or a mixture of several thereof, bearing one or more functionalities

with strong affinity for the surface of the particles to be dispersed. In particular, they may become physically or chemically attached to the surface of the pigments. These dispersants also contain at least one functional group that is compatible with or soluble in the continuous medium. In particular, 12-hydroxystearic acid esters and $C_8$ to $C_{20}$ fatty acid esters of polyols such as glycerol or diglycerol are used, such as poly(12-hydroxystearic acid) stearate with a molecular weight of approximately 750 g/mol, such as the product sold under the name Solsperse 21 000 by the company Avecia, polyglyceryl-2 dipolyhydroxystearate (CTFA name) sold under the reference Dehymyls PGPH by the company Henkel, or polyhydroxystearic acid such as the product sold under the reference Arlacel P100 by the company Uniqema, and mixtures thereof.

As other dispersants that may be used in the compositions of the invention, mention may be made of quaternary ammonium derivatives of polycondensed fatty acids, for instance Solsperse 17 000 sold by the company Avecia, and polydimethylsiloxane/oxypropylene mixtures such as those sold by the company Dow Corning under the references DC2-5185 and DC2-5225 C.

The pigments used in the composition may be surface-treated with an organic agent.

Thus, the pigments that have been surface-treated beforehand, which are useful in the context of the invention, are pigments that have totally or partially undergone a surface treatment of chemical, electronic, electrochemical, mechanochemical or mechanical nature, with an organic agent such as those described notably in Cosmetics and Toiletries, February 1990, Vol. 105, pages 53-64, before being dispersed in the composition in accordance with the invention. These organic agents may be chosen, for example, from waxes, for example carnauba wax and beeswax; fatty acids, fatty alcohols and derivatives thereof, such as stearic acid, hydroxystearic acid, stearyl alcohol, hydroxystearyl alcohol and lauric acid and derivatives thereof; anionic surfactants; lecithins; sodium, potassium, magnesium, iron, titanium, zinc or aluminum salts of fatty acids, for example aluminum stearate or laurate; metal alkoxides; polyethylene; (meth)acrylic polymers, for example polymethyl methacrylates; polymers and copolymers containing acrylate units; alkanolamines; silicone compounds, for example silicones, in particular polydimethylsiloxanes; organofluorine compounds, for example perfluoroalkyl ethers; fluorosilicone compounds.

The surface-treated pigments that are useful in the composition may also have been treated with a mixture of these compounds and/or may have undergone several surface treatments.

The surface-treated pigments that are useful in the context of the present invention may be prepared according to surface-treatment techniques that are well known to those skilled in the art, or may be commercially available as is.

Preferably, the surface-treated pigments are coated with an organic layer.

The organic agent with which the pigments are treated may be deposited on the pigments by solvent evaporation, chemical reaction between the molecules of the surface agent or creation of a covalent bond between the surface agent and the pigments.

The surface treatment may thus be performed, for example, by chemical reaction of a surface agent with the surface of the pigments and creation of a covalent bond between the surface agent and the pigments or the fillers. This method is notably described in patent US 4 578 266.

An organic agent covalently bonded to the pigments will preferably be used.

The agent for the surface treatment may represent from 0.1% to 50% by weight relative to the total weight of the surface-treated pigment, preferably from 0.5% to 30% by weight and even more preferentially from 1% to 20% by weight relative to the total weight of the surface-treated pigment.

Preferably, the surface treatments of the pigments are chosen from the following treatments:

- a PEG-silicone treatment, for instance the AQ surface treatment sold by LCW;
- a methicone treatment, for instance the SI surface treatment sold by LCW;
- a dimethicone treatment, for instance the Covasil 3.05 surface treatment sold by LCW;
- a dimethicone/trimethyl siloxysilicate treatment, for instance the Covasil 4.05 surface treatment sold by LCW;
- a magnesium myristate treatment, for instance the MM surface treatment sold by LCW;
- an aluminum dimyristate treatment, such as the MI surface treatment sold by Miyoshi;
- a perfluoropolymethylisopropyl ether treatment, for instance the FHC surface treatment sold by LCW;
- an isostearyl sebacate treatment, for instance the HS surface treatment sold by Miyoshi;
- a perfluoroalkyl phosphate treatment, for instance the PF surface treatment sold by Daito;
- an acrylate/dimethicone copolymer and perfluoroalkyl phosphate treatment, for instance the FSA surface treatment sold by Daito;
- a polymethylhydrosiloxane/perfluoroalkyl phosphate treatment, for instance the FS01 surface treatment sold by Daito;
- an acrylate/dimethicone copolymer treatment, for instance the ASC surface treatment sold by Daito;
- an isopropyl titanium triisostearate treatment, for instance the ITT surface treatment sold by Daito;
- an acrylate copolymer treatment, for instance the APD surface treatment sold by Daito;
- a perfluoroalkyl phosphate/isopropyl titanium triisostearate treatment, for instance the PF + ITT surface treatment sold

by Daito.

According to a particular embodiment of the invention, the dispersant is present with organic or mineral pigments in submicron-sized particulate form in the dye composition.

The term "submicron" or "submicronic" refers to pigments having a particle size that has been micronized by a micronization method and having a mean particle size of less than a micrometer ($\mu$m), in particular between 0.1 and 0.9 $\mu$m, and preferably between 0.2 and 0.6 $\mu$m.

According to one embodiment, the dispersant and the pigment(s) are present in an amount (dispersant:pigment) of between 1:4 and 4:1, particularly between 1.5:3.5 and 3.5:1 or better still between 1.75:3 and 3:1.

The dispersant(s) may therefore have a silicone backbone, such as silicone polyether and dispersants of aminosilicone type other than the silicones described previously. Among the suitable dispersants, mention may be made of:

- aminosilicones, i.e. silicones comprising one or more amino groups such as those sold under the names and references: BYK LPX 21879 by BYK, GP-4, GP-6, GP-344, GP-851, GP-965, GP-967 and GP-988-1, sold by Genesee Polymers,
- silicone acrylates such as Tego® RC 902, Tego® RC 922, Tego® RC 1041, and Tego® RC 1043, sold by Evonik,
- polydimethylsiloxane (PDMS) silicones with carboxyl groups such as X- 22162 and X-22370 by Shin-Etsu, epoxy silicones such as GP-29, GP-32, GP-502, GP-504, GP-514, GP-607, GP-682, and GP-695 by Genesee Polymers, or Tego® RC 1401, Tego® RC 1403, Tego® RC 1412 by Evonik.

According to a particular embodiment, the dispersant(s) are of amino silicone type other than the silicones described previously and are cationic.

Preferably, the pigment(s) are chosen from mineral, mixed mineral-organic or organic pigments.

In one variant of the invention, the pigment(s) according to the invention are organic pigments, preferentially organic pigments surface-treated with an organic agent chosen from silicone compounds. In another variant of the invention, the pigment(s) according to the invention are mineral pigments.

Composition (C) may comprise one or more direct dyes.

The term "direct dye" means natural and/or synthetic dyes, other than oxidation dyes. These are dyes that will spread superficially on the fiber.

They may be ionic or nonionic, preferably cationic or nonionic.

Examples of suitable direct dyes that may be mentioned include azo direct dyes; (poly)methine dyes such as cyanines, hemicyanines and styryls; carbonyl dyes; azine dyes; nitro(hetero)aryl dyes; tri(hetero)arylmethane dyes; porphyrin dyes; phthalocyanine dyes and natural direct dyes, alone or in the form of mixtures.

The direct dyes are preferably cationic direct dyes. Mention may be made of the hydrazono cationic dyes of formulae (XIII) and (XIV) and the azo cationic dyes (XV) and (XVI) below:
[Chem. 31]

$$Hét^{+}\text{-}C(Ra)=N\text{-}N(Rb)\text{-}Ar, Q \qquad (XIII)$$

[Chem. 32]

$$Hét^{+}\text{-}N(Ra)\text{-}N=C(Rb)\text{-}Ar, Q\text{-} \qquad (XIV)$$

[Chem. 33]

$$Hét^{+}\text{-}N=N\text{-}Ar, Q\text{-} \qquad (XV)$$

[Chem. 34]

$$Ar^{+}\text{-}N=N\text{-}Ar'', Q\text{-} \qquad (XVI)$$

in which formulae (XIII) to (XVI):

- **Het⁺** represents a cationic heteroaryl radical, preferentially bearing an endocyclic cationic charge, such as imidazolium, indolium or pyridinium, which is optionally substituted, preferentially with at least one ($C_1$-$C_8$) alkyl group such as methyl;
- **Ar⁺** represents an aryl radical, such as phenyl or naphthyl, bearing an exocyclic cationic charge, preferentially

ammonium, particularly tri($C_1$-$C_8$)alkylammonium, such as trimethylammonium;

- **Ar** represents an aryl group, notably phenyl, which is optionally substituted, preferentially with one or more electron-donating groups such as i) optionally substituted ($C_1$-$C_8$)alkyl, ii) optionally substituted ($C_1$-$C_8$)alkoxy, iii) (di)($C_1$-$C_8$)(alkyl)amino optionally substituted on the alkyl group(s) with a hydroxyl group, iv) aryl($C_1$-$C_8$)alkylamino, v) optionally substituted N-($C_1$-$C_8$)alkyl-N-aryl($C_1$-$C_8$)alkylamino or alternatively Ar represents a julolidine group;
- **Ar"** represents an optionally substituted (hetero)aryl group, such as phenyl or pyrazolyl, which are optionally substituted, preferentially with one or more ($C_1$-$C_8$)alkyl, hydroxyl, (di)($C_1$-$C_8$)(alkyl)amino, ($C_1$-$C_8$)alkoxy or phenyl groups;
- **Ra** and **Rb,** which may be identical or different, represent a hydrogen atom or a ($C_1$-$C_8$)alkyl group, which is optionally substituted, preferentially with a hydroxyl group; or else the substituent Ra with a substituent of Het+ and/or Rb with a substituent of Ar form, together with the atoms that bear them, a (hetero)cycloalkyl; in particular, Ra and Rb represent a hydrogen atom or a ($C_1$-$C_4$)alkyl group optionally substituted with a hydroxyl group;
- **Q-** represents an organic or mineral anionic counterion, such as a halide or an alkyl sulfate.

In particular, mention may be made of the azo and hydrazono direct dyes bearing an endocyclic cationic charge of formulae (XIII) to (XVI) as defined previously, more particularly, the cationic direct dyes bearing an endocyclic cationic charge described in patent applications WO 95/15144, WO 95/01772 and EP 714 954, preferentially the following direct dyes:

[Chem. 35]

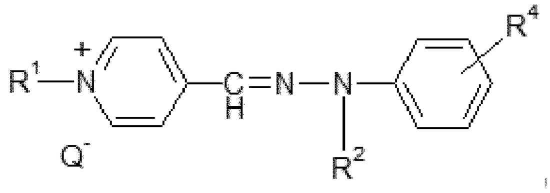

(XVII)

[Chem. 36]

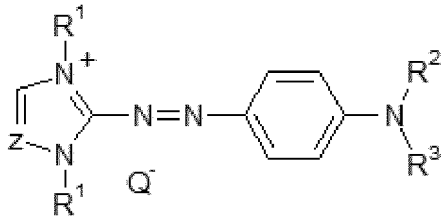

(XVIII)

in which formulae (XVII) and (XVIII):

- **R¹** represents a ($C_1$-$C_4$)alkyl group such as methyl;
- **R²** and **R³,** which may be identical or different, represent a hydrogen atom or a ($C_1$-$C_4$)alkyl group, such as methyl; and
- **R⁴** represents a hydrogen atom or an electron-donating group such as optionally substituted ($C_1$-$C_8$)alkyl, optionally substituted ($C_1$-$C_8$)alkoxy, or (di)($C_1$-$C_8$)(alkyl)amino optionally substituted on the alkyl group(s) with a hydroxyl group; particularly, $R^4$ is a hydrogen atom,
- **Z** represents a CH group or a nitrogen atom, preferentially CH,
- **Q-** is an anionic counterion as defined previously, in particular a halide, such as chloride, or an alkyl sulfate, such as methyl sulfate or mesityl;

In particular, the dyes of formulae (XVII) and (XVIII) are chosen from Basic Red 51, Basic Yellow 87 and Basic Orange 31 or derivatives thereof with Q' being an anionic counterion as defined previously, particularly halide such as chloride, or an alkyl sulfate such as methyl sulfate or mesityl.

The direct dyes may be chosen from anionic direct dyes. The anionic direct dyes of the invention are dyes commonly referred to as "acid" direct dyes owing to their affinity for alkaline substances. The term "anionic direct dye"

means any direct dye including in its structure at least one $CO_2R$ or $SO_3R$ substituent with R denoting a hydrogen atom or a cation originating from a metal or an amine, or an ammonium ion. The anionic dyes may be chosen from direct nitro acid dyes, azo acid dyes, azine acid dyes, triarylmethane acid dyes, indoamine acid dyes, anthraquinone acid dyes, indigoid dyes and natural acid dyes.

As acid dyes according to the invention, mention may be made of the dyes of formulae (XIX), (XIX'), (XX), (XX'), (XXI), (XXI'), (XXII), (XXII'), (XXIII), (XXIV), (XXV) and (XXVI) below:

a) the diaryl anionic azo dyes of formula (XIX) or (XIX'):

[Chem. 37]

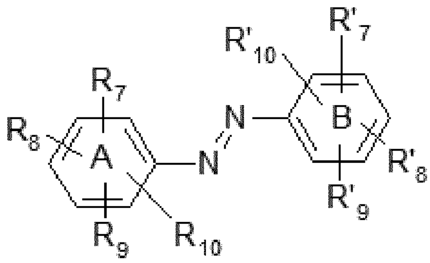

(XIX)

[Chem. 38]

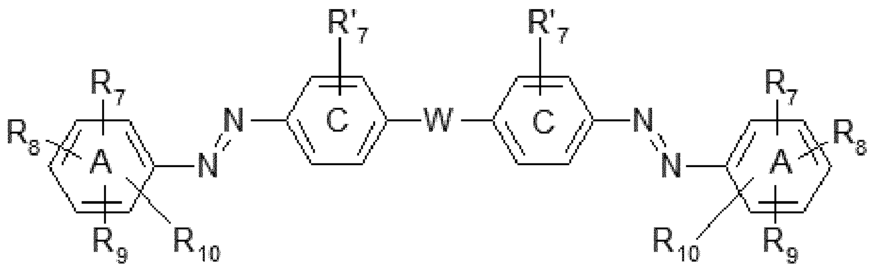

(XIX')

in which formulae (XIX) and (XIX'):

- **$R_7$, $R_8$, $R_9$, $R_{10}$, $R'_7$, $R'_8$, $R'_9$** and **$R'_{10}$**, which may be identical or different, represent a hydrogen atom or a group chosen from:
- alkyl;
- alkoxy, alkylthio;
- hydroxyl, mercapto;
- nitro, nitroso;
- R°-C(X)-X'-, R°-X'-C(X)-, R°-X'-C(X)-X''- with R° representing a hydrogen atom or an alkyl or aryl group; X, X' and X'', which may be identical or different, representing an oxygen or sulfur atom, or NR with R representing a hydrogen atom or an alkyl group;
- $(O)_2S(O$-)-, M+ with M+ representing a hydrogen atom or a cationic counterion;
- (O)CO$^-$-, $M^+$ with $M^+$ as defined previously;
- R'' -$S(O)_2$-, with R'' representing a hydrogen atom or an alkyl, aryl, (di)(alkyl)amino or aryl(alkyl)amino group; preferentially a phenylamino or phenyl group;
- R'''-$S(O)_2$-X'- with R''' representing an alkyl or optionally substituted aryl group, X' as defined previously;
- (di)(alkyl)amino;
- aryl(alkyl)amino optionally substituted with one or more groups chosen from i) nitro; ii) nitroso; iii) (O)2S(O-)-, M+ and iv) alkoxy with M+ as defined previously;
- optionally substituted heteroaryl; preferentially a benzothiazolyl group;
- cycloalkyl; notably cyclohexyl;

- Ar-N=N- with Ar representing an optionally substituted aryl group, preferentially a phenyl optionally substituted with one or more alkyl, (O)2S(O-)-, M+ or phenylamino groups;
- or alternatively two contiguous groups $R_7$ with $R_8$ or $R_8$ with $R_9$ or $R_9$ with $R_{10}$ together form a fused benzo group A'; and $R'_7$ with $R'_8$ or $R'_8$ with $R'_9$ or $R'_9$ with $R'_{10}$ together form a fused benzo group B'; with A' and B' optionally substituted with one or more groups chosen from i) nitro; ii) nitroso; iii) $(O)_2S(O\text{-})\text{-}$, M+; iv) hydroxyl; v) mercapto; vi) (di)(alkyl)amino; vii) R°-C(X)-X'-; viii) R°-X'-C(X)-; ix) R°-X'-C(X)-X''-; x) Ar-N=N- and xi) optionally substituted aryl(alkyl)amino; with M+, R°, X, X', X'' and Ar as defined previously;
- **W** represents a sigma bond $\sigma$, an oxygen or sulfur atom, or a divalent radical i) - NR- with R as defined previously, or ii) methylene -C(Ra)(Rb)- with Ra and Rb, which may be identical or different, representing a hydrogen atom or an aryl group, or alternatively Ra and Rb form, with the carbon atom that bears them, a spiro cycloalkyl; preferentially, W represents a sulfur atom or Ra and Rb together form a cyclohexyl;

it being understood that formulae (XIX) and (XIX') comprise at least one sulfonate radical $(O)_2S(O\text{-})\text{-}$, M+ or one carboxylate radical (O)CO--, M+ on one of the rings A, A', B, B' or C; preferentially sodium sulfonate.

As examples of dyes of formula (XIX), mention may be made of: Acid Red 1, Acid Red 4, Acid Red 13, Acid Red 14, Acid Red 18, Acid Red 27, Acid Red 28, Acid Red 32, Acid Red 33, Acid Red 35, Acid Red 37, Acid Red 40, Acid Red 41, Acid Red 42, Acid Red 44, Pigment Red 57, Acid Red 68, Acid Red 73, Acid Red 135, Acid Red 138, Acid Red 184, Food Red 1, Food Red 13, Acid Orange 6, Acid Orange 7, Acid Orange 10, Acid Orange 19, Acid Orange 20, Acid Orange 24, Yellow 6, Acid Yellow 9, Acid Yellow 36, Acid Yellow 199, Food Yellow 3, Acid Violet 7, Acid Violet 14, Acid Blue 113, Acid Blue 117, Acid Black 1, Acid Brown 4, Acid Brown 20, Acid Black 26, Acid Black 52, Food Black 1, Food Black 2, Food yellow 3 or sunset yellow;

and, as examples of dyes of formula (XIX'), mention may be made of: Acid Red 111, Acid Red 134, Acid yellow 38.

b) the pyrazolone anionic azo dyes of formulae (XX) and (XX'):

[Chem. 39]

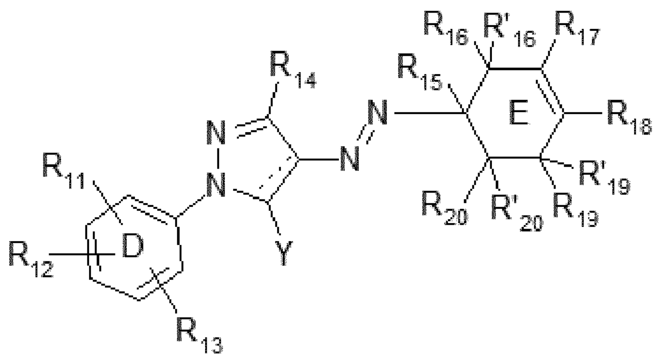

(XX)

[Chem. 40]

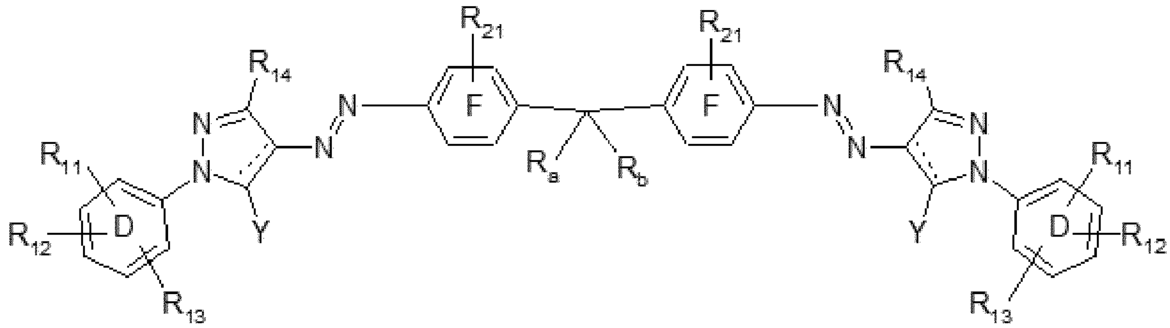

(XX')

in which formulae (XX) and (XX'):

- **$R_{11}$, $R_{12}$** and **$R_{13}$,** which may be identical or different, represent a hydrogen or halogen atom, an alkyl group or $-(O)_2S(O-)$, M+ with M+ as defined previously;
- **$R_{14}$** represents a hydrogen atom, an alkyl group or a group -C(O)O-, M+ with M+ as defined previously;
- **$R_{15}$** represents a hydrogen atom;
- **$R_{16}$** represents an oxo group, in which case $R'_{16}$ is absent, or alternatively **$R_{15}$** with **$R_{16}$** together form a double bond;
- **$R_{17}$** and **$R_{18}$,** which may be identical or different, represent a hydrogen atom, or a group chosen from:
  - $(O)_2S(O-)-$, M+ with M+ as defined previously;
  - Ar-O-$S(O)_2$- with Ar representing an optionally substituted aryl group; preferentially a phenyl optionally substituted with one or more alkyl groups;
  - **$R_{19}$** and **$R_{20}$** together form either a double bond, or a benzo group D', which is optionally substituted;
  - **$R'_{16}$**, **$R'_{19}$** and **$R'_{20}$,** which may be identical or different, represent a hydrogen atom or an alkyl or hydroxyl group;
  - **$R_{21}$** represents a hydrogen atom or an alkyl or alkoxy group;
  - **$R_a$** and **$R_b$,** which may be identical or different, are as defined previously, preferentially $R_a$ represents a hydrogen atom and $R_b$ represents an aryl group;
  - **Y** represents either a hydroxyl group or an oxo group;
  - - - - - represents a single bond when Y is an oxo group; and represents a double bond when Y represents a hydroxyl group;

it being understood that formulae (XX) and (XX') comprise at least one sulfonate radical $(O)_2S(O-)-$, M+ or one carboxylate radical -C(O)O-, M+ on one of the rings D or E; preferentially sodium sulfonate.

As examples of dyes of formula (XX), mention may be made of: Acid Red 195, Acid Yellow 23, Acid Yellow 27, Acid Yellow 76, and as examples of dyes of formula (XX'), mention may be made of: Acid Yellow 17;

c) the anthraquinone dyes of formulae (XXI) and (XXI'):

[Chem. 41]

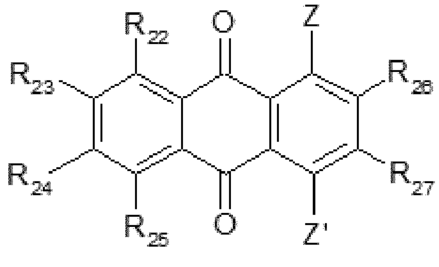

(XXI)

[Chem. 42]

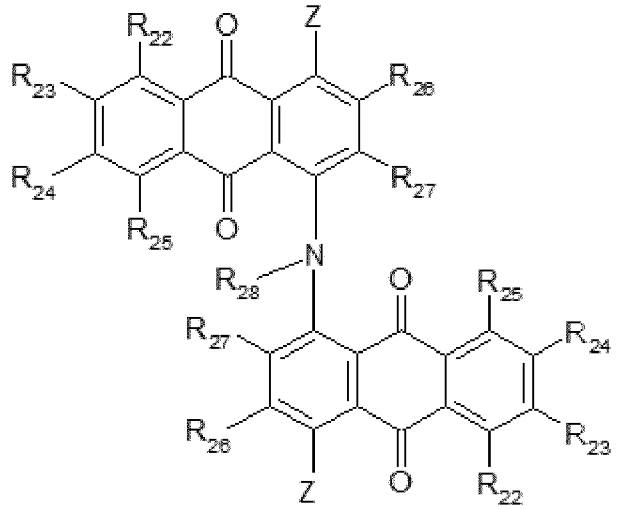

(XXI')

in which formulae (XXI) and (XXI'):

- $R_{22}$, $R_{23}$, $R_{24}$, $R_{25}$, $R_{26}$ and $R_{27}$, which may be identical or different, represent a hydrogen or halogen atom, or a group chosen from:
- alkyl;
- hydroxyl, mercapto;
- alkoxy, alkylthio;
- optionally substituted aryloxy or arylthio, preferentially substituted with one or more groups chosen from alkyl and $(O)_2S(O-)$-, M+ with M+ as defined previously;
- aryl(alkyl)amino optionally substituted with one or more groups chosen from alkyl and (O)2S(O-)-, M+ with M+ as defined previously;
- (di)(alkyl)amino;
- (di)(hydroxyalkyl)amino;
- $(O)_2S(O-)$-, M+ with M+ as defined previously;

Z' represents a hydrogen atom or a group NR28R29 with R28 and R29, which may be identical or different, representing a hydrogen atom or a group chosen from:

- alkyl;
- polyhydroxyalkyl such as hydroxyethyl;
- aryl optionally substituted with one or more groups, particularly i) alkyl such as methyl, n-dodecyl, n-butyl; ii) $(O)_2S(O-)$-, M+ with M+ as defined previously; iii) R°-C(X)-X'-, R°-X'-C(X)-, R°-X'-C(X)-X"- with R°, X, X' and X'' as defined previously, preferentially R° represents an alkyl group;
- cycloalkyl; notably cyclohexyl;

Z, represents a group chosen from hydroxyl and NR'28R'29 with R'28 and R'29, which may be identical or different, representing the same atoms or groups as R28 and R29 as defined previously;
it being understood that formulae (XXI) and (XXI') comprise at least one sulfonate radical $(O)_2S(O-)$-, M+ or one carboxylate radical C(O)O-, M+; preferentially sodium sulfonate.
As examples of dyes of formula (XXI), mention may be made of: Acid Blue 25, Acid Blue 43, Acid Blue 62, Acid Blue 78, Acid Blue 129, Acid Blue 138, Acid Blue 140, Acid Blue 251, Acid Green 25, Acid Green 41, Acid Violet 42, Acid Violet 43, Mordant Red 3; EXT violet No. 2; and, as an example of a dye of formula (XXI'), mention may be made of: Acid Black 48;
d) the nitro dyes of formulae (XXII) and (XXII'):

[Chem. 43]

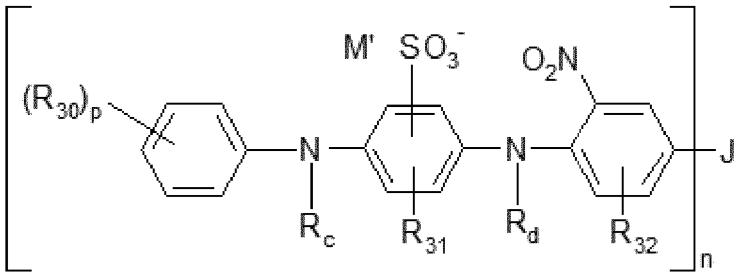

(XXII)

[Chem. 44]

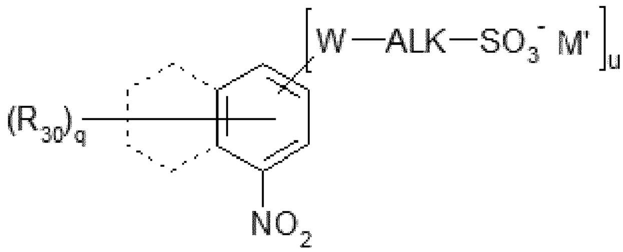

(XXII')

in which formulae (XXII) and (XXII'):

- $R_{30}$, $R_{31}$ and $R_{32}$, which may be identical or different, represent a hydrogen or halogen atom, or a group chosen from:
  - alkyl;
  - alkoxy optionally substituted with one or more hydroxyl groups, alkylthio optionally substituted with one or more hydroxyl groups;
  - hydroxyl, mercapto;
  - nitro, nitroso;
  - polyhaloalkyl;
  - R°-C(X)-X'-, R°-X'-C(X)-, R°-X'-C(X)-X''- with R°, X, X' and X'' as defined previously;
  - $(O)_2S(O\text{-})$-, M+ with M+ as defined previously;
  - $(O)CO^-$-, with as defined previously;
  - (di)(alkyl)amino;
  - (di)(hydroxyalkyl)amino;
  - heterocycloalkyl such as piperidino, piperazino or morpholino; in particular, $R_{30}$, $R_{31}$ and $R_{32}$ represent a hydrogen atom;
  - Rc and Rd, which may be identical or different, represent a hydrogen atom or an alkyl group;
  - W is as defined previously; W particularly represents an -NH- group;
  - ALK represents a linear or branched divalent $C_1$-$C_6$ alkylene group; in particular, ALK represents a $-CH_2$-$CH_2$- group;
  - n is 1 or 2;
  - p represents an integer inclusively between 1 and 5;
  - q represents an integer inclusively between 1 and 4;
  - u is 0 or 1;
  - when n is 1, J represents a nitro or nitroso group; particularly nitro;
  - when n is 2, J represents an oxygen or sulfur atom, or a divalent radical -S(O)m- with m representing an integer 1 or 2; preferentially, J represents an -SO2- radical;
  - M' represents a hydrogen atom or a cationic counterion;

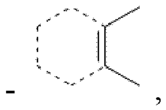

which may be present or absent, represents a benzo group optionally substituted with one or more groups R30 as defined previously;

it being understood that formulae (XXII) and (XXII') comprise at least one sulfonate radical $(O)_2S(O-)$-, M+ or one carboxylate radical -C(O)O-, M+; preferentially sodium sulfonate.

As examples of dyes of formula (XXII), mention may be made of: Acid Brown 13 and Acid Orange 3; as examples of dyes of formula (XXII'), mention may be made of: Acid Yellow 1, the sodium salt of 2,4-dinitro-1-naphthol-7-sulfonic acid, 2-piperidino-5-nitrobenzenesulfonic acid, 2-(4'-N,N-(2"-hydroxyethyl)amino-2'-nitro)anilineethanesulfonic acid, 4-β-hydroxyethylamino-3-nitrobenzenesulfonic acid; EXT D&C Yellow 7;

e) the triarylmethane dyes of formula (XXIII):

[Chem. 45]

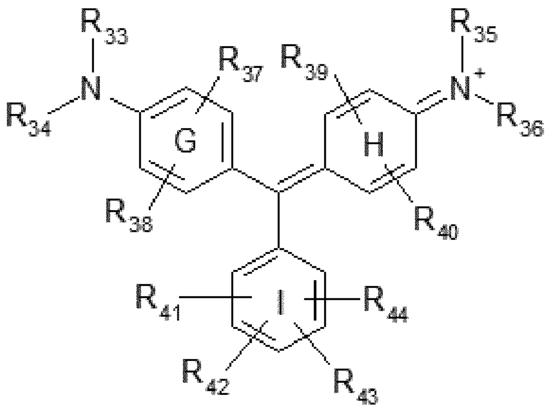

(XXIII)

in which formula (XXIII):

- $R_{33}$, $R_{34}$, $R_{35}$ and $R_{36}$, which may be identical or different, represent a hydrogen atom or a group chosen from alkyl, optionally substituted aryl and optionally substituted arylalkyl; particularly an alkyl and benzyl group optionally substituted with a group $(O)_mS(O-)$-, M+ with M+ and m as defined previously;
- $R_{37}$, $R_{38}$, $R_{39}$, $R_{40}$, $R_{41}$, $R_{42}$, $R_{43}$ and $R_{44}$, which may be identical or different, represent a hydrogen atom or a group chosen from:
  - alkyl;
  - alkoxy, alkylthio;
  - (di)(alkyl)amino;
  - hydroxyl, mercapto;
  - nitro, nitroso;
  - R°-C(X)-X'-, R°-X'-C(X)-, R°-X'-C(X)-X''- with R° representing a hydrogen atom or an alkyl or aryl group; X, X' and X'', which may be identical or different, representing an oxygen or sulfur atom, or NR with R representing a hydrogen atom or an alkyl group;
  - $(O)_2S(O-)$-, M+ with M+ representing a hydrogen atom or a cationic counterion;
  - $(O)CO^-$-, with as defined previously;
  - or alternatively two contiguous groups $R_{41}$ with $R_{42}$ or $R_{42}$ with $R_{43}$ or $R_{43}$ with $R_{44}$ together form a fused benzo group: I'; with I' optionally substituted with one or more groups chosen from i) nitro; ii) nitroso; iii) $(O)_2S(O-)$-, M+; iv) hydroxyl; v) mercapto; vi) (di)(alkyl)amino; vii) R°-C(X)-X'-; viii) R°-X'-C(X)- and ix) R°-X'-C(X)-X''-; with M+, R°, X, X' and X'' as defined previously;

in particular, $R_{37}$ to $R_{40}$ represent a hydrogen atom, and $R_{41}$ to $R_{44}$, which may be identical or different, represent a

hydroxyl group or $(O)_2S(O\text{-})$-, M+; and when $R_{43}$ with $R_{44}$ together form a benzo group, it is preferentially substituted with an $(O)_2S(O\text{-})$- group;

it being understood that at least one of the rings G, H, I or I' comprises at least one sulfonate radical $(O)_2S(O^-)$- or a carboxylate radical -C(O)O-; preferentially sulfonate;

As examples of dyes of formula (XXIII), mention may be made of: Acid Blue 1; Acid Blue 3; Acid Blue 7, Acid Blue 9; Acid Violet 49; Acid Green 3; Acid Green 5 and Acid Green 50;

f) the xanthene-based dyes of formula (XXIV):

[Chem. 46]

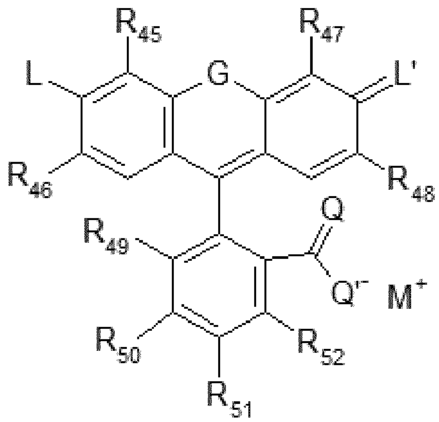

(XXIV)

in which formula (XIV):

- $R_{45}$, $R_{46}$, $R_{47}$ and $R_{48}$, which may be identical or different, represent a hydrogen or halogen atom;
- $R_{49}$, $R_{50}$, $R_{51}$ and $R_{52}$, which may be identical or different, represent a hydrogen or halogen atom, or a group chosen from:

  - alkyl;
  - alkoxy, alkylthio;
  - hydroxyl, mercapto;
  - nitro, nitroso;
  - $(O)_2S(O\text{-})$-, M+ with M+ representing a hydrogen atom or a cationic counterion;
  - $(O)CO^-$-, with as defined previously;
    particularly, $R_{53}$, $R_{54}$, $R_{55}$ and $R_{48}$ represent a hydrogen or halogen atom;
  - G represents an oxygen or sulfur atom or a group NRe with Re as defined previously; particularly G represents an oxygen atom;
  - L represents an alkoxide O-, M+; a thioalkoxide S-, M+ or a group NRf, with Rf representing a hydrogen atom or an alkyl group, and M+ as defined previously; M+ is particularly sodium or potassium;
  - L' represents an oxygen or sulfur atom or an ammonium group: N+RfRg, with Rf and Rg, which may be identical or different, representing a hydrogen atom or an optionally substituted alkyl or aryl group; L' particularly represents an oxygen atom or a phenylamino group optionally substituted with one or more alkyl or $(O)_mS(O\text{-})$-, M+ groups with m and M+ as defined previously;
  - Q and Q', which may be identical or different, represent an oxygen or sulfur atom; in particular, Q and Q' represent an oxygen atom;
  - M+ is as defined previously.

As an example of dyes of formula (XXIV), mention may be made of: Acid Yellow 73; Acid Red 51; Acid Red 52; Acid Red 87; Acid Red 92; Acid Red 95; Acid Violet 9;

f) the indole-based dyes of formula (XXV):

[Chem. 47]

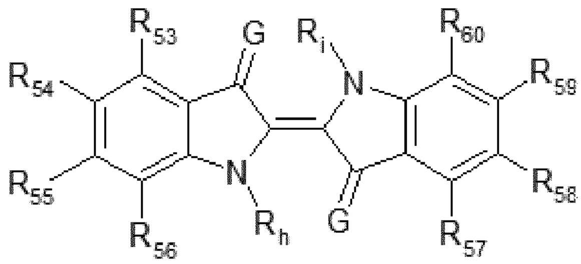

(XXV)

$R_{53}$, $R_{54}$, $R_{55}$, $R_{56}$, $R_{57}$, $R_{58}$, $R_{59}$ and $R_{60}$, which may be identical or different, represent a hydrogen atom or a group chosen from:

- alkyl;
- alkoxy, alkylthio;
- hydroxyl, mercapto;
- nitro, nitroso;
- R°-C(X)-X'-, R°-X'-C(X)-, R°-X'-C(X)-X''- with R° representing a hydrogen atom or an alkyl or aryl group; X, X' and X'', which may be identical or different, representing an oxygen or sulfur atom, or NR with R representing a hydrogen atom or an alkyl group;
- $(O)_2S(O-)-$, M+ with M+ representing a hydrogen atom or a cationic counterion;
- (O)CO$^-$-, with as defined previously;
- G represents an oxygen or sulfur atom or a group NRe with Re as defined previously; particularly G represents an oxygen atom;
- Ri and Rh, which may be identical or different, represent a hydrogen atom or an alkyl group; it being understood that formula (XXV) comprises at least one sulfonate radical $(O)_2S(O-)-$, M+ or one carboxylate radical -C(O)O-, M+; preferentially sodium sulfonate.

As an example of dyes of formula (XXV), mention may be made of: Acid Blue 74.
h) the quinoline-based dyes of formula (XXVI):

[Chem. 48]

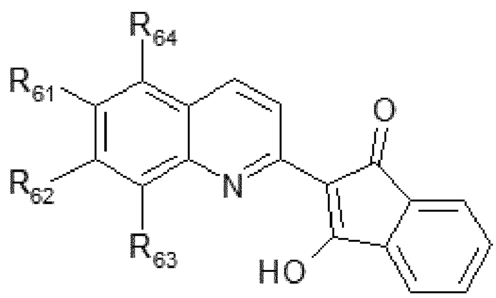

(XXVI)

- $R_{61}$ represents a hydrogen or halogen atom or an alkyl group;
- $R_{62}$, $R_{63}$ and $R_{64}$, which may be identical or different, represent a hydrogen atom or a group $(O)_2S(O-)-$, M+ with M+ representing a hydrogen atom or a cationic counterion;

or alternatively $R_{61}$ with $R_{62}$, or $R_{61}$ with $R_{64}$, together form a benzo group optionally substituted with one or more groups $(O)_2S(O-)-$, M+ with M+ representing a hydrogen atom or a cationic counterion;
it being understood that formula (XXV) comprises at least one sulfonate radical $(O)_2S(O-)-$, M+ preferentially sodium sulfonate.

As examples of dyes of formula (XXVI), mention may be made of: Acid Yellow 2, Acid Yellow 3 and Acid Yellow 5.
Among the natural direct dyes that may be used according to the invention, mention may be made of lawsone, juglone, alizarin, purpurin, carminic acid, kermesic acid, purpurogallin, protocatechaldehyde, indigo, isatin, curcumin, spinulosin, apigenidin and orceins. Use may also be made of extracts or decoctions comprising these natural dyes and in particular henna-based poultices or extracts.
Preferably, the direct dye(s) are chosen from anionic direct dyes.
The coloring agent(s) may be present in a total content ranging from 0.001% to 20% by weight and preferably from 0.005% to 15% by weight relative to the total weight of composition (C).
The pigment(s) may be present in a total content ranging from 0.05% to 20% by weight, preferably from 0.1% to 15% by weight and better still from 1% to 10% by weight, relative to the total weight of composition (C).
The direct dye(s) may be present in a total content ranging from 0.001% to 10% by weight relative to the total weight of the composition, preferably from 0.005% to 5% by weight relative to the total weight of composition (C).
Composition (C) according to the invention may comprise water. Preferably, water is present in a content ranging from 0.1% to 95% by weight, more preferentially from 1% to 90% by weight and better still from 10% to 90% by weight relative to the total weight of the composition.

**Organic solvents:**

Composition (C) according to the invention may comprise one or more organic solvents.
Examples of organic solvents that may be mentioned include lower $C_1$-$C_4$ alkanols, such as ethanol and isopropanol; polyols and polyol ethers, for instance glycerol, 2-butoxyethanol, propylene glycol, propylene glycol monomethyl ether and diethylene glycol monoethyl ether and monomethyl ether, and also aromatic alcohols, for instance benzyl alcohol or phenoxyethanol, and mixtures thereof.
The organic solvent(s) may be present in a total amount inclusively between 0.1% and 20% by weight approximately relative to the total weight of the dye composition, preferably between 0.5% and 15% by weight and more preferentially inclusively between 1% and 15% by weight relative to the total weight of composition (C).

**Additives:**

Composition (C) may also contain any adjuvant or additive usually used.
Among the additives that may be contained in the composition, mention may be made of reducing agents, thickeners, softeners, antifoams, moisturizers, UV-screening agents, peptizers, solubilizers, fragrances, anionic, cationic, nonionic or amphoteric surfactants, proteins, vitamins, polymers, preserving agents, waxes and mixtures thereof.
The composition according to the invention may notably be in the form of a suspension, a dispersion, a gel, an emulsion, notably an oil-in-water (O/W) or water-in-oil (W/O) emulsion, or a multiple emulsion (W/O/W or polyol/O/W or O/W/O), in the form of a cream, a mousse, a stick, a dispersion of vesicles, notably of ionic or nonionic lipids, or a two-phase or multi-phase lotion.
A person skilled in the art may select the appropriate presentation form, and also the method for preparing it, on the basis of his general knowledge, taking into account firstly the nature of the constituents used, notably their solubility in the support, and secondly the intended application of the composition.

**Process for treating keratin fibers**

Preferably, the process for treating the keratin fibers according to the invention also comprises a step of applying to the keratin fibers a composition (D) comprising at least one silicone compound comprising at least one carboxylic group.

**Silicone compound bearing a carboxylic function:**

Composition (D) according to the invention comprises at least one silicone compound comprising at least one carboxylic group.
The term "carboxylic group" means a COOH or $COO^-$ functional group, the counterion of the $COO^-$ group possibly being chosen from alkali metals, alkaline-earth metals and quaternary ammoniums.
The silicones that may be used may be soluble or insoluble in composition (D) according to the invention; they may be in the form of oil, wax, resin or gum; silicone oils and gums are preferred.
Silicones are notably described in detail in Walter Noll's Chemistry and Technology of Silicones (1968), Academic Press.
Preferably, the silicone compound(s) comprising at least one carboxylic group are chosen from the organosiloxanes of formula (XXVII) below:

[Chem.49]

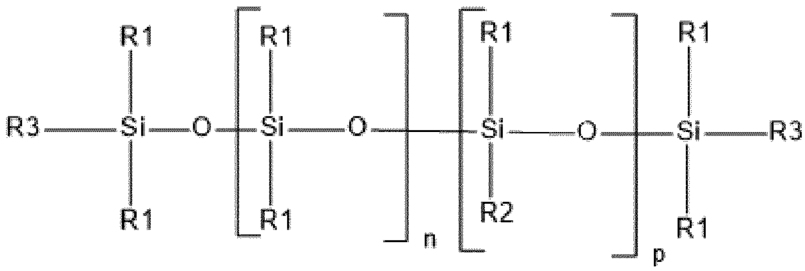

(XXVII)

in which:

- **R1** independently represents an alkyl group containing from 1 to 20 carbon atoms, preferably from 1 to 10 carbon atoms; a hydroxyl group; an alkoxy group containing from 1 to 20 carbon atoms or an aryl group containing from 6 to 12 carbon atoms;
- **R2** independently represents a group R4-COOM with R4 representing a linear or branched alkylene group containing from 1 to 20 carbon atoms, preferably from 4 to 16 carbon atoms, and M representing a hydrogen atom; an alkali metal or alkaline-earth metal or a quaternary ammonium NR'3, with R', which may be identical or different, representing H or alkyl containing from 1 to 4 carbon atoms; a pyrrolidine radical comprising a carboxylic group COOH or a group Ra-(ORb)x-COOM with Ra representing a linear or branched alkylene group containing from 1 to 4 carbon atoms, Rb representing an alkyl group containing from 1 to 4 carbon atoms, **x** being an integer ranging from 1 to 200; and **M** representing a hydrogen atom, an alkali metal or alkaline-earth metal or a quaternary ammonium $NR'_3$, with **R'**, which may be identical or different, representing H or an alkyl containing from 1 to 4 carbon atoms;
- **R3** independently represent an alkyl group containing from 1 to 20 carbon atoms; a hydroxyl group; a group R4-COOM with **R4** representing a linear or branched alkylene group containing from 1 to 20 carbon atoms, preferably from 4 to 16 carbon atoms, and **M** representing a hydrogen atom; an alkali metal or alkaline-earth metal or a quaternary ammonium NR'3, with **R'**, which may be identical or different, representing H or alkyl containing from 1 to 4 carbon atoms; an alkoxy group containing from 1 to 20 carbon atoms; an aryl group containing from 6 to 12 carbon atoms or a group $R_a$-$(OR_b)_x$-COOM with $\mathbf{R_a}$ representing a linear or branched alkylene group containing from 1 to 4 carbon atoms, $\mathbf{R_b}$ representing an alkyl group containing from 1 to 4 carbon atoms, **x** being an integer ranging from 1 to 200; and **M** representing a hydrogen atom, an alkali metal or alkaline-earth metal or a quaternary ammonium $NR'_3$, with **R'**, which may be identical or different, representing H or an alkyl containing from 1 to 4 carbon atoms;
- **n** denotes an integer ranging from 1 to 1000;
- **p** denotes an integer ranging from 0 to 1000;

it being understood that at least one of the radicals R2 and/or R3 comprises a carboxylic group COOH or COOM with M representing an alkali metal or alkaline-earth metal or a quaternary ammonium $NR'_3$, with **R'**, which may be identical or different, representing H or an alkyl containing from 1 to 4 carbon atoms.

Notably, the silicone compound(s) comprising at least one carboxylic group may be chosen from the organosiloxanes of formula (XXVIII) below:

[Chem. 50]

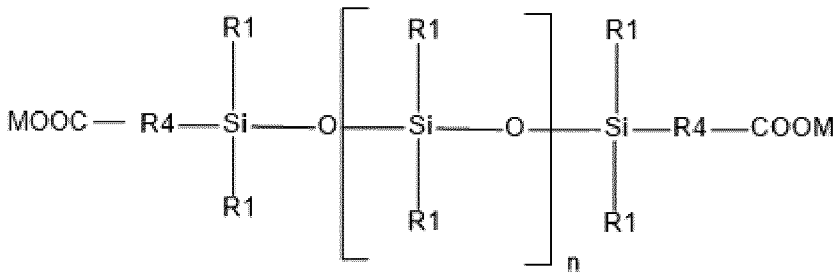

(XXVIII)

in which:

- R1 independently represents a linear or branched alkyl group containing from 1 to 20 carbon atoms, preferably from 1 to 10 carbon atoms and better still from 1 to 6 carbon atoms, preferentially methyl;
- **R4** independently represents a linear or branched alkylene group containing from 1 to 20 carbon atoms, preferably from 4 to 16 carbon atoms; or a divalent group $R_a$-$(OR_b)_x$- with $\mathbf{R_a}$ representing a linear or branched alkylene group containing from 1 to 4 carbon atoms, $\mathbf{R_b}$ representing an alkylene group containing from 1 to 4 carbon atoms, and **x** being an integer ranging from 1 to 200;
- **M** independently represents a hydrogen atom, an alkali metal or alkaline-earth metal or a quaternary ammonium $NR'_3$, with **R'**, which may be identical or different, representing H or an alkyl containing from 1 to 4 carbon atoms;
- **n** denotes an integer ranging from 1 to 1000;

- the organosiloxanes of formula (XXIX) below:

[Chem. 51]

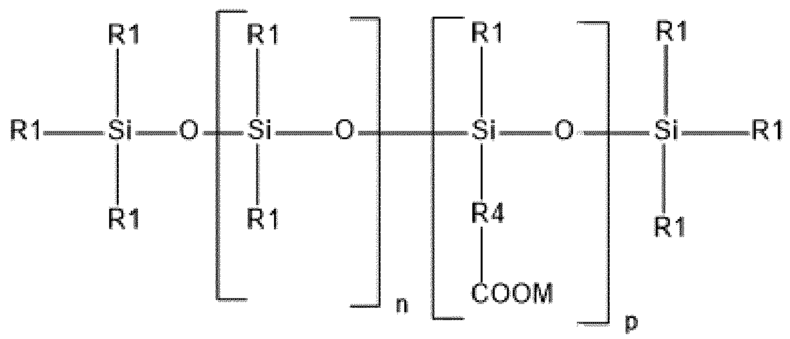

(XXIX)

in which:

- **R1** independently represents an alkyl group containing from 1 to 10 carbon atoms, preferably from 1 to 6 carbon atoms, more preferentially a methyl;
- **R4** represents a saturated or unsaturated, linear or branched alkylene group containing from 1 to 20 carbon atoms, preferably from 4 to 16 carbon atoms; or a divalent group $R_a$-$(OR_b)_x$-with $\mathbf{R_a}$ representing a linear or branched alkylene group containing from 1 to 4 carbon atoms, $\mathbf{R_b}$ representing an alkylene group containing from 1 to 4 carbon atoms, and **x** being an integer ranging from 1 to 200;
- **M** represents a hydrogen atom, an alkali metal or alkaline-earth metal or a quaternary ammonium $NR'_3$, with **R',** which may be identical or different, representing H or an alkyl containing from 1 to 4 carbon atoms;
- **p** denotes an integer ranging from 1 to 1000;
- **n** denotes an integer ranging from 1 to 1000;

- the organosiloxanes of formula (XXX) below:

[Chem. 52]

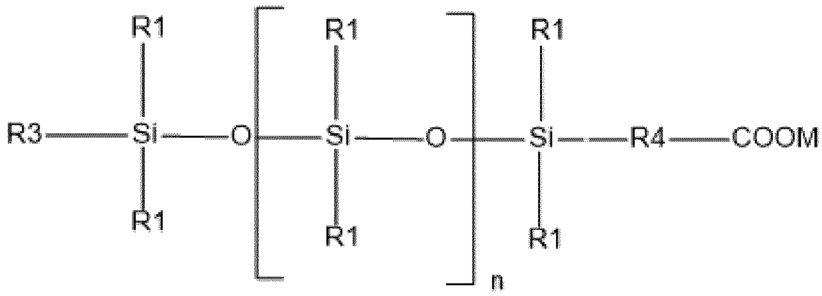

(XXX)

in which:

- R**1** independently represents a linear or branched alkyl group containing from 1 to 20 carbon atoms, preferably from 1 to 10 carbon atoms and better still from 1 to 6 carbon atoms, preferentially methyl;
- **R4** represents a linear or branched alkylene group containing from 1 to 20 carbon atoms, preferably from 4 to 16 carbon atoms; or a divalent group $R_a$-($OR_b$)$_x$- with $\mathbf{R_a}$ representing a linear or branched alkylene group containing from 1 to 4 carbon atoms, $\mathbf{R_b}$ representing an alkylene group containing from 1 to 4 carbon atoms, and **x** being an integer ranging from 1 to 200;
- **R3** represents an alkyl group containing from 1 to 20 carbon atoms, an alkoxy group containing from 1 to 20 carbon atoms or an aryl group containing from 6 to 12 carbon atoms;
- **M** independently represents a hydrogen atom, an alkali metal or alkaline-earth metal or a quaternary ammonium NR'$_3$, with **R',** which may be identical or different, representing H or an alkyl containing from 1 to 4 carbon atoms;
- **n** denotes an integer ranging from 1 to 1000;

- the organosiloxanes of formula (XXXI) below:

[Chem. 53]

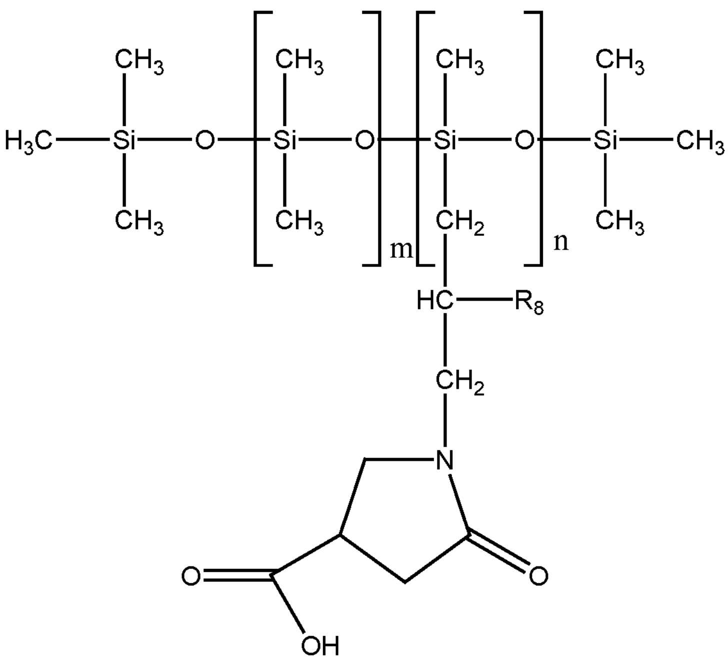

(XXXI)

in which:

- **R8** represents an alkyl group containing from 1 to 6 carbon atoms, preferably a methyl;
- **m** denotes an integer ranging from 1 to 1000;
- **n** denotes an integer ranging from 1 to 1000;

- and mixtures thereof.

Among the organosiloxanes of formula (XXVIII), mention may be made of polydimethylsiloxanes (PDMS) bearing a carboxyl end function, such as the compounds sold by the company Momentive under the trade name Silform INX (INCI name: Bis-Carboxydecyl Dimethicone).
Among the organosiloxanes of formula (XXIX), mention may be made of polydimethylsiloxanes (PDMS) bearing a carboxyl side function, such as the compounds sold by the company Shin-Etsu under the trade name X-22-3701E.
Among the organosiloxanes of formula (XXX), mention may be made of polydimethylsiloxanes (PDMS) bearing a carboxyl end function, such as the compounds sold by the company Shin-Etsu under the trade name X-22-3710.
Among the organosiloxanes of formula (XXXI), mention may be made of the compounds sold by the company Grant Industries under the trade name Grandsil SiW-PCA-10 (INCI name: Dimethicone (and) PCA Dimethicone (and) Butylene Glycol (and) Decyl Glucoside).
The silicone compounds comprising a carboxylic group may correspond, for example, to the compounds described in the patent application EP 186 507 in the name of Chisso Corporation, introduced herein by reference.
Preferably, the silicone compound(s) comprising at least one carboxylic group are chosen from the organosiloxanes of formula (XXVIII), the organopolysiloxanes of formula (XXIX) and mixtures thereof.
More preferentially, the silicone compound(s) comprising at least one carboxylic group are chosen from the organopolysiloxanes of formula (XXIXa) below:

[Chem. 54]

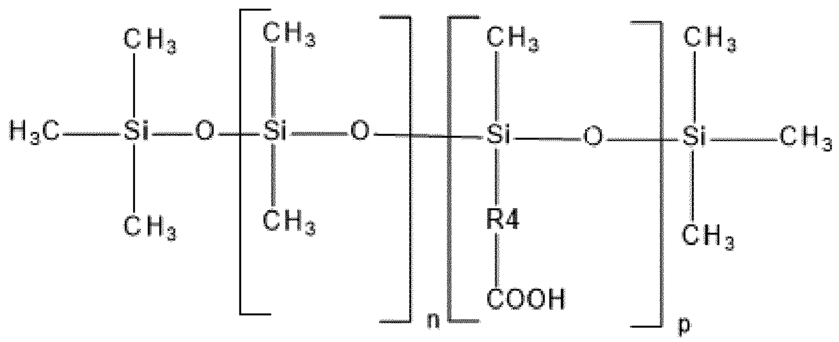

(XXIXa)

in which:

- **R4** represents a saturated or unsaturated, linear or branched alkylene group containing from 1 to 20 carbon atoms, preferably from 4 to 16 carbon atoms, or even from 8 to 12 carbon atoms;
- **p** denotes an integer ranging from 1 to 1000;
- **n** denotes an integer ranging from 1 to 1000.

The total amount of the silicone compound(s) comprising at least one carboxylic group, present in composition (D) according to the invention, preferably ranges from 0.01% to 20% by weight, more preferentially from 0.1% to 15% by weight and better still from 0.5% to 10% by weight relative to the total weight of composition (D).

**Oils**

Composition (D) may comprise one or more oils.
Preferably, composition (D) comprises one or more oils. More preferentially, composition (D) comprises one or more oils chosen from alkanes.
The term *"oil"* means a fatty substance that is liquid at room temperature (25°C) and at atmospheric pressure (760 mmHg or $1.013\times10^5$ Pa).
The oil may be volatile or nonvolatile.

The term *"volatile oil"* refers to an oil that can evaporate on contact with the skin in less than one hour, at room temperature and atmospheric pressure. The volatile oil is a cosmetic volatile oil, which is liquid at room temperature. More specifically, a volatile oil has an evaporation rate of between 0.01 and 200 mg/cm$^2$/min, limits included (see protocol for measuring the evaporation rate indicated in the text below).

The term *"nonvolatile oil"* refers to an oil that remains on the skin or the keratin fiber at room temperature and atmospheric pressure. More specifically, a nonvolatile oil has an evaporation rate of strictly less than 0.01 mg/cm2/min (see protocol for measuring the evaporation rate indicated in the text below).

Preferably, the composition comprises one or more oils chosen from $C_6$-$C_{16}$ alkanes and/or mixtures thereof.

As regards the $C_6$-$C_{16}$ alkanes, they may be linear or branched, and possibly cyclic.

Mention may notably be made of branched $C_8$-$C_{16}$ alkanes, such as $C_8$-$C_{16}$ isoalkanes (also known as isoparaffins), isododecane, isodecane or isohexadecane, and for example the oils sold under the Isopar or Permethyl trade names, and mixtures thereof.

Mention may also be made of linear alkanes, preferably of plant origin, comprising from 7 to 15 carbon atoms, in particular from 9 to 14 carbon atoms and more particularly from 11 to 13 carbon atoms.

As examples of linear alkanes that are suitable for use in the invention, mention may be made of n-heptane (C7), n-octane (C8), n-nonane (C9), n-decane (C10), n-undecane (C11), n-dodecane (C12), n-tridecane (C13), n-tetradecane (C14) and n-pentadecane (C15), and mixtures thereof, and in particular the mixture of n-undecane (C11) and n-tridecane (C13) described in Example 1 of patent application WO 2008/155 059 by the company Cognis.

Mention may also be made of n-dodecane (C12) and n-tetradecane (C14) sold by Sasol under the references, respectively, Parafol 12-97 and Parafol 14-97, and also mixtures thereof.

As examples of alkanes that are suitable for use in the invention, mention may be made of the alkanes described in patent applications WO 2007/068 371 and WO 2008/155 059. These alkanes are obtained from fatty alcohols, which are themselves obtained from coconut kernel oil or palm oil.

According to a particular embodiment, the composition comprises isododecane. Such a compound is, for example, the isododecane sold under the reference Isododecane by Ineos.

Preferably, composition (D) according to the invention comprises one or more oils chosen from $C_8$-$C_{16}$ alkanes, more preferentially from isododecane, isohexadecane, tetradecane and/or mixtures thereof.

More preferentially, composition (D) comprises isododecane.

The composition according to the invention may comprise one or more oils present in a total amount of between 10% and 99% by weight, preferably between 20% and 99% by weight and better still between 30% and 99% by weight, relative to the total weight of the composition.

Composition (D) may comprise at least one coloring agent chosen from pigments, direct dyes and mixtures thereof as described previously.

**Protocol:**

Composition (C) and/or composition (D) described above may be used on wet or dry keratin fibers, and also on any type of fair or dark, natural or dyed, permanent-waved, bleached or relaxed fibers.

According to a preferred embodiment, composition (C) and composition (D) are applied simultaneously to the keratin fibers.

According to another preferred embodiment, composition (D) is applied to the keratin fibers after applying composition (C) to the keratin fibers.

According to another preferred embodiment, composition (D) is applied to the keratin fibers before applying composition (C) to the keratin fibers.

According to a particular embodiment of the invention, the keratin fibers are washed before applying composition (C) and/or composition (D).

Preferably, a washing, rinsing, draining or drying step is performed after applying composition (C) to the keratin fibers and before applying composition (D) to the keratin fibers.

More preferentially, a drying step is performed after applying composition (C) to the keratin fibers and before applying composition (D) to the keratin fibers.

The application to the fibers may be performed via any standard means, in particular using a comb, a fine brush, a coarse brush, a sponge or with the fingers.

The application of composition (C) and/or composition (D) to the keratin fibers is generally performed at room temperature (between 15 and 25°C).

After applying composition (C) to the keratin fibers, it is possible to wait for between 1 minute and 6 hours, in particular between 1 minute and 2 hours, more particularly between 1 minute and 1 hour, more preferentially between 1 minute and 30 minutes, before, for example, applying composition (D) to the keratin fibers or, for example, a washing, rinsing, draining or drying step.

Preferably, there is no leave-on time after applying composition (C) to the keratin fibers and before applying composition (D) to the keratin fibers.

After applying composition (C) and/or composition (D), the fibers may be left to dry or may be dried, for example at a temperature of greater than or equal to 30°C.

The process according to the invention may thus comprise a step of applying heat to the keratin fibers using a heating tool.

Preferably, the process according to the invention comprises, after applying composition (C) or applying composition (C) and composition (D) on the keratin fibers, a step of applying heat to the keratin fibers using a heating tool at a temperature between 30°C and 220°C, more preferably between 50°C and 90°C.

More preferably, the heating tool delivers a flow of air on the keratin fibers.

The heat application step of the process of the invention may be performed using a hood, a hairdryer, a straightening iron, a curling iron, a Climazon, etc.

Preferably, the heat application step of the process of the invention is performed using a hairdryer.

When the process of the invention involves a step of applying heat to the keratin fibers, the step of applying heat to the keratin fibers takes place after the application of composition (C) and/or composition (D) to the keratin fibers.

During the step of applying heat to the keratin fibers, a mechanical action may be exerted on the locks, such as combing, brushing or running the fingers through.

When the step of applying heat to the keratin fibers is performed using a hood or a hairdryer, the temperature is preferably between 30°C and 110°C, preferentially between 50°C and 90°C.

When the step of applying heat to the keratin fibers is performed using a straightening iron, the temperature is preferably between 110°C and 220°C, preferably between 140°C and 200°C.

In a particular variant, the process of the invention involves a step (b1) of applying heat using a hood, a hairdryer or a Climazon, preferably a hairdryer, and a step (b2) of applying heat using a straightening or curling iron, preferably a straightening iron.

Step (b1) may be performed before step (b2).

During step (b1), also referred to as the drying step, the fibers may be dried, for example at a temperature above or equal to 30°C. According to a particular embodiment, this temperature is above 40°C. According to a particular embodiment, this temperature is above 45°C and below 110°C.

Preferably, if the fibers are dried, they are dried, in addition to a supply of heat, with a flow of air. This flow of air during drying makes it possible to improve the strand separation of the coating.

During drying, a mechanical action may be exerted on the locks, such as combing, brushing or running the fingers through.

During step (b2), the passage of the straightening or curling iron, preferably the straightening iron, may be performed at a temperature ranging from 110°C to 220°C, preferably between 140°C and 200°C.

After the drying step, a shaping step may be performed, for example with a straightening iron; the temperature for the shaping step is between 110 and 220°C, preferably between 140 and 200°C.

Preferably, the invention is a process for treating keratin fibers, i.e. hair, comprising:

i) the application to said fibers of a composition (C) comprising:

- a) at least one (poly)carbodiimide compound as described previously,
- b) at least one aqueous dispersion of particles of polymer(s) chosen from polyurethanes, acrylic polymers, and mixtures thereof, as described previously,
- c) at least one silicone as described previously,
- d) at least one coloring agent chosen from pigments, direct dyes and mixtures thereof, and then

ii) optionally a leave-on time of said composition (C) on the fibers of from 1 minute to 30 minutes, preferably from 1 to 20 minutes, and then

iii) optionally a step of washing, rinsing, draining or drying said fibers, and then

iv) the application to said fibers of a composition (D) comprising at least one silicone compound comprising at least one carboxylic group as described previously; and then

v) optionally a leave-on time of said composition (D) on the fibers of from 1 minute to 30 minutes, preferably from 1 to 20 minutes, and then

vi) optionally a step of washing, rinsing, draining or drying said fibers.

Preferably, the step of applying composition (C) to the keratin fibers is repeated several times.

According to a preferred embodiment, the process for treating keratin fibers is a process for treating keratin fibers i.e. hair, which consists in extemporaneously mixing, at the time of use, at least two compositions (C1) and (C2) and in

applying the mixture to the keratin fibers, with:

- composition (C1) comprising at least one (poly)carbodiimide compound as described previously; and
- composition (C2) comprising at least one aqueous dispersion of particles of polymer(s) chosen from polyurethanes, acrylic polymers, and mixtures thereof, as described previously; composition (C1) and/or composition (C2) comprising at least one coloring agent chosen from pigments, direct dyes, and mixtures thereof, and comprising at least one silicone as defined previously.

Preferably, composition (C2) comprises at least one coloring agent chosen from pigments, direct dyes, and mixtures thereof.
Preferably, composition (C1) does not comprise at least one coloring agent chosen from pigments, direct dyes, and mixtures thereof.
According to this embodiment, compositions (C1) and (C2) are mixed preferably less than 15 minutes before application to the keratin fibers, more preferentially less than 10 minutes before application, better still less than 5 minutes before application.
The weight ratio between composition (C1) and composition (C2) preferably ranges from 0.1 to 10, preferentially from 0.2 to 5 and better still from 0.5 to 2, or even from 0.6 to 1.5. In a particular embodiment, the weight ratio between composition (C1) and composition (C2) is equal to 1.
According to another particular embodiment, the process for treating keratin fibers is a process for treating keratin fibers such as the hair, which consists in extemporaneously mixing, at the time of use, at least two compositions (C1) and (C2) and in applying the mixture to the keratin fibers, with:

- composition (C1) comprising at least one (poly)carbodiimide compound as described previously; and
- composition (C2) comprising at least one aqueous dispersion of particles of polymer(s) chosen from polyurethanes, acrylic polymers, and mixtures thereof, as described previously; composition (C1) and/or composition (C2) comprising at least one coloring agent chosen from pigments, direct dyes, and mixtures thereof, and comprising at least one silicone as defined previously, and

a composition (D) as described previously being applied to the keratin fibers before and/or after the application of the mixture of compositions (C1) and (C2) to the keratin fibers.
The present invention also relates to a device for treating keratin fibers, comprising one or more compartments containing:

- in a first compartment (E1), a composition (C) as defined previously; and
- optionally, in a second compartment (E2), a composition (D) as defined previously.

According to a particular variant of the invention, the present invention relates to a device for treating keratin fibers such as the hair, comprising at least two compartments containing:

- in a first compartment (F1), a composition (C1) comprising at least one (poly)carbodiimide compound as defined previously; and
- in a second compartment (F2), a composition (C2) comprising at least one aqueous dispersion of particles of polymer(s) chosen from polyurethanes, acrylic polymers, and mixtures thereof, as defined previously;
composition (C1) and/or composition (C2) comprising at least one coloring agent chosen from pigments, direct dyes, and mixtures thereof, and comprising at least one silicone as defined previously; and
- optionally, in a third compartment (F3), a composition (D) as defined previously.

The total amount of the (poly)carbodiimide compound(s) preferably ranges from 0.01% to 40% by weight, more preferentially from 0.1% to 30% by weight, better still from 0.5% to 25% by weight, and even better still from 1% to 10% by weight relative to the total weight of composition (C1).
The total amount of the aqueous dispersion(s) of polymer particles preferably ranges from 0.1% to 40% by weight, more preferentially from 0.1% to 35% by weight, and better still from 0.2% to 30% by weight, relative to the total weight of composition (C2).
The silicone(s) may be present in a total amount ranging from 0.01% to 20% by weight relative to the total weight of the composition, preferably from 0.05% to 15% by weight, more preferentially from 0.1% to 10% by weight, more preferentially from 0.1% to 5% by weight relative to the total weight of composition (C1) and/or (C2).
The present invention will now be described more specifically by means of examples, which do not in any way limit the scope of the invention. However, the examples make it possible to support specific features, variants and preferred

embodiments of the invention.

The (poly)carbodiimide(s) of the invention are accessible via synthetic methods known to those skilled in the art starting from commercial products or reagents that can be synthesized according to chemical reactions that are also known to those skilled in the art. Mention may be made, for example, of the book Sciences of Synthesis - Houben - Weyl Methods of Molecular Transformations, 2005, Georg Thiem Verlag Kg, Rudigerstrasse 14, D-70469 Stuttgart, or the American patent US 4 284 730 or the Canadian patent application CA 2 509 861.

More particularly, the process for preparing the (poly)carbodiimides of the invention involves, in a first step, a diisocyanate reagent (1):

$$O=C=N-L_1-N=C=O \qquad (1)$$

Formula (1) wherein $L_1$ is as defined previously, which reacts in the presence of a carboimidation catalyst (2) such as those described in US 4 284 730, notably phosphorus-based catalysts particularly chosen from phospholene oxides and phospholene sulfoxides, diaza- and oxaza-phospholanes, preferably under an inert atmosphere (nitrogen or argon), and in particular in a polar solvent which is preferably aprotic such as THF, glyme, diglyme, 1,4-dioxane or DMF, at a temperature between room temperature and the reflux temperature of the solvent, preferably at about 140°C; to give the carbodiimide diisocyanate compound (3):

$$O=C=N-L_1-(N=C=N-L_1)_n-N=C=O \qquad (3)$$

Formula (3) wherein $L_1$ and n are as defined previously. Benzoyl halogen such as benzoyl chloride may be added to deactivate the catalyst.

To obtain "symmetrical" (poly)carbodiimides, during the second step of the preparation process, compound (3) reacts with 1 molar equivalent (1 eq.) of nucleophilic reagent $R_1$-$X_1$-H and then 0.5 eq. of reagent H-E-H with $R_1$, $X_1$ and E as defined previously, to give the "symmetrical" compound according to the invention (4):

$$[R_1-X_1-C(O)-NH-L_1-(N=C=N-L_1)_n-NH-C(O)]_2-E \qquad (4)$$

Formula (4) wherein $R_1$, $X_1$, $L_1$, n and E are as defined previously. According to one variant to obtain compound (4) from (3), it is possible first to add 0.5 eq. of reagent H-E-H and then 1 eq. of reagent $R_1$-$X_1$-H.

To obtain "dissymmetrical" (poly)carbodiimides, during the second step of the preparation process, compound (3) reacts with 1 molar equivalent (1 eq.) of nucleophilic reagent $R_1$-$X_1$-H and then 1 eq. of reagent H-E-H with $R_1$, $X_1$ and E as defined previously, to give compound (5):

$$R_1-X_1-C(O)-NH-L_l-(N=C=N-L_1)_n-NH-C(O)-E-H \qquad (5)$$

Formula (5) wherein $R_1$, $X_1$, $L_1$, n and E are as defined previously.

According to one variant to obtain compound (5) from (3), it is possible first to add 1 eq. of reagent $R_1$-$X_1$-H and then 0.5 eq. of reagent H-E-H.

During a third step, compound (5) reacts with 1 eq. of compound (6):

$$R_2-X_2-C(O)-NH-Li-(N=C=N-Li)_z-N=C=O \qquad (6),$$

said compound (6) is prepared beforehand from compound (3'):

$$O=C=N-L_1-(N=C=N-L_1)_z-N=C=O \qquad (3'),$$

Formula (3') wherein $L_1$ and z are as defined previously, which reacts with 1 eq. of nucleophilic reagent $R_2$-$X_2$-H with $L_1$, $R_2$, $X_2$ and z as defined previously, to give the dissymmetrical compound (7):

$$R_1-X_1-C(O)-NH-L_1-(N=C=N-L_1)_n-NH-C(O)-E-C(O)-NH-L_1-(N=C=N-L_1)_z-NH-C(O)-X_2-R_2 \qquad (7)$$

Formula (7) wherein $L_1$, $R_1$, $X_1$, $R_2$, $X_2$, n, z and E are as defined previously.

It is also possible to react 1 eq of compound (3') $O=C=N-L_1-(N=C=N-L_1)_z-N=C=O$ (3'), with 1/w equivalent of H-E-H then with 1 eq. of nucleophilic reagent $R_2$-$X_2$-H to give compound (8):

$$H-[E-C(O)-NH-L_1-\{N=C=N-L_1)_{\ z}]_w-NH-C(O)-X_2-R_2 \qquad (8)$$

Formula (8) wherein $L_1$, $R_2$, $X_2$, z and E are as defined previously, and w denotes an integer ranging from 1 to 3, preferably equal to 1.

The latter compound (8) can then react with 1 eq. of compound (4') :

$$R_1\text{-}X_1\text{-}C(O)\text{-}NH\text{-}L_1\text{-}(N{=}C{=}N\text{-}L_1)_n\text{-}N{=}C{=}O \qquad (4')$$

(the said compound (4') can be synthetized by reaction of 0.5 eq. of nucleophilic reagent $R_1$-$X_1$-H with 1 eq. of compound (3)) to give the (poly)carbodiimide (9) of the invention :

$$R_1\text{-}X_1\text{-}C(O)\text{-}NH\text{-}L_1\text{-}(N{=}C{=}N\text{-}L_1)_n\text{-}NH\text{-}C(O)\text{-}[E\text{-}C(O)\text{-}NH\text{-}L_1\text{-}(N{=}C{=}N\text{-}L_1)_z]_w\text{-}NH\text{-}C(O)\text{-}X_2\text{-}R_2 \qquad (9)$$

Formula (9) wherein $L_1$, $R_1$, $X_1$, $R_2$, $X_2$, n, z, w and E are as defined previously.

The (poly)carbodiimide compounds, and similarly all the reaction intermediates and reagents, may be purified via conventional methods known to those skilled in the art, such as extraction with water and water-immiscible organic solvent, facilitation, centrifugation, filtration and/or chromatography.

**Example**

**Example 1: Process for synthesizing the (poly)carbodiimide compound**

50 g of 4,4'-dicyclohexylmethane diisocyanate and 0.5 g of 4,5-dihydro-3-methyl-1-phenyl-1H-phosphole 1-oxide were placed with stirring in a 500 mL three-necked roundbottomed flask equipped with a thermometer, a stirrer and a reflux tube.

The reaction medium was heated at 140°C under nitrogen for 4 hours, the reaction being monitored by infrared spectroscopy by means of the absorption of the isocyanate functions between 2200 and 2300 $cm^{-1}$, and then cooled to 120°C.

A mixture of 5.3 g of polyethylene glycol monomer methyl ether and 1.2 g of 1,4-butanediol are introduced with stirring into the reaction medium. The temperature of 120°C is maintained until the isocyanate functions have totally disappeared, monitored by infrared spectroscopy at 2200-2300 $cm^{-1}$, and is then cooled to room temperature.

After cooling to room temperature, the reaction medium is poured dropwise with vigorous stirring into a 500 mL glass beaker containing 85 g of distilled water, to give the desired product in the form of a translucent yellow liquid.

**Example 2:**

Base Coat Composition: amounts expressed in g of active material as obtained/100 g

[Tables 1]

| Composition | C1 |
|---|---|
| Polycarbodiimide(1) | 24 |
| Amodimethicone (and) Trideceth-5 (and) Trideceth-10 (2) | 5 |
| Preserving agent(s), optionally neutralized thickener | qs |
| Water | qs 100 |
| [00508] (1) synthesized according to the synthetic process described in example 1 (containing 40% active material in water),<br>(2) sold by the company Wacker under the name Belsil ADM LOG 1 | |

[Tables 2]

| Composition | C2 |
|---|---|
| Acrylates copolymer(3) | 40 |
| Amodimethicone (and) Trideceth-5 (and) Trideceth-10 (2) | 5 |
| Iron oxide (CI 77491) | 12 |

(continued)

| Composition | C2 |
|---|---|
| Preserving agent(s), optionally neutralized thickener | qs |
| Water | qs 100 |
| (3) sold by the company Daito Kasei Kogyo under the trade name Daitosol 3000SLPN-PE1 (aqueous dispersion containing 30% active material) | |

Composition C1 is mixed with composition C2 in a 50/50 ratio to obtain composition C.
Top Coat Composition: amounts expressed in g of active material as obtained/100 g

[Tables 3]

| Composition | D |
|---|---|
| Organopolysiloxane bearing carboxylic groups(4) | 2 |
| Isododecane | qs 100 |
| (4) sold by the company Shin-Etsu under the trade name X-22-3701E | |

Protocol:

Composition C (base coat composition) is applied to locks of natural dry hair containing 90% white hairs, in a proportion of 0.5 g of composition per gram of lock.
The locks of hair are then dried with a hairdryer with a comb, and then combed.
Next, two different protocols are then applied to the locks of hair thus treated:

- composition D (top coat composition) is applied to said locks of dry hair treated with composition C, in a proportion of 0.5 g of composition per gram of lock; or
- no composition D (top coat composition) is applied to the locks of hair treated with composition C.

The locks of hair treated with composition C and composition C + D are then combed and dried with a hairdryer. The locks of hair are left at room temperature for 24 hours.

Results:

The locks of hair treated with composition C + D show smooth, uniform, colored coating of the hair and have good cosmetic properties, notably in terms of softness, feel and good hair strand separation.
The locks of hair treated with composition C alone show colored coating of the hair but have poorer cosmetic properties, notably in terms of softness and feel, than the locks of hair treated with composition C + D.

**Example 3:**

Composition C as described in example 2 is prepared.
The compositions below are prepared.
Base Coat Composition: amounts expressed in g of active material as obtained/100 g

[Tables 4]

| Composition | C1' |
|---|---|
| Polycarbodiimide(1) | 24 |
| Preserving agent(s), optionally neutralized thickener | qs |
| Water | qs 100 |
| (1) synthesized according to the synthetic process described in example 1 (containing 40% active material in water). | |

[Tables 5]

| Composition | C2' |
|---|---|
| Acrylates copolymer | 40 |
| Iron oxide (CI 77491) | 12 |
| Preserving agent(s), optionally neutralized thickener | qs |
| Water | qs 100 |
| (2) sold by the company Daito Kasei Kogyo under the trade name Daitosol 3000SLPN-PE1 | |

Composition C1' is mixed with composition C2' in a 50/50 ratio to obtain composition C'.

Protocol:

Compositions C and C' (base coat composition) are applied to locks of natural dry hair containing 90% white hairs, in a proportion of 0.5g of composition per gram of lock.

The locks of hair are then dried with a hairdryer with a comb, and then combed.

Next, composition D (top coat composition) described in example 2 is applied to said locks of dry hair treated with composition C and with composition C', in a proportion of 0.5 g of composition per gram of lock.

The locks of hair treated with composition D are then combed and dried with a hairdryer. The locks of hair are left at room temperature for 24 hours.

The locks of hair thus dyed are then subjected to a test of several repeated shampoo washes so as to evaluate the fastness (persistence) of the coloring obtained with respect to shampoo washing, according to the shampoo wash protocol described below.

Shampoo wash protocol:

The locks of dyed hair are combed and moistened with water at 35°C before being passed between the fingers five times for 5 seconds. The locks of hair are then drained between two fingers.

A standard shampoo (Garnier Ultra Doux) is applied uniformly to the dyed locks, in a proportion of 0.4 g of standard shampoo per gram of locks, the locks of hair being massaged gently along the length (6 passes) for 15 seconds, from the root to the end.

The locks of hair are then placed in a watch glass and left to stand for 1 minute.

Next, the locks of hair are rinsed with water while passing the lock between the fingers (15 passes). The locks of hair are then drained between two fingers before the next shampoo wash.

Once the tests of several shampoo washes have been performed, the locks of hair are combed and dried with a hairdryer.

Results:

The persistence of the color of the locks was evaluated in the CIE L* a* b* system, using a Minolta Spectrophotometer CM3600A colorimeter (illuminant D65, angle 10°, specular component included).

In this L*a*b* system, L* represents the intensity of the color, a* indicates the green/red color axis and b* the blue/yellow color axis.

The persistence of the coloring is evaluated by the color difference ΔE between the dyed locks before shampooing, then after having undergone five shampoo washes according to the protocol described above. The lower the ΔE value, the more persistent the color with respect to shampoo washing.

The ΔE value is calculated according to the following equation:

[Math.1]

$$\Delta E = \sqrt{(L^* - L_o^*)^2 + (a^* - a_o^*)^2 + (b^* - b_o^*)^2}$$

In this equation, L*a*b* represent the values measured after dyeing the hair and after performing the shampoo washes, and $L^*_0 a_0^* b_0^*$ represent the values measured after dyeing the hair but before shampoo washing.

[Tables 6]

| Compositions | Number of shampoo washes | L* | a* | b* | ΔE |
|---|---|---|---|---|---|
| Composition C+D | 0 | 35.2 | 29.5 | 22.9 | 0 |
| | 5 | 37.0 | 27.7 | 21.5 | 2.9 |
| Composition C'+D | 0 | 34.8 | 28.1 | 21.9 | - |
| | 5 | 42.9 | 21.4 | 16.8 | 11.7 |

The locks of hair dyed with composition C + D according to the invention and washed with five shampoo washes have low ΔE values. Thus, the colored coating of the keratin fibers obtained with compositions C+ D shows good persistence with respect to shampoo washing.

The locks of hair dyed with composition C'+ D and washed with five shampoo washes also show good persistence with respect to shampoo washing.

**Example 4:**

Base Coat Composition: amounts expressed in g of active material as obtained/100 g

[Tables 7]

| **Composition** | **C"** |
|---|---|
| Polycarbodiimide(1) | 12 |
| Amodimethicone (and) Trideceth-5 (and) Trideceth-10 (2) | 5 |
| Acrylates copolymer(3) | 20 |
| Iron oxide (CI 77491) | 6 |
| Preserving agent(s), optionally neutralized thickener | qs |
| Water | qs 100 |

(1) synthesized according to the synthetic process described in example 1 (containing 40% active material in water).
(2) sold by the company Wacker under the name Belsil ADM LOG 1
(3) sold by the company Daito Kasei Kogyo under the trade name Daitosol 3000SLPN-PE1 (aqueous dispersion containing 30% active material)

Protocol:

Composition C" (base coat composition) is applied to locks of natural dry hair containing 90% white hairs, in a proportion of 0.5 g of composition per gram of lock.

The locks of hair are then dried with a hairdryer with a comb, and then combed.

Next, composition D (top coat composition) described in example 2 is applied to said locks of dry hair treated with composition C", in a proportion of 0.5 g of composition per gram of lock.

The locks of hair treated with composition D are then combed and dried with a hairdryer. The locks of hair are left at room temperature for 24 hours.

Results:

The locks of hair treated with composition C" + D show smooth, uniform, colored coating of the hair and have good cosmetic properties, notably in terms of softness, feel and good hair strand separation. The colored coating of the keratin fibers shows good persistence with respect to shampoo washing.

## Claims

1. A process for dyeing hair, comprising the application to the hair of a composition (C) comprising:

a) at least one (poly)carbodiimide compound,
b) at least one aqueous dispersions of particles of polymer(s) chosen from polyurethanes, acrylic polymers, and mixtures thereof,
c) at least one silicone, and
d) at least one coloring agent chosen from pigments, direct dyes and mixtures thereof.

**2.** The process as claimed in claim 1, **characterized in that** the (poly)carbodiimide compound(s) are chosen from the compounds of formula (I) below:

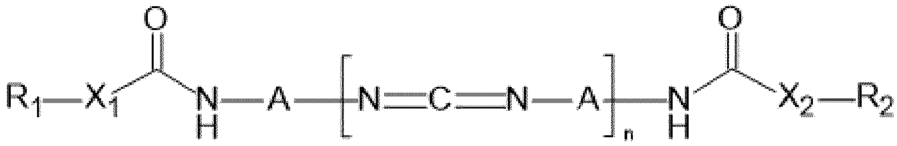

(I)

in which:

- $\mathbf{X_1}$ and $\mathbf{X_2}$ independently represent an oxygen atom O, a sulfur atom S or an NH group,
- $\mathbf{R_1}$ and $\mathbf{R_2}$ independently represent a hydrocarbon-based radical, preferably alkyl, optionally interrupted with one or more heteroatoms,
- **n** denotes an integer ranging from 1 to 100, and
- **A** is a monomer chosen from the compounds below:

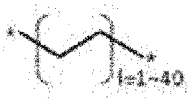
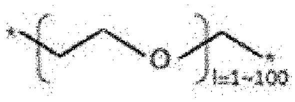
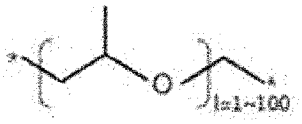
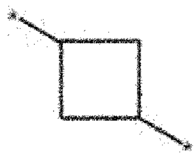

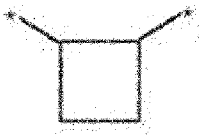
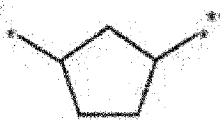
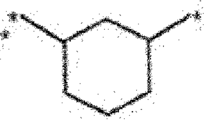
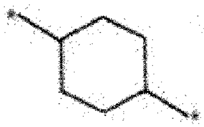
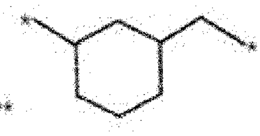

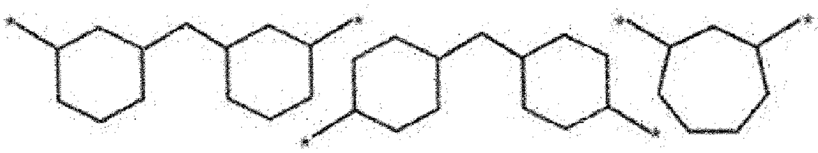
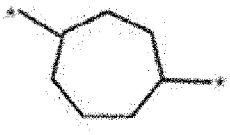

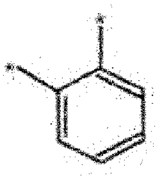
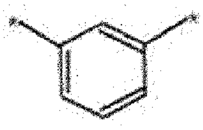
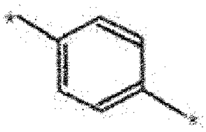
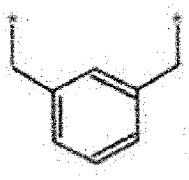
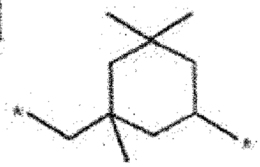

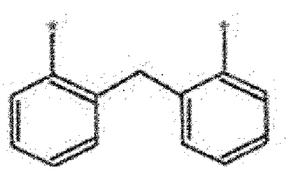
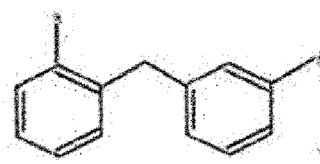
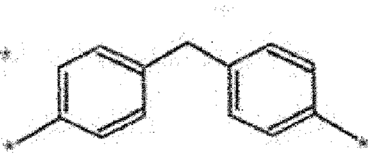

**3.** The process as claimed in claim 1, **characterized in that** the (poly)carbodiimide compound(s) are chosen from the compounds of formula (II) below:

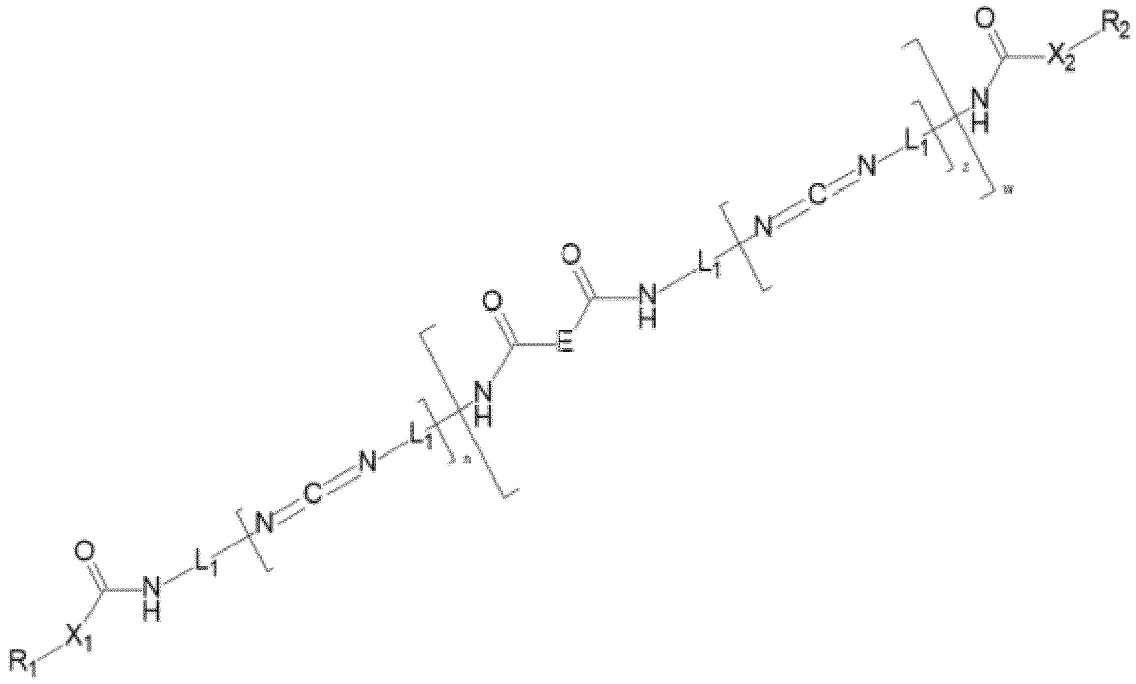

(II)

in which formula (II):

- $\mathbf{X_1}$ and $\mathbf{X_2}$ independently represent an oxygen atom O, a sulfur atom S or a nitrogen atom NH;
- $\mathbf{R_1}$ and $\mathbf{R_2}$ independently represent a hydrocarbon-based radical optionally interrupted with one or more heteroatoms,
- **n** and **z** denote an integer ranging from 1 to 20, with $n+z \geq 2$ and w denotes an integer ranging from 1 to 3;
- $\mathbf{L_1}$ independently represents a $C_1$-$C_{18}$ divalent aliphatic hydrocarbon-based radical, a $C_3$-$C_{15}$ cycloalkylene radical, a $C_3$-$C_{12}$ heterocycloalkylene group or a $C_6$-$C_{14}$ arylene group, and mixtures thereof;
- E independently represents a group chosen from:

- $-O-R_3-O-$; $-S-R_4-S-$; $-R_5-N(R_6)-R_4-N(R_6)-R_5-$;

in which $\mathbf{R_3}$ and $\mathbf{R_4}$ independently represent a divalent hydrocarbon-based radical optionally interrupted with one or more heteroatoms,
- $\mathbf{R_5}$ independently represents a covalent bond or a saturated divalent hydrocarbon-based radical, optionally interrupted with one or more heteroatoms;
- $\mathbf{R_6}$ independently represents a hydrogen atom or a hydrocarbon-based radical, optionally interrupted with one or more heteroatoms.

**4.** The process as claimed in claim 3, **characterized in that** the (poly)carbodiimide compound(s) are chosen from the compounds of formula (II) in which:

- $\mathbf{X_1}$ and $\mathbf{X_2}$ independently represent an oxygen atom;
- $\mathbf{R_1}$ and $\mathbf{R_2}$ are independently chosen from dialkylamino alcohols, alkyl esters of hydroxycarboxylic acid and monoalkyl ethers of (poly)alkylene glycol, in which a hydroxyl group has been removed, and mixtures thereof;
- **n** and **z** denote an integer ranging from 1 to 20, with $n+z \geq 2$ and w is equal to 1;
- $\mathbf{L_1}$ is chosen from a $C_1$-$C_{18}$ divalent aliphatic hydrocarbon-based radical, a $C_3$-$C_{15}$ cycloalkylene radical, a $C_3$-$C_{12}$ heterocycloalkylene group or a $C_6$-$C_{14}$ arylene group, and mixtures thereof;
- **E** independently represents a group chosen from:

- $-O-R_3-O-$; $-S-R_4-S-$; $-R_5-N(R_6)-R_4-N(R_6)-R_5-$;

in which **$R_3$** and **$R_4$** are independently chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof;
- when **$R_5$** is not a covalent bond, **$R_5$** is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof; and
- **$R_6$** is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof.

**5.** The process as claimed in either of claims 3 and 4, **characterized in that** the (poly)carbodiimide compound(s) are chosen from the compounds of formula (II) in which:

- **$X_1$** and **$X_2$** independently represent an oxygen atom;
- **$R_1$** and **$R_2$** are, independently, monoalkyl ethers of (poly)alkylene glycol, in which a hydroxyl group has been removed;
- **n** and **z** denote an integer ranging from 1 to 20, with $n+z \geq 2$ and w is equal to 1;
- **$L_1$** is a $C_3$-$C_{15}$ cycloalkylene radical;
- **E** independently represents a group chosen from:

- $-O-R_3-O-$; $-S-R_4-S-$; $-R_5-N(R_6)-R_4-N(R_6)-R_5-$;

in which **$R_3$** and **$R_4$** are independently chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof;
- when **$R_5$** is not a covalent bond, **$R_5$** is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof; and
- **$R_6$** is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof.

**6.** The process as claimed in any one of claims 3 to 5, **characterized in that** the (poly)carbodiimide compound(s) are chosen from the compounds of formula (II) in which:

- **$X_1$** and **$X_2$** independently represent an oxygen atom;
- **$R_1$** and **$R_2$** independently represent the compound of formula (VI) below:

$$R_{13}-[O-CH_2-C(H)(R_{14})]_q- \qquad (VI)$$

in which **$R_{13}$** represents a $C_1$-$C_4$ alkyl group or a phenyl, preferably a $C_1$-$C_4$ alkyl group, more preferentially a methyl, $R_{14}$ represents a hydrogen atom or a $C_1$-$C_4$ alkyl group, preferably a hydrogen atom and **q** denotes an integer ranging from 4 to 30;
- **n** and **z** denote an integer ranging from 2 to 20, with **n+z** ranging from 4 to 10 and w is equal to 1;
- **$L_1$** is an $C_3$-$C_{15}$ cycloalkylene radical such as cyclopentylene, cycloheptylene, cyclohexylene and 4,4-dicyclohexylenemethane; and
- **E** represents a group $-O-R_3-O-$ in which **$R_3$** is chosen from a $C_6$-$C_{14}$ arylene radical, a $C_3$-$C_{12}$ cycloalkylene radical, a linear or branched $C_1$-$C_{18}$ alkylene radical, optionally interrupted with one or more heteroatoms, and mixtures thereof.

**7.** The process as claimed in any one of claims 3 to 6, **characterized in that** the (poly)carbodiimide compound(s) are chosen from the compounds of formula (II) in which:

- **$X_1$** and **$X_2$** independently represent an oxygen atom;
- **$R_1$** and **$R_2$** independently represent the compound of formula (VI) below:

$$R_{13}-[O-CH_2-C(H)(R_{14})]_q- \qquad (VI)$$

in which **$R_{13}$** represents a $C_1$-$C_4$ alkyl group or a phenyl, preferably a $C_1$-$C_4$ alkyl group, more preferentially a methyl, $R_{14}$ represents a hydrogen atom or a $C_1$-$C_4$ alkyl group, preferably a hydrogen atom and **q** denotes an integer ranging from 4 to 30;
- **n and z** denote an integer ranging from 1 to 20, with n+z ranging from 4 to 10 and w is equal to 1;
- **$L_1$** is an $C_3$-$C_{15}$ cycloalkylene radical such as cyclopentylene, cycloheptylene, cyclohexylene and 4,4-dicyclohexylenemethane, preferably 4,4-dicyclohexylenemethane; and
- **E** represents a group -O-$R_3$-O- in which **$R_3$** represents a linear or branched $C_1$-$C_{18}$ alkylene radical such as methylene, butylene, propylene or ethylene, optionally interrupted with one or more heteroatoms.

**8.** The process as claimed in any one of claims 3 to 7, **characterized in that** the (poly)carbodiimide compound(s) are chosen from the compounds of formula (XII) below:

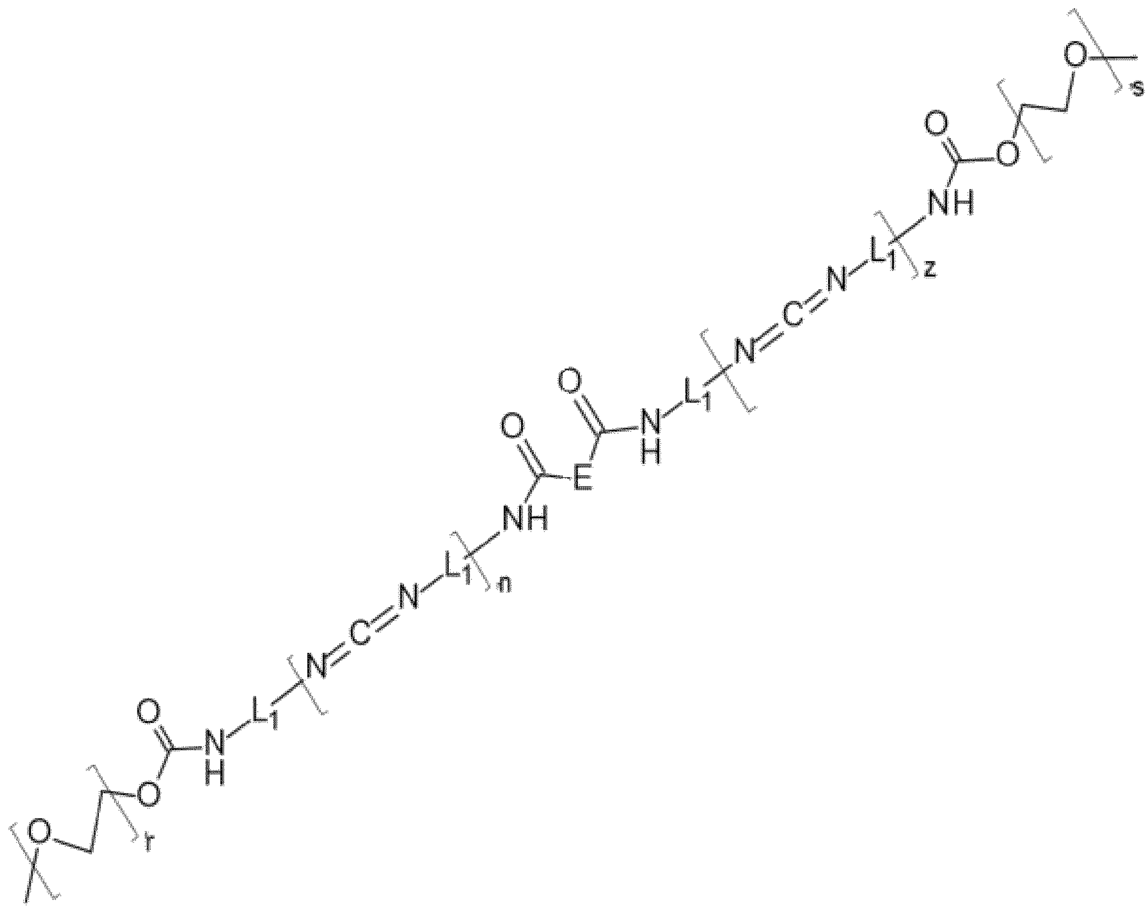

(XII)

in which **$L_1$** is 4,4-dicyclohexylenemethane, **n** and **z** denote an integer ranging from 1 to 20, with n+z ranging from 4 to 10, **E** represents a group -O-$R_3$-O- in which **$R_3$** represents a linear or branched $C_1$-$C_{18}$ alkylene radical such as methylene, propylene, butylene or ethylene, optionally interrupted with one or more heteroatoms, and **r** and **s** denote an integer ranging from 4 to 30.

**9.** The process as claimed in any one of the preceding claims, **characterized in that** the total amount of the (poly)carbodiimide compound(s) ranges from 0.01% to 40% by weight, preferably from 0.1% to 30% by weight, better still from 0.5% to 25% by weight and even better still from 1% to 10% by weight, relative to the total weight of composition (C).

**10.** The process as claimed in any one of the preceding claims, **characterized in that** the aqueous dispersion(s) of particles of polymer(s) are chosen from aqueous dispersions of acrylic polymer particles, and preferably from

aqueous dispersions of film-forming acrylic polymer.

**11.** The process as claimed in any one of the preceding claims, **characterized in that** the acrylic polymer(s) comprise one or more units derived from the following monomers:

a) (meth)acrylic acid; and
b) $C_1$ to $C_{30}$, more preferentially $C_1$ to $C_{20}$, better still $C_1$ to $C_{10}$, and even more particularly $C_1$ to $C_4$, alkyl (meth) acrylates.

**12.** The process as claimed in any one of the preceding claims, **characterized in that** the total amount of the aqueous dispersion(s) of polymer particles present in the composition ranges from 0.1% to 40% by weight, more preferentially from 0.1% to 35% by weight and better still from 0.2% to 30% by weight, relative to the total weight of composition (C).

**13.** The process as claimed in any one of the preceding claims, **characterized in that** the silicone(s) are chosen from non-amino silicones, amino silicones and mixtures thereof.

**14.** The process as claimed in any one of the preceding claims, **characterized in that** said silicone(s) are present in a total amount ranging from 0.01% to 20% by weight relative to the total weight of the composition, preferably from 0.05% to 15% by weight, more preferentially from 0.1% to 10% by weight and even more preferentially from 0.1% to 5% by weight relative to the total weight of composition (C).

**15.** The process as claimed in any one of the preceding claims, **characterized in that** it also comprises a step of applying to the hair a composition (D) comprising at least one silicone compound comprising at least one carboxylic group.

**16.** The process as claimed in Claim 15, **characterized in that** the silicone compound(s) comprising at least one carboxylic group are chosen from the organosiloxanes of formula (XXVII) below:

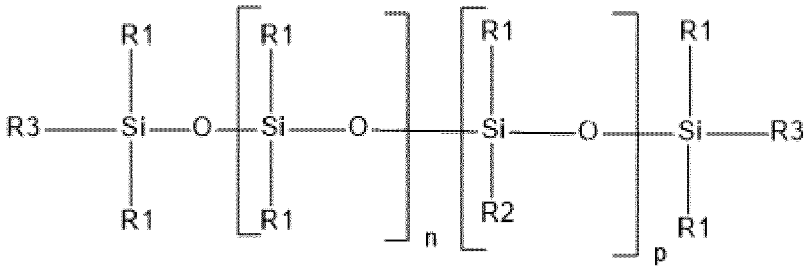

(XXVII)

in which:

- **R1** independently represent an alkyl group containing from 1 to 20 carbon atoms, preferably from 1 to 10 carbon atoms; a hydroxyl group; an alkoxy group containing from 1 to 20 carbon atoms or an aryl group containing from 6 to 12 carbon atoms;
- **R2** independently represents a group R4-COOM with **R4** representing a linear or branched alkylene group containing from 1 to 20 carbon atoms, preferably from 4 to 16 carbon atoms, and **M** representing a hydrogen atom; an alkali metal or alkaline-earth metal or a quaternary ammonium NR'3, with **R',** which may be identical or different, representing H or alkyl containing from 1 to 4 carbon atoms; a pyrrolidone radical comprising a carboxylic group COOH or a group $R_a$-$(OR_b)_x$-COOM with $\mathbf{R_a}$ representing a linear or branched alkylene group containing from 1 to 4 carbon atoms, $\mathbf{R_b}$ representing an alkyl group containing from 1 to 4 carbon atoms, **x** being an integer ranging from 1 to 200; and **M** representing a hydrogen atom, an alkali metal or alkaline-earth metal or a quaternary ammonium $NR'_3$, with **R',** which may be identical or different, representing H or an alkyl containing from 1 to 4 carbon atoms;
- **R3** independently represent an alkyl group containing from 1 to 20 carbon atoms; a hydroxyl group; a group R4-COOM with **R4** representing a linear or branched alkylene group containing from 1 to 20 carbon atoms, preferably from 4 to 16 carbon atoms, and **M** representing a hydrogen atom; an alkali metal or alkaline-earth metal or a quaternary ammonium $NR'_3$, with **R',** which may be identical or different, representing H or alkyl containing from 1 to 4 carbon atoms; an alkoxy group containing from 1 to 20 carbon atoms; an aryl group containing from 6 to 12 carbon atoms or a group $R_a$-$(OR_b)_x$-COOM with $\mathbf{R_a}$ representing a linear or branched alkylene group containing

from 1 to 4 carbon atoms, $\mathbf{R_b}$ representing an alkyl group containing from 1 to 4 carbon atoms, **x** being an integer ranging from 1 to 200; and **M** representing a hydrogen atom, an alkali metal or alkaline-earth metal or a quaternary ammonium $NR'_3$, with **R'**, which may be identical or different, representing H or an alkyl containing from 1 to 4 carbon atoms;
- **n** denotes an integer ranging from 1 to 1000;
- **p** denotes an integer ranging from 0 to 1000;

it being understood that at least one of the radicals R2 and/or R3 comprises a carboxylic group COOH or COOM with **M** representing an alkali metal or alkaline-earth metal or a quaternary ammonium $NR'_3$, with **R'**, which may be identical or different, representing H or an alkyl containing from 1 to 4 carbon atoms.

**17.** The process as claimed in either of claims 15 and 16, **characterized in that** the silicone compound(s) comprising at least one carboxylic group are chosen from the organosiloxanes of formula (XXVIII) below:

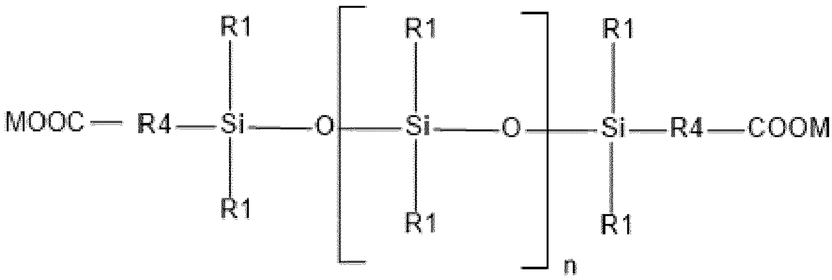

(XXVIII)

in which:

- R**1** independently represents a linear or branched alkyl group containing from 1 to 20 carbon atoms, preferably from 1 to 10 carbon atoms and better still from 1 to 6 carbon atoms, preferentially methyl;
- **R4** independently represents a linear or branched alkylene group containing from 1 to 20 carbon atoms, preferably from 4 to 16 carbon atoms; or a divalent group $R_a$-$(OR_b)_x$- with $\mathbf{R_a}$ representing a linear or branched alkylene group containing from 1 to 4 carbon atoms, $\mathbf{R_b}$ representing an alkylene group containing from 1 to 4 carbon atoms, and **x** being an integer ranging from 1 to 200;
- **M** independently represents a hydrogen atom, an alkali metal or alkaline-earth metal or a quaternary ammonium $NR'_3$, with **R'**, which may be identical or different, representing H or an alkyl containing from 1 to 4 carbon atoms;
- **n** denotes an integer ranging from 1 to 1000.

**18.** The process as claimed in either of claims 15 and 16, **characterized in that** the silicone compound(s) comprising at least one carboxylic group are chosen from the organosiloxanes of formula (XXIX) below:

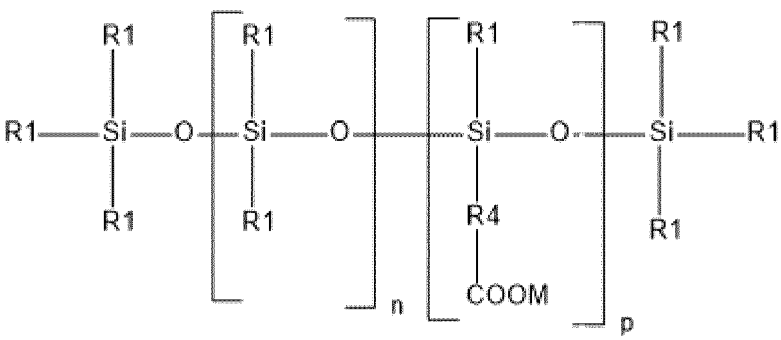

(XXIX)

in which:

- **R1** independently represents an alkyl group containing from 1 to 10 carbon atoms, preferably from 1 to 6 carbon

atoms, more preferentially a methyl;
- **R4** represents a saturated or unsaturated, linear or branched alkylene group containing from 1 to 20 carbon atoms, preferably from 4 to 16 carbon atoms; or a divalent group $R_a$-$(OR_b)_x$-with $\mathbf{R_a}$ representing a linear or branched alkylene group containing from 1 to 4 carbon atoms, $\mathbf{R_b}$ representing an alkylene group containing from 1 to 4 carbon atoms, and x being an integer ranging from 1 to 200;
- **M** represents a hydrogen atom, an alkali metal or alkaline-earth metal or a quaternary ammonium $NR'_3$, with **R'**, which may be identical or different, representing H or an alkyl containing from 1 to 4 carbon atoms;
- **p** denotes an integer ranging from 1 to 1000;
- **n** denotes an integer ranging from 1 to 1000.

**19.** The process as claimed in any one of claims 15 to 18, **characterized in that** the total amount of the silicone compound(s) comprising at least one carboxylic group present in composition (D) ranges from 0.01% to 20% by weight, more preferentially from 0.1% to 15% by weight and better still from 0.5% to 10% by weight, relative to the total weight of composition (D).

**20.** The process as claimed in any one of claims 15 to 19, **characterized in that** composition (D) comprises one or more oil(s), preferably chosen from $C_8$-$C_{16}$ alkanes, more preferentially from isododecane, isohexadecane, tetradecane and/or mixtures thereof.

**21.** The process as claimed in any one of claims 15 to 20, **characterized in that** composition (C) and composition (D) are applied simultaneously to the hair.

**22.** The process as claimed in any one of claims 15 to 20, **characterized in that** composition (D) is applied to the hair after the application of composition (C) to the hair.

**23.** A device for treating hair, comprising one or more compartments containing:

- in a first compartment (E1), a composition (C) as defined in any one of claims 1 to 14; and
- optionally, in a second compartment (E2), a composition (D) as defined in any one of claims 15 to 20.

**24.** A device for treating hair, comprising at least two compartments containing:

- in a first compartment (F1), a composition (C1) comprising a) at least one (poly)carbodiimide compound as defined in any one of claims 1 to 8; and
- in a second compartment (F2), a composition (C2) comprising at least one aqueous dispersion of particles of polymer(s) chosen from polyurethanes, acrylic polymers, and mixtures thereof, as defined in either of claims 10 and 11;
composition (C1) and/or composition (C2) comprising at least one coloring agent chosen from pigments, direct dyes, and mixtures thereof, and comprising at least one silicone as defined in claim 13; and
- optionally, in a third compartment (F3), a composition (D) as defined in any one of claims 15 to 20.

## Patentansprüche

**1.** Verfahren zum Färben von Haaren, umfassend das Aufbringen einer Folgendes umfassenden Zusammensetzung (C) auf das Haar:

a) mindestens eine (Poly)carbodiimidverbindung,
b) mindestens eine wässrige Dispersion von aus Polyurethanen, Acrylpolymeren und Mischungen davon ausgewählten Polymerpartikeln,
c) mindestens ein Silikon und
d) mindestens ein aus Pigmenten, Direktfarbstoffen und Mischungen davon ausgewähltes Farbmittel.

**2.** Verfahren nach Anspruch 1, **dadurch gekennzeichnet, dass** die (Poly)carbodiimidverbindung(en) aus den Verbindungen der folgenden Formel (I) ausgewählt ist/sind:

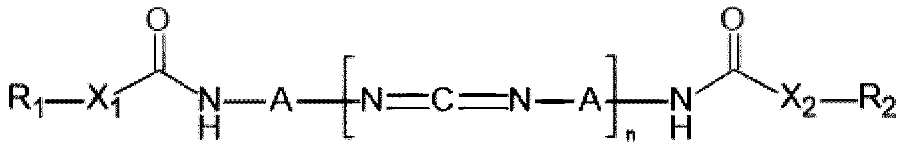

(I)

wobei:

- $X_1$ und $X_2$ unabhängig für ein Sauerstoffatom O, ein Schwefelatom S oder eine NH-Gruppe stehen,
- $R_1$ und $R_2$ unabhängig für einen Rest auf Kohlenwasserstoffbasis, vorzugsweise Alkyl, der gegebenenfalls durch ein oder mehrere Heteroatome unterbrochen ist, stehen,
- n eine ganze Zahl im Bereich von 1 bis 100 bedeutet und
- A für ein aus den folgenden Verbindungen ausgewähltes Monomer steht:

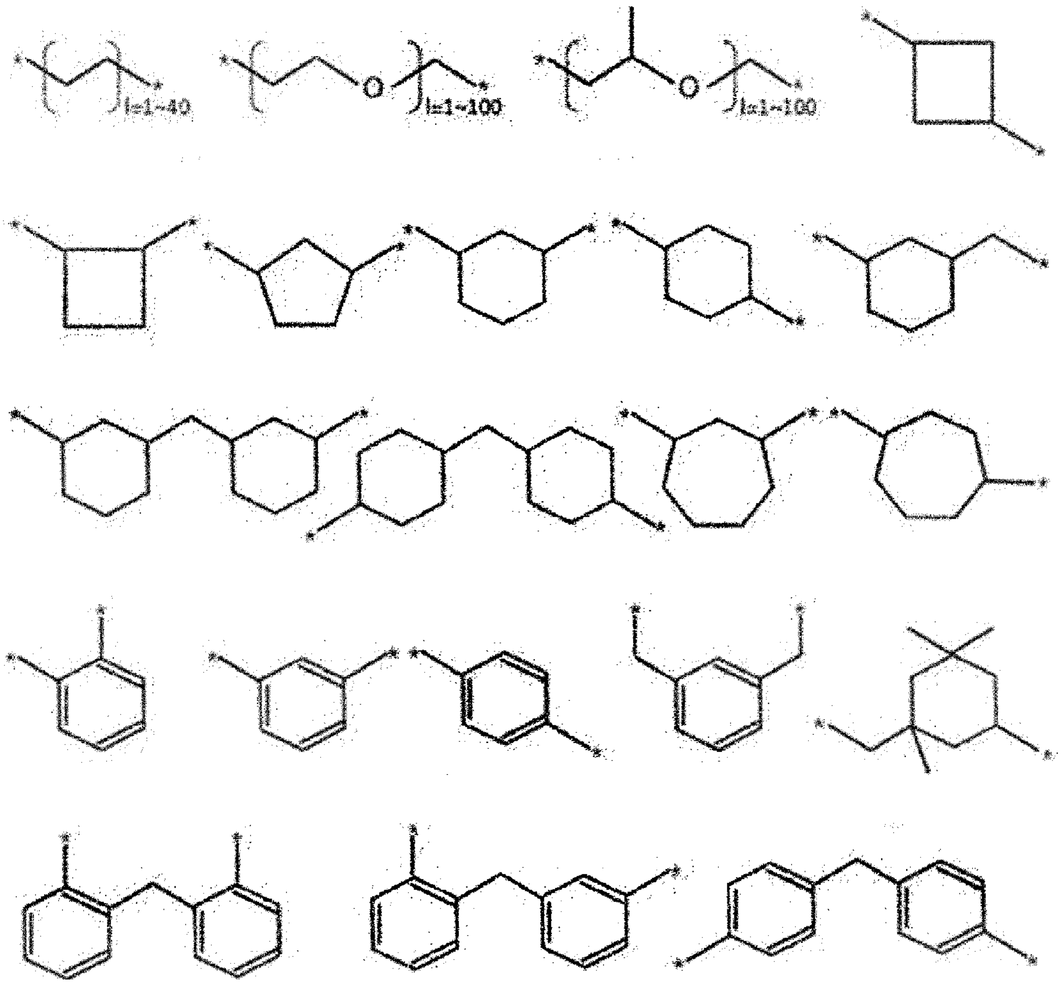

**3.** Verfahren nach Anspruch 1, **dadurch gekennzeichnet, dass** die (Poly)carbodiimidverbindung(en) aus den Verbindungen der folgenden Formel (II) ausgewählt ist/sind:

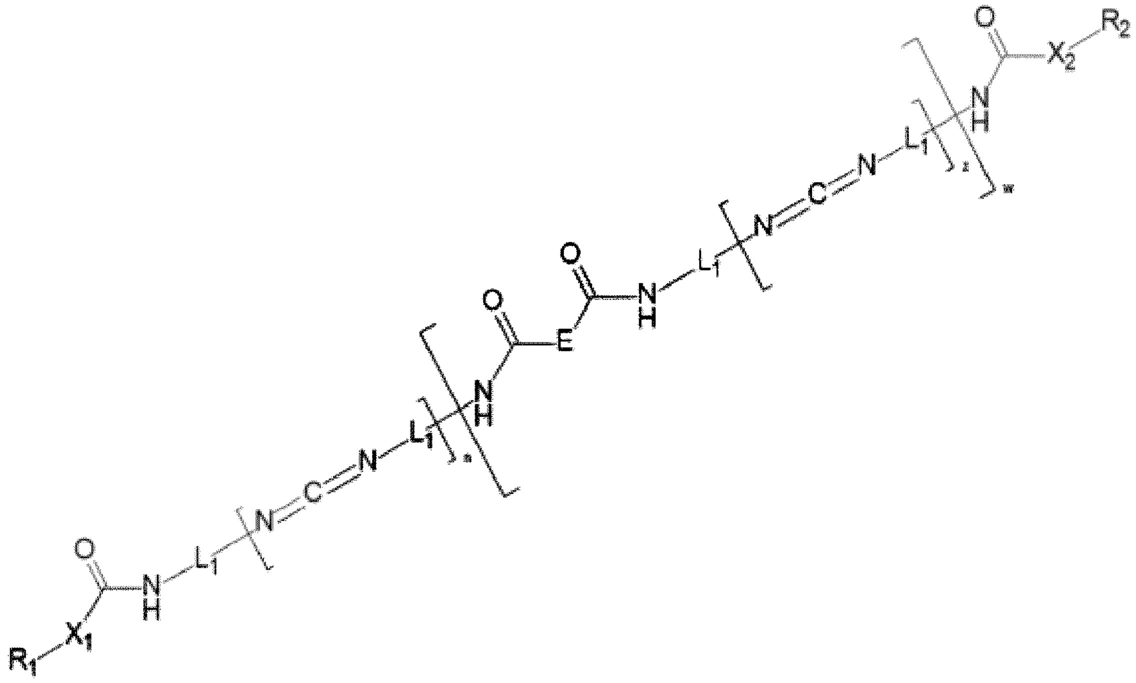

(II)

wobei in Formel (II):

- $X_1$ und $\mathbf{X_2}$ unabhängig für ein Sauerstoffatom O, ein Schwefelatom S oder ein Stickstoffatom stehen,
- $R_1$ und $R_2$ unabhängig für einen Rest auf Kohlenwasserstoffbasis, der gegebenenfalls durch ein oder mehrere Heteroatome unterbrochen ist, stehen,
- n und z eine ganze Zahl im Bereich von 1 bis 20 mit $n+z \geq 2$ bedeuten und w eine ganze Zahl im Bereich von 1 bis 3 bedeutet,
- $L_1$ unabhängig für einen zweiwertigen aliphatischen $C_1$-$C_{13}$-Rest auf Kohlenwasserstoffbasis, einen $C_3$-$C_{15}$-Cycloalkylenrest, eine $C_3$-$C_{12}$-Heterocycloalkylengruppe oder eine $C_6$-$C_{14}$-Arylengruppe und Mischungen davon steht,
- E unabhängig für eine aus den Folgenden ausgewählte Gruppe steht:

- $-O-R_3-O-$, $-S-R_4-S-$, $-R_5-N(R_6)-R_4-N(R_6)-R_5-$,

wobei $R_3$ und $R_4$ unabhängig für einen zweiwertigen Rest auf Kohlenwasserstoffbasis, der gegebenenfalls durch ein oder mehrere Heteroatome unterbrochen ist, stehen,
- $R_5$ unabhängig für eine kovalente Bindung oder einen gesättigten zweiwertigen Rest auf Kohlenwasserstoffbasis, der gegebenenfalls durch ein oder mehrere Heteroatome unterbrochen ist, steht,
- $R_6$ unabhängig für ein Wasserstoffatom oder einen Rest auf Kohlenwasserstoffbasis, der gegebenenfalls durch ein oder mehrere Heteroatome unterbrochen ist, steht.

4. Verfahren nach Anspruch 3, **dadurch gekennzeichnet, dass** die (Poly)carbodiimidverbindung(en) aus den Verbindungen der Formel (II) ausgewählt ist/sind, wobei:

- $X_1$ und $X_2$ unabhängig für ein Sauerstoffatom stehen,
- $R_1$ und $R_2$ unabhängig aus Dialkylaminoalkoholen, Hydroxycarbonsäurealkylestern und Monoalkylethern von (Poly)alkylenglykol, in denen eine Hydroxylgruppe entfernt wurde, und Mischungen davon ausgewählt sind,
- n und z eine ganze Zahl im Bereich von 1 bis 20 mit $n+z \geq 2$ bedeuten und w gleich 1 ist,
- $L_1$ aus einem zweiwertigen aliphatischen $C_1$-$C_{13}$-Rest auf Kohlenwasserstoffbasis, einem $C_3$-$C_{15}$-Cycloalkylenrest, einer $C_3$-$C_{12}$-Heterocycloalkylengruppe oder einer $C_6$-$C_{14}$-Arylengruppe und Mischungen davon ausgewählt ist,
- E unabhängig für eine aus den Folgenden ausgewählte Gruppe steht:

- $-O-R_3-O-$, $-S-R_4-S-$, $-R_5-N(R_6)-R_4-N(R_6)-R_6-$,

wobei $R_3$ und $R_4$ unabhängig aus einem $C_6$-$C_{14}$-Arylenrest, einem $C_3$-$C_{12}$-Cycloalkylenrest, einem geradkettigen oder verzweigten $C_1$-$C_{13}$-Alkylenrest, der gegebenenfalls durch ein oder mehrere Heteroatome unterbrochen ist, und Mischungen davon ausgewählt sind,

- dann, wenn $R_5$ keine kovalente Bindung ist, $R_5$ aus einem $C_6$-$C_{14}$-Arylenrest, einem $C_3$-$C_{12}$-Cycloalkylenrest, einem geradkettigen oder verzweigten $C_1$-$C_{18}$-Alkylenrest, der gegebenenfalls durch ein oder mehrere Heteroatome unterbrochen ist, und Mischungen davon ausgewählt ist und
- $R_6$ aus einem $C_6$-$C_{14}$-Arylenrest, einem $C_3$-$C_{12}$-Cycloalkylenrest, einem geradkettigen oder verzweigten $C_1$-$C_{18}$-Alkylenrest, der gegebenenfalls durch ein oder mehrere Heteroatome unterbrochen ist, und Mischungen davon ausgewählt ist.

5. Verfahren nach einem der Ansprüche 3 und 4, **dadurch gekennzeichnet, dass** die (Poly)carbodiimidverbindung(en) aus den Verbindungen der Formel (II) ausgewählt ist/sind, wobei:

- $X_1$ und $X_2$ unabhängig für ein Sauerstoffatom stehen,
- $R_1$ und $R_2$ unabhängig für Monoalkylether von (Poly)alkylenglykol, in denen eine Hydroxylgruppe entfernt wurde, stehen,
- n und z eine ganze Zahl im Bereich von 1 bis 20 mit $n+z \geq 2$ bedeuten und w gleich 1 ist,
- $L_1$ für einen $C_3$-$C_{15}$-Cycloalkylenrest steht,
- E unabhängig für eine aus den Folgenden ausgewählte Gruppe steht:

- $-O-R_3-O-$, $-S-R_4-S-$, $-R_5-N(R_6)-R_4-N(R_6)-R_5-$,

wobei $R_3$ und $R_4$ unabhängig aus einem $C_6$-$C_{14}$-Arylenrest, einem $C_3$-$C_{12}$-Cycloalkylenrest, einem geradkettigen oder verzweigten $C_1$-$C_{18}$-Alkylenrest, der gegebenenfalls durch ein oder mehrere Heteroatome unterbrochen ist, und Mischungen davon ausgewählt sind,

- dann, wenn $R_5$ keine kovalente Bindung ist, $R_5$ aus einem $C_6$-$C_{14}$-Arylenrest, einem $C_3$-$C_{12}$-Cycloalkylenrest, einem geradkettigen oder verzweigten $C_1$-$C_{18}$-Alkylenrest, der gegebenenfalls durch ein oder mehrere Heteroatome unterbrochen ist, und Mischungen davon ausgewählt ist und
- $R_6$ aus einem $C_6$-$C_{14}$-Arylenrest, einem $C_3$-$C_{12}$-Cycloalkylenrest, einem geradkettigen oder verzweigten $C_1$-$C_{18}$-Alkylenrest, der gegebenenfalls durch ein oder mehrere Heteroatome unterbrochen ist, und Mischungen davon ausgewählt ist.

6. Verfahren nach einem der Ansprüche 3 bis 5, **dadurch gekennzeichnet, dass** die (Poly)carbodiimidverbindung(en) aus den Verbindungen der Formel (II) ausgewählt ist/sind, wobei:

- $X_1$ und $X_2$ unabhängig für ein Sauerstoffatom stehen,
- $R_1$ und $R_2$ unabhängig für die Verbindung der folgenden Formel (VI) stehen:

$$R_{13}\text{-}[O\text{-}CH_2\text{-}C(H)(R_{14})]_q\text{-} \qquad (VI)$$

wobei $R_{13}$ für eine $C_1$-$C_4$-Alkylgruppe oder ein Phenyl, vorzugsweise eine $C_1$-$C_4$-Alkylgruppe, besonders bevorzugt Methyl, steht, $R_{14}$ für ein Wasserstoffatom oder eine $C_1$-$C_4$-Alkylgruppe, vorzugsweise ein Wasserstoffatom, steht und q eine ganze Zahl im Bereich von 4 bis 30 bedeutet,

- n und z eine ganze Zahl im Bereich von 2 bis 20 mit n+z im Bereich von 4 bis 10 bedeuten und w gleich 1 ist,
- $L_1$ für einen $C_3$-$C_{15}$-Cycloalkylenrest wie Cyclopentylen, Cycloheptylen, Cyclohexylen und 4,4-Dicyclohexylenmethan steht und
- E für eine Gruppe $-O-R_3-O-$ steht, wobei $R_3$ aus einem $C_6$-$C_{14}$-Arylenrest, einem $C_3$-$C_{12}$-Cycloalkylenrest, einem geradkettigen oder verzweigten $C_1$-$C_{18}$-Alkylenrest, der gegebenenfalls durch ein oder mehrere Heteroatome unterbrochen ist, und Mischungen davon ausgewählt ist.

7. Verfahren nach einem der Ansprüche 3 bis 6, **dadurch gekennzeichnet, dass** die (Poly)carbodiimidverbindung(en) aus den Verbindungen der Formel (II) ausgewählt ist/sind, wobei:

- $X_1$ und $X_2$ unabhängig für ein Sauerstoffatom stehen,
- $R_1$ und $R_2$ unabhängig für die Verbindung der folgenden Formel (VI) stehen:

$R_{13}$-[O-$CH_2$-C(H)($R_{14}$)]$_q$- (VI)

wobei $R_{13}$ für eine $C_1$-$C_4$-Alkylgruppe oder ein Phenyl, vorzugsweise eine $C_1$-$C_4$-Alkylgruppe, besonders bevorzugt Methyl, steht, $R_{14}$ für ein Wasserstoffatom oder eine $C_1$-$C_4$-Alkylgruppe, vorzugsweise ein Wasserstoffatom, steht und q eine ganze Zahl im Bereich von 4 bis 30 bedeutet,

- n und z eine ganze Zahl im Bereich von 1 bis 20 mit n+z im Bereich von 4 bis 10 bedeuten und w gleich 1 ist,
- $L_1$ für einen $C_3$-$C_{15}$-Cycloalkylenrest wie Cyclopentylen, Cycloheptylen, Cyclohexylen und 4,4-Dicyclohexylenmethan, vorzugsweise 4,4-Dicyclohexylenmethan, steht und
- E für eine Gruppe -O-$R_3$-O- steht, wobei $R_3$ für einen geradkettigen oder verzweigten $C_1$-$C_{18}$-Alkylenrest wie Methylen, Butylen, Propylen oder Ethylen, der gegebenenfalls durch ein oder mehrere Heteroatome unterbrochen ist, steht.

**8.** Verfahren nach einem der Ansprüche 3 bis 7, **dadurch gekennzeichnet**, **dadurch gekennzeichnet, dass** die (Poly) carbodiimidverbindung(en) aus den Verbindungen der folgenden Formel (XII) ausgewählt ist/sind:

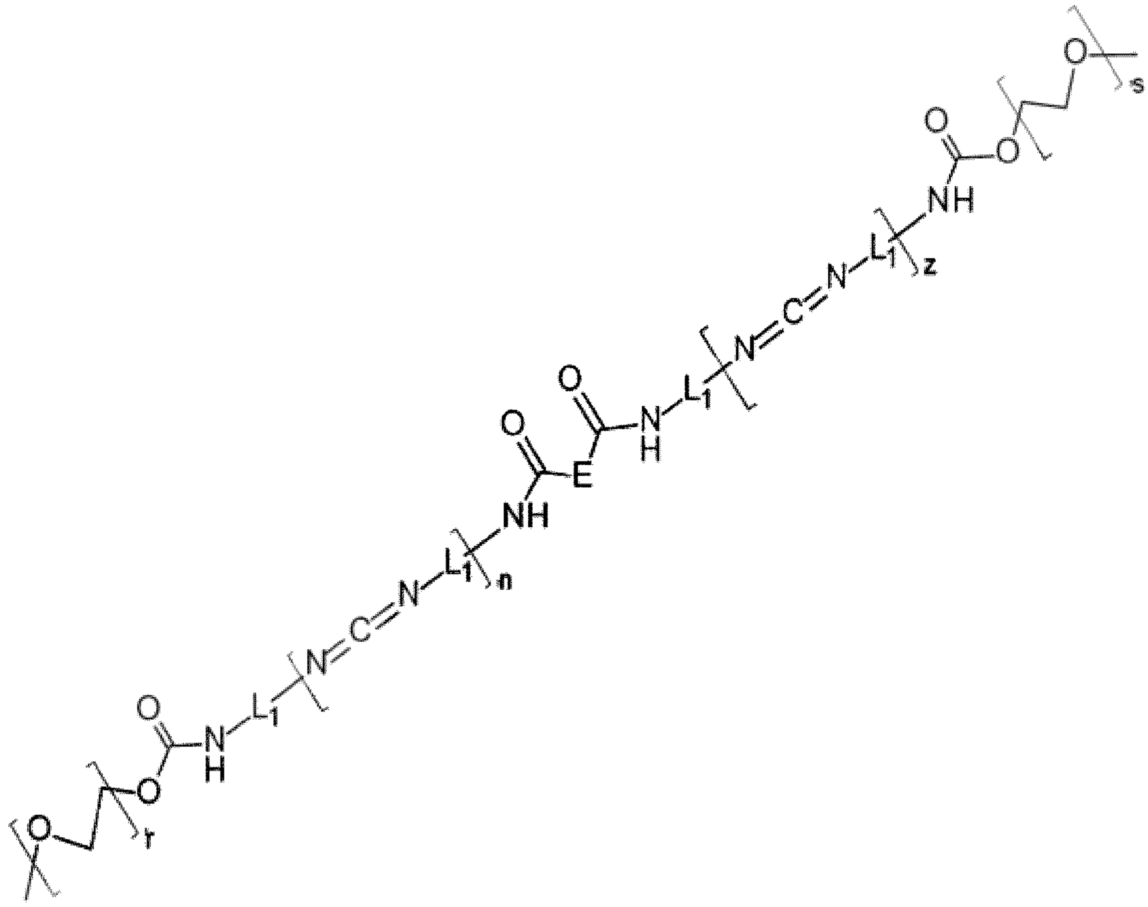

(XII)

wobei $L_1$ für 4,4-Dicyclohexylenmethan steht, n und z eine ganze Zahl im Bereich von 1 bis 20 mit n+z im Bereich von 4 bis 10 bedeuten, E für eine Gruppe -O-$R_3$-O- steht, wobei $R_3$ für einen geradkettigen oder verzweigten $C_1$-$C_{18}$-Alkylenrest wie Methylen, Propylen, Butylen oder Ethylen, der gegebenenfalls durch ein oder mehrere Heteroatome unterbrochen ist, steht und r und s eine ganze Zahl im Bereich von 4 bis 30 bedeuten.

**9.** Verfahren nach einem der vorhergehenden Ansprüche, **dadurch gekennzeichnet, dass** die Gesamtmenge der (Poly)carbodiimidverbindung(en) im Bereich von 0,01 bis 40 Gew.-%, vorzugsweise von 0,1 bis 30 Gew.-%, noch besser von 0,5 bis 25 Gew.-% und sogar noch besser von 1 bis 10 Gew.-%, bezogen auf das Gesamtgewicht von Zusammensetzung (C), liegt.

**10.** Verfahren nach einem der vorhergehenden Ansprüche, **dadurch gekennzeichnet, dass** die wässrige(n) Dispersion(en) von Polymerpartikeln aus wässrigen Dispersionen von Acrylpolymerpartikeln und vorzugsweise aus wässrigen Dispersionen von filmbildendem Acrylpolymer ausgewählt ist/sind.

**11.** Verfahren nach einem der vorhergehenden Ansprüche, **dadurch gekennzeichnet, dass** das Acrylpolymer bzw. die Acrylpolymere eine oder mehrere Einheiten umfassen, die sich von den folgenden Monomeren ableiten:

a) (Meth)acrylsäure und
b) $C_1$- bis $C_{30}$-, weiter bevorzugt $C_1$- bis $C_{20}$-, noch besser $C_1$- bis $C_{10}$- und noch spezieller $C_1$- bis $C_4$-Alkyl(meth) acrylate.

**12.** Verfahren nach einem der vorhergehenden Ansprüche, **dadurch gekennzeichnet, dass** die Gesamtmenge der in der Zusammensetzung vorhandenen wässrigen Dispersion(en) der Polymerpartikel im Bereich von 0,1 bis 40 Gew.-%, weiter bevorzugt von 0,1 bis 35 Gew.-% und noch besser von 0,2 bis 30 Gew.-%, bezogen auf das Gesamtgewicht von Zusammensetzung (C), liegt.

**13.** Verfahren nach einem der vorhergehenden Ansprüche, **dadurch gekennzeichnet, dass** das Silikon bzw. die Silikone aus Nicht-Aminosilikonen, Aminosilikonen und Mischungen davon ausgewählt ist bzw. sind.

**14.** Verfahren nach einem der vorhergehenden Ansprüche, **dadurch gekennzeichnet, dass** das Silikon bzw. die Silikone in einer Gesamtmenge im Bereich von 0,01 bis 20 Gew.-%, bezogen auf das Gesamtgewicht der Zusammensetzung, vorzugsweise von 0,05 bis 15 Gew.-%, weiter bevorzugt von 0,1 bis 10 Gew.-% und noch weiter bevorzugt von 0,1 bis 5 Gew.-%, bezogen auf das Gesamtgewicht der Zusammensetzung (C), vorliegt bzw. vorliegen.

**15.** Verfahren nach einem der vorhergehenden Ansprüche, **dadurch gekennzeichnet, dass** es außerdem einen Schritt des Aufbringens einer Zusammensetzung (D), die mindestens eine Silikonverbindung mit mindestens einer Carboxylgruppe umfasst, auf das Haar umfasst.

**16.** Verfahren nach Anspruch 15, **dadurch gekennzeichnet, dass** die Silikonverbindung(en) mindestens eine aus den Organosiloxanen der nachstehenden Formel (XXVII) ausgewählte Carbonsäuregruppe umfassen:

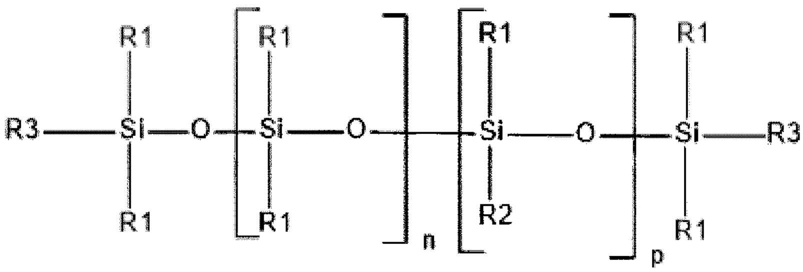

(XXVII)

wobei:

- R1 unabhängig für eine Alkylgruppe mit 1 bis 20 Kohlenstoffatomen, vorzugsweise 1 bis 10 Kohlenstoffatomen, eine Hydroxylgruppe, eine Alkoxygruppe mit 1 bis 20 Kohlenstoffatomen oder eine Arylgruppe mit 6 bis 12 Kohlenstoffatomen steht,
- R2 unabhängig für eine Gruppe R4-COOM, wobei R4 für eine geradkettige oder verzweigte Alkylengruppe mit 1 bis 20 Kohlenstoffatomen, vorzugsweise 4 bis 16 Kohlenstoffatomen, und M für ein Wasserstoffatom, ein Alkalimetall oder Erdalkalimetall oder ein quartäres Ammonium NR'3 steht, wobei R', das gleich oder verschieden sein kann, für H oder Alkyl mit 1 bis 4 Kohlenstoffatomen steht, einen Pyrrolidonrest, der eine Carboxylgruppe COOH umfasst, oder eine Gruppe $R_a$-$(OR_b)_x$-COOM, wobei $R_a$ für eine geradkettige oder verzweigte Alkylengruppe mit 1 bis 4 Kohlenstoffatomen steht, $R_b$ für eine Alkylgruppe mit 1 bis 4 Kohlenstoffatomen steht, wobei x für eine ganze Zahl im Bereich von 1 bis 200 steht, und M für ein Wasserstoffatom, ein Alkali- oder Erdalkalimetall oder ein quartäres Ammonium $NR'_3$ steht, wobei R', die gleich oder verschieden sein können, für H oder Alkyl mit 1 bis 4 Kohlenstoffatomen steht, steht,
- R3 unabhängig für eine Alkylgruppe mit 1 bis 20 Kohlenstoffatomen, eine Hydroxylgruppe, eine Gruppe R4-COOM, wobei R4 für eine geradkettige oder verzweigte Alkylengruppe mit 1 bis 20 Kohlenstoffatomen, vorzugsweise 4 bis 16 Kohlenstoffatomen, und M für ein Wasserstoffatom, ein Alkalimetall oder Erdalkalimetall oder ein quartäres Ammonium NR'3 steht, wobei R', das gleich oder verschieden sein kann, für H oder Alkyl mit 1 bis 4 Kohlenstoffatomen steht, eine Alkoxygruppe mit 1 bis 20 Kohlenstoffatomen, eine Arylgruppe mit 6 bis 12 Kohlenstoffatomen oder eine Gruppe $R_a$-$(OR_b)_x$-COOM, wobei $R_a$ für eine geradkettige oder verzweigte Alkylengruppe mit 1 bis 4 Kohlenstoffatomen steht, $R_b$ für eine Alkylgruppe mit 1 bis 4 Kohlenstoffatomen steht, wobei x für eine ganze Zahl im Bereich von 1 bis 200 steht und M für ein Wasserstoffatom, ein Alkali- oder Erdalkalimetall oder ein quartäres Ammonium $NR'_3$ steht, wobei R', die gleich oder verschieden sein können, für H oder Alkyl mit 1 bis 4 Kohlenstoffatomen steht, steht,
- n eine ganze Zahl im Bereich von 1 bis 1000 bedeutet,
- p eine ganze Zahl im Bereich von 0 bis 1000 bedeutet, wobei es sich versteht, dass mindestens einer der Reste R2 und/oder R3 eine Carbonsäuregruppe COOH oder COOM umfasst, wobei M für ein Alkali- oder Erdalkalimetall oder ein quartäres Ammonium $NR'_3$ steht, wobei R', das gleich oder verschieden sein kann, für H oder ein Alkyl mit 1 bis 4 Kohlenstoffatomen steht.

**17.** Verfahren nach einem der Ansprüche 15 oder 16, **dadurch gekennzeichnet, dass** die Silikonverbindung(en) mindestens eine aus den Organosiloxanen der nachstehenden Formel (XXVIII) ausgewählte Carbonsäuregruppe umfassen:

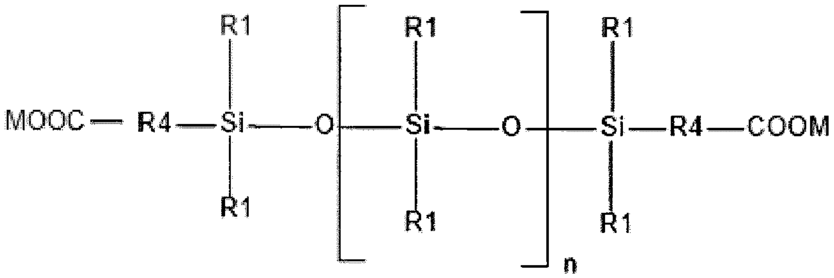

(XXVIII)

wobei:

- R1 unabhängig für eine geradkettige oder verzweigte Alkylgruppe mit 1 bis 20 Kohlenstoffatomen, vorzugsweise 1 bis 10 Kohlenstoffatomen und noch besser 1 bis 6 Kohlenstoffatomen, vorzugsweise Methyl, steht,
- R4 unabhängig für eine geradkettige oder verzweigte Alkylengruppe mit 1 bis 20 Kohlenstoffatomen, vorzugsweise 4 bis 16 Kohlenstoffatomen, oder für eine zweiwertige Gruppe $R_a$-$(OR_b)_x$-, wobei $R_a$ für eine geradkettige oder verzweigte Alkylengruppe mit 1 bis 4 Kohlenstoffatomen steht, $R_b$ für eine Alkylengruppe mit 1 bis 4 Kohlenstoffatomen steht und x für eine ganze Zahl im Bereich von 1 bis 200 steht, steht,
- M unabhängig für ein Wasserstoffatom, ein Alkali- oder Erdalkalimetall oder ein quartäres Ammonium $NR'_3$ steht, wobei R', das gleich oder verschieden sein kann, für H oder Alkyl mit 1 bis 4 Kohlenstoffatomen steht,
- n eine ganze Zahl im Bereich von 1 bis 1000 bedeutet.

**18.** Verfahren nach einem der Ansprüche 15 oder 16, **dadurch gekennzeichnet, dass** die Silikonverbindung(en) mindestens eine aus den Organosiloxanen der nachstehenden Formel (XXIX) ausgewählte Carbonsäuregruppe umfassen:

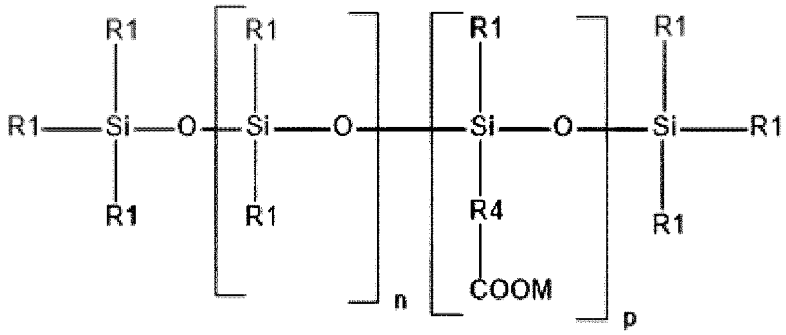

(XXIX)

wobei:

- R1 unabhängig für eine Alkylgruppe mit 1 bis 10 Kohlenstoffatomen, vorzugsweise 1 bis 6 Kohlenstoffatomen, besonders bevorzugt Methyl steht,
- R4 für eine gesättigte oder ungesättigte, geradkettige oder verzweigte Alkylengruppe mit 1 bis 20 Kohlenstoffatomen, vorzugsweise 4 bis 16 Kohlenstoffatomen, oder für eine zweiwertige Gruppe $R_a$-$(OR_b)_x$-, wobei $R_a$ für eine geradkettige oder verzweigte Alkylengruppe mit 1 bis 4 Kohlenstoffatomen steht, $R_b$ für eine Alkylengruppe mit 1 bis 4 Kohlenstoffatomen steht und x für eine ganze Zahl im Bereich von 1 bis 200 steht, steht,
- M für ein Wasserstoffatom, ein Alkali- oder Erdalkalimetall oder ein quartäres Ammonium $NR'_3$ steht, wobei R', das gleich oder verschieden sein kann, für H oder Alkyl mit 1 bis 4 Kohlenstoffatomen steht,
- p eine ganze Zahl im Bereich von 1 bis 1000 bedeutet,
- n eine ganze Zahl im Bereich von 1 bis 1000 bedeutet.

**19.** Verfahren nach einem der Ansprüche 15 bis 18, **dadurch gekennzeichnet, dass** die Gesamtmenge der Silikonverbindung bzw. Silikonverbindungen mit mindestens einer Carboxylgruppe, die in Zusammensetzung D vorliegt bzw. vorliegen, im Bereich von 0,01 bis 20 Gew.-%, besonders bevorzugt von 0,1 bis 15 Gew.-% und noch besser von 0,5 bis 10 Gew.-%, bezogen auf das Gesamtgewicht der Zusammensetzung (D), liegt.

**20.** Verfahren nach einem der Ansprüche 15 bis 19, **dadurch gekennzeichnet, dass** die Zusammensetzung (D) ein oder mehrere Öle enthält, das bzw. die vorzugsweise aus $C_8$-$C_{16}$-Alkanen, weiter bevorzugt aus Isododecan, Isohexadecan, Tetradecan und/oder Mischungen davon, ausgewählt ist bzw. sind.

**21.** Verfahren nach einem der Ansprüche 15 bis 20, **dadurch gekennzeichnet, dass** man die Zusammensetzung (C) und die Zusammensetzung (D) gleichzeitig auf das Haar aufbringt.

**22.** Verfahren nach einem der Ansprüche 15 bis 20, **dadurch gekennzeichnet, dass** man die Zusammensetzung (D) nach dem Aufbringen der Zusammensetzung (C) auf das Haar auf das Haar aufbringt.

**23.** Vorrichtung zur Behandlung von Haar, umfassend ein oder mehrere Kompartimente, die Folgendes enthalten:

- in einem ersten Kompartiment (E1) eine Zusammensetzung (C) gemäß einem der Ansprüche 1 bis 14 und
- gegebenenfalls in einem zweiten Kompartiment (E2) eine Zusammensetzung (D) gemäß einem der Ansprüche 15 bis 20.

**24.** Vorrichtung zur Behandlung von Haar, umfassend mindestens zwei Kompartimente, die Folgendes enthalten:

- in einem ersten Kompartiment (F1) eine Zusammensetzung (C1), die a) mindestens eine (Poly)carbodiimidverbindung gemäß einem der Ansprüche 1 bis 8 umfasst, und
- in einem zweiten Kompartiment (F2) eine Zusammensetzung (C2), die mindestens eine wässrige Dispersion von aus Polyurethanen, Acrylpolymeren und Mischungen davon ausgewählten Polymerpartikeln, wie in einem der Ansprüche 10 oder 11 definiert, umfasst, wobei Zusammensetzung (C1) und/oder Zusammensetzung (C2) mindestens ein aus Pigmenten, Direktfarbstoffen und Mischungen davon ausgewähltes Farbmittel umfasst/umfassen und mindestens ein Silikon gemäß Anspruch 13 umfasst/umfassen, und
- gegebenenfalls in einem dritten Kompartiment (F3) eine Zusammensetzung (D) gemäß einem der Ansprüche 15 bis 20.

## Revendications

**1.** Procédé de coloration des cheveux, comprenant l'application sur les cheveux d'une composition (C) comprenant :

a) au moins un composé (poly)carbodiimide,
b) au moins une dispersion aqueuse de particules de polymère(s) choisi(s) parmi les polyuréthanes, les polymères acryliques, et leurs mélanges,
c) au moins une silicone, et
d) au moins un agent colorant choisi parmi les pigments, les colorants directs, et leurs mélanges.

**2.** Procédé selon la revendication 1, **caractérisé en ce que** le ou les composé(s) (poly)carbodiimide sont choisis parmi les composés de formule (I) suivante :

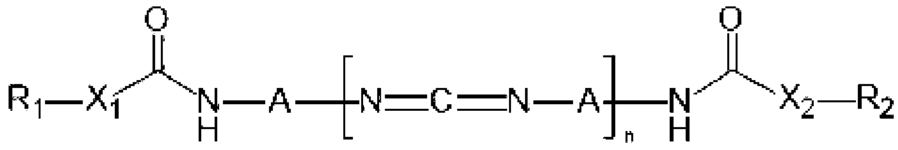

(I)

dans laquelle :

- **$X_1$** et **$X_2$** représentent, indépendamment, un atome d'oxygène O, un atome de soufre S ou un groupement NH ,
- **$R_1$** et **$R_2$** représentent indépendamment un radical hydrocarboné, de préférence alkyle, éventuellement interrompu par un ou plusieurs hétéroatome(s),
- **n** désigne un nombre entier allant de 1 à 100, et
- A est un monomère choisi parmi les composés ci-dessous :

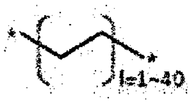
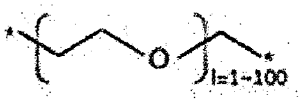
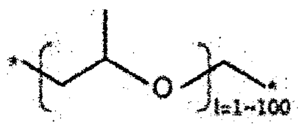
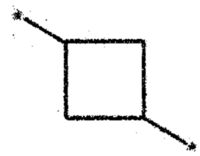

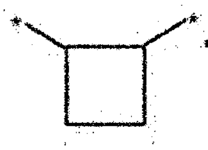
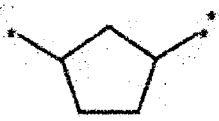
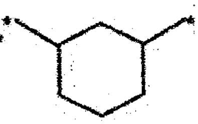
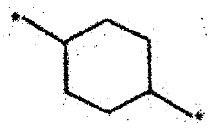
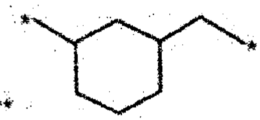

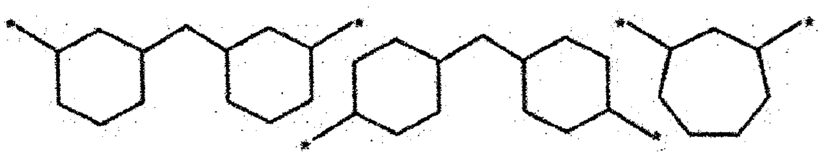
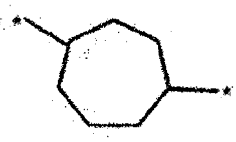

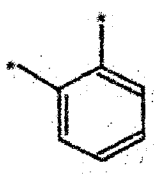
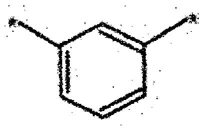
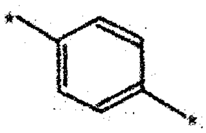
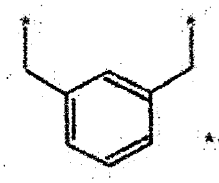
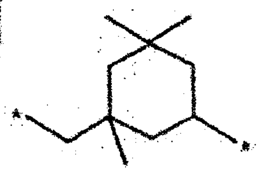

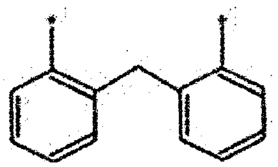

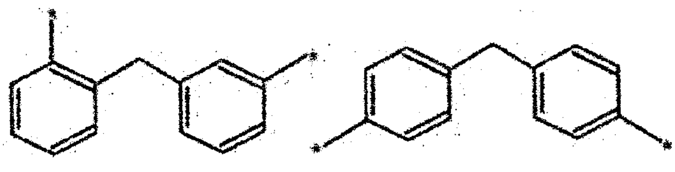

3. Procédé selon la revendication 1, **caractérisé en ce que** le ou les composé(s) (poly)carbodiimide sont choisis parmi les composés de formule (II) suivante :

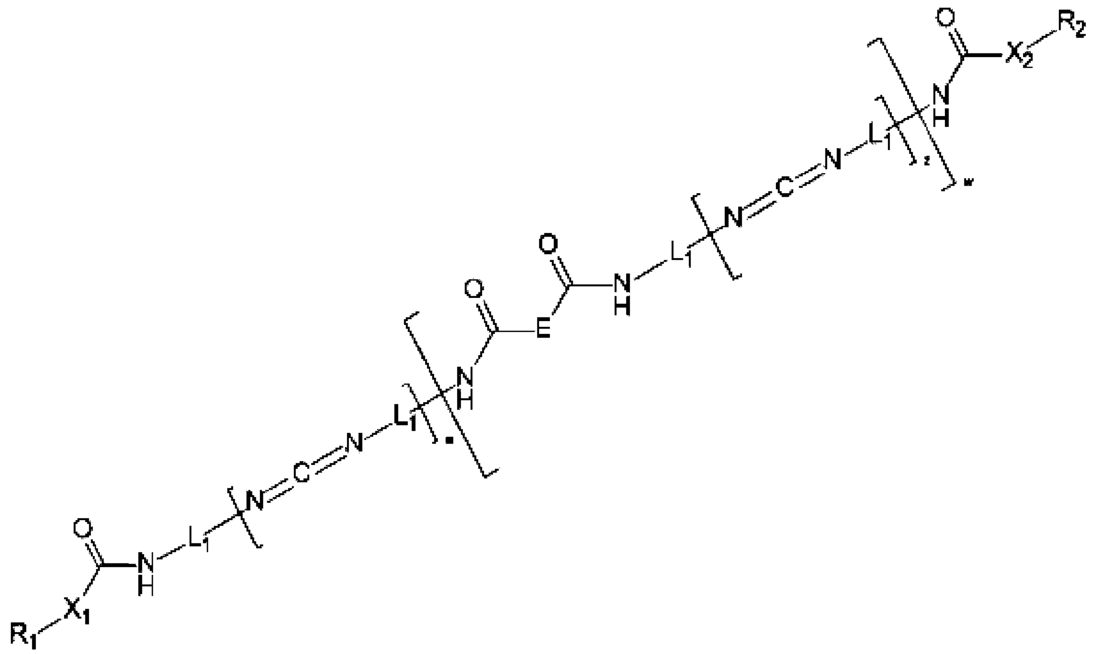

(II)

formule (II) dans laquelle :

- **$X_1$** et **$X_2$** représentent, indépendamment, un atome d'oxygène O, un atome de soufre S ou un atome d'azote NH ;
- **$R_1$** et **$R_2$** représentent indépendamment un radical hydrocarboné éventuellement interrompu par un ou plusieurs hétéroatome(s),
- **n** et **z** désignent un nombre entier allant de 1 à 20, avec n+z ≥ 2 et w désigne un nombre entier allant de 1 à 3 ;
- **$L_1$** représente indépendamment un radical hydrocarboné aliphatique divalent en $C_1$-$C_{18}$, un radical cycloalkylène en $C_3$-$C_{15}$, un groupement hétérocycloalkylène en $C_3$-$C_{12}$, ou un groupement arylène en $C_6$-$C_{14}$, et leurs mélanges ;
- E représente indépendamment un groupement choisi parmi :
-

$$-O-R_3-O-;\ -S-R_4-S-;\ -R_5-N(R_6)-R_4-N(R_6)-R_5-;$$

dans lequel **$R_3$** et **$R_4$** représentent indépendamment un radical hydrocarboné divalent éventuellement interrompu par un ou plusieurs hétéroatome(s) ;
- **$R_5$** représente indépendamment une liaison covalente ou un radical hydrocarboné saturé divalent, éventuellement interrompu par un ou plusieurs hétéroatome(s) ;
- **$R_6$** représente indépendamment un atome d'hydrogène, ou un radical hydrocarboné éventuellement interrompu par un ou plusieurs hétéroatome(s).

4. Procédé selon la revendication 3, **caractérisé en ce que** le ou les composé(s) (poly)carbodiimide sont choisis parmi les composés de formule (II) dans laquelle :

- **$X_1$** et **$X_2$** représentent indépendamment un atome d'oxygène ;

- **$R_1$** et **$R_2$**, sont choisis indépendamment parmi les dialkylaminoalcools, les esters alkyliques d'acide hydroxycarboxylique et les éthers monoalkyliques de (poly)alkylèneglycol, dans lesquels un groupement hydroxy a été retiré, et leurs mélanges ;
- **n** et **z** désignent un nombre entier allant de 1 à 20, avec $n+z \geq 2$ et w est égal à 1 ;
- **$L_1$** est choisi parmi un radical hydrocarboné aliphatique divalent en $C_1$-$C_{18}$, un radical cycloalkylène en $C_3$-$C_{15}$, un groupement hétérocycloalkylène en $C_3$-$C_{12}$, ou un groupement arylène en $C_6$-$C_{14}$, et leurs mélanges ;
- **E** représente indépendamment un groupement choisi parmi :

$$- -O-R_3-O-; -S-R_4-S-; -R_5-N(R_6)-R_4-N(R_6)-R_5-;$$

dans lequel **$R_3$** et **$R_4$** sont choisis indépendamment parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges ;
- lorsque **$R_5$** n'est pas une liaison covalente, **$R_5$** est choisi parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges ; et
- **$R_6$** est choisi parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges.

**5.** Procédé selon l'une quelconque des revendications 3 ou 4, **caractérisé en ce que** le ou les composé(s) (poly) carbodiimide sont choisis parmi les composés de formule (II) dans laquelle :

- **$X_1$** et **$X_2$** représentent indépendamment un atome d'oxygène ;
- **$R_1$** et **$R_2$**, sont indépendamment des éthers monoalkyliques de (poly)alkylèneglycol, dans lesquels un groupement hydroxy a été retiré;
- **n** et **z** désignent un nombre entier allant de 1 à 20, avec $n+z \geq 2$ et w est égal à 1 ;
- **$L_1$** est un radical cycloalkylène en $C_3$-$C_{15}$;
- **E** représente indépendamment un groupement choisi parmi :

$$- -O-R_3-O-; -S-R_4-S-; -R_5-N(R_6)-R_4-N(R_6)-R_5-;$$

dans lequel **$R_3$** et **$R_4$** sont choisis indépendamment parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges ;
- lorsque **$R_3$** n'est pas une liaison covalente, **$R_5$** est choisi parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges ; et
- **$R_6$** est choisi parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges.

**6.** Procédé selon l'une quelconque des revendications 3 à 5, **caractérisé en ce que** le ou les composé(s) (poly) carbodiimide sont choisis parmi les composés de formule (II) dans laquelle :

- **$X_1$** et **$X_2$** représentent indépendamment un atome d'oxygène ;
- **$R_1$** et **$R_2$**, représentent indépendamment le composé de formule (VI) suivante :

$$R_{13}\text{-}[O\text{-}CH_2\text{-}C(H)(R_{14})]_q\text{-} \qquad \text{(VI)}$$

dans laquelle **$R_{13}$** représente un groupe alkyle en $C_1$-$C_4$ ou un phényle, de préférence un groupe alkyle en $C_1$-$C_4$, plus préférentiellement un méthyl, **$R_{14}$** représente un atome d'hydrogène ou un groupe alkyl en $C_1$-$C_4$, de préférence un atome d'hydrogène et **q** désigne un nombre entier allant de 4 à 30 ;
- **n** et **z** désignent un nombre entier allant de 2 à 20, avec n+z allant de 4 à 10 et w est égal à 1;
- **$L_1$** est un radical cycloalkylène en $C_3$-$C_{15}$ tel que cyclopentylène, cycloheptylène, cyclohexylène et le 4,4-dicyclohexylène méthane, et
- **E** représente un groupement -O-$R_3$-O- dans lequel **$R_3$** est choisi parmi un radical arylène en $C_6$-$C_{14}$, un radical cycloalkylène en $C_3$-$C_{12}$, un radical alkylène linéaire ou ramifié en $C_1$-$C_{18}$, éventuellement interrompu par un ou plusieurs hétéroatome(s), et leurs mélanges .

**7.** Procédé selon l'une quelconque des revendications 3 à 6, **caractérisé en ce que** le ou les composé(s) (poly) carbodiimide sont choisis parmi les composés de formule (II) dans laquelle :

- $\mathbf{X_1}$ et $\mathbf{X_2}$ représentent indépendamment un atome d'oxygène ;
- $\mathbf{R_1}$ et $\mathbf{R_2}$, représentent indépendamment le composé de formule (VI) suivante :

$$R_{13}\text{-}[O\text{-}CH_2\text{-}C(H)(R_{14})]_q\text{-} \qquad (VI)$$

dans laquelle $\mathbf{R_{13}}$ représente un groupe alkyle en $C_1$-$C_4$ ou un phényle, de préférence un groupe alkyle en $C_1$-$C_4$, plus préférentiellement un méthyl, $R_{14}$ représente un atome d'hydrogène ou un groupe alkyl en $C_1$-$C_4$, de préférence un atome d'hydrogène et **q** désigne un nombre entier allant de 4 à 30 ;
- **n** et **z** désignent un nombre entier allant de 1 à 20, avec n+z allant de 4 à 10 et w est égal à 1;
- $\mathbf{L_1}$ est un radical cycloalkylène en $C_3$-$C_{15}$ tel que le cyclopentylène, le cycloheptylène, le cyclohexylène et le 4,4-dicyclohexylène méthane, de préférence le 4,4-dicyclohexylène méthane; et
- E représente un groupement -O-$R_3$-O- dans lequel $\mathbf{R_3}$ représente un radical alkylène linéaire ou ramifié en $C_1$-$C_{18}$ tel que le méthylène, propylène, butylène, éthylène, éventuellement interrompu par un ou plusieurs hétéroatome(s).

**8.** Procédé selon l'une quelconque des revendications 3 à 7, **caractérisé en ce que** le ou les composé(s) (poly) carbodiimide sont choisis parmi les composés de formule (XII) suivante :

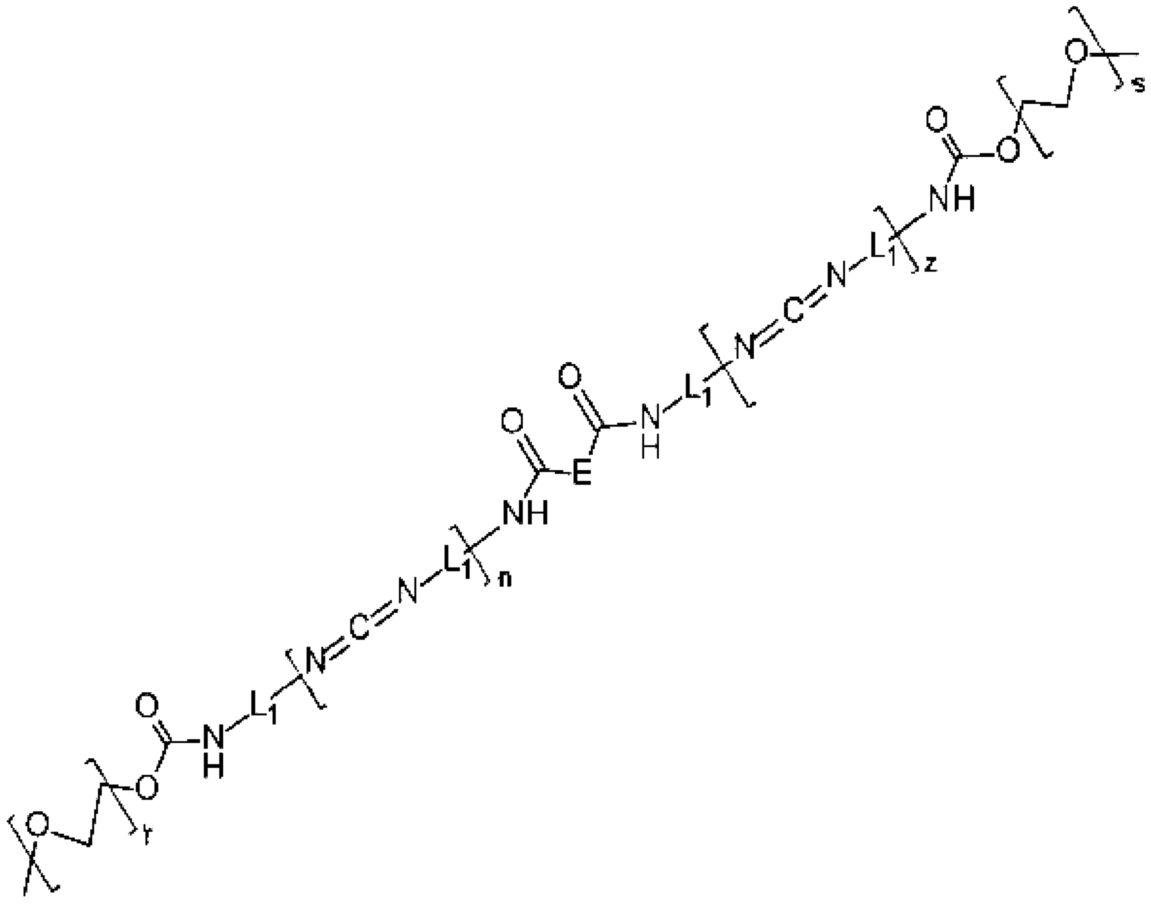

(XII)

dans laquelle $\mathbf{L_1}$ est le 4,4-dicyclohexylène méthane, n et z désignent un nombre entier allant de 1 à 20, avec n+z allant de 4 à 10, E représente un groupement -O-$R_3$-O- dans lequel $\mathbf{R_3}$ représente un radical alkylène linéaire ou ramifié en $C_1$-$C_{18}$ tel que méthylène, propylène, butylène éthylène, éventuellement interrompu par un ou plusieurs hétéroatome(s), et r et s désignent un nombre entier allant de 4 à 30.

**9.** Procédé selon l'une quelconque des revendications précédentes, **caractérisé en ce que** la quantité totale du ou des composé(s) (poly)carbodiimide va de 0,01% à 40% en poids, de préférence de 0,1% à 30% en poids, mieux de 0,5% à 25% en poids, et mieux encore de 1% à 10% en poids, par rapport au poids total de la composition (C).

**10.** Procédé selon l'une quelconque des revendications précédentes, **caractérisé en ce que** le ou les dispersions aqueuses de particules de polymère(s) sont choisies parmi les dispersions aqueuses de particules de polymères acryliques, de préférence parmi les dispersions aqueuses de particules de polymères acryliques filmogènes.

**11.** Procédé selon l'une quelconque des revendications précédentes, **caractérisé en ce que** le ou les polymères acryliques comprennent un ou plusieurs motifs issus des monomères suivants :

a) acide (méth)acrylique; et
b) (méth)acrylate d'alkyle en $C_1$ à $C_{30}$, plus préférentiellement en $C_1$ à $C_{20}$, encore mieux en $C_1$ à $C_{10}$, et encore plus particulièrement en $C_1$ à $C_4$.

**12.** Procédé selon l'une quelconque des revendications précédentes, **caractérisé en ce que** la quantité totale de la ou des dispersions aqueuses de particules de polymères, présente(s) dans la composition, va de 0,1% à 40% en poids, plus préférentiellement de 0,1% à 35% en poids, et mieux encore de 0,2% à 30% en poids, par rapport au poids total de la composition (C).

**13.** Procédé selon l'une quelconque des revendications précédentes, **caractérisé en ce que** la ou les silicones sont choisies parmi les silicones non-aminées, les silicones aminées et leurs mélanges.

**14.** Procédé selon l'une quelconque des revendications précédentes, **caractérisé en ce que** la ou lesdites silicone(s) sont présentes en une quantité totale variant de 0,01% à 20% en poids par rapport au poids total de la composition, de préférence de 0,05% à 15% en poids, plus préférentiellement de 0,1% à 10% en poids, plus préférentiellement de 0,1% à 5% en poids par rapport au poids total de la composition (C).

**15.** Procédé selon l'une quelconque des revendications précédentes, **caractérisé en ce qu'**il comprend en outre une étape d'application sur les cheveux d'une composition (D) comprenant au moins un composé siliconé comprenant au moins un groupement carboxylique.

**16.** Procédé selon la revendication 15, **caractérisé en ce que** le ou les composés siliconés comprenant au moins un groupement carboxylique sont choisis parmi les organosiloxanes de formule (XXVII) suivante :

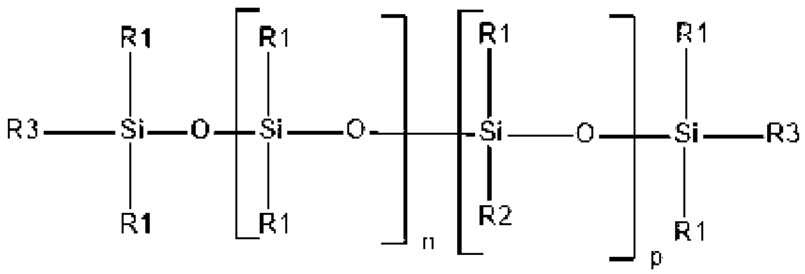

(XXVII)

dans laquelle :

- **R1** représentent indépendamment un groupe alkyle ayant de 1 à 20 atomes de carbone, de préférence de 1 à 10 atomes de carbone ; un groupement hydroxy ; un groupe alcoxy ayant de 1 à 20 atomes de carbone ou un groupe aryle ayant de 6 à 12 atomes de carbone;
- **R2** représente indépendamment un groupe R4-COOM avec **R4** représentant un groupe alkylène linéaire ou ramifié, ayant de 1 à 20 atomes de carbone, de préférence de 4 à 16 atomes de carbone et **M** représentant un atome d'hydrogène ; un métal alcalin ou alcalino-terreux ou un ammonium quaternaire $NR'_3$, avec **R'** identiques ou différents, représentant H ou alkyle ayant de 1 à 4 atomes de carbone ; un radical pyrrolidone comprenant un groupement carboxylique COOH ou un groupe $R_a$-$(OR_b)_x$-COOM avec $\mathbf{R_a}$ représentant un groupe alkylène, linéaire ou ramifié, ayant de 1 à 4 atomes de carbone, $\mathbf{R_b}$ représentant un groupe alkyle ayant de 1 à 4 atomes de carbone, **x** étant un nombre entier allant de 1 à 200; et **M** représentant un atome d'hydrogène ; un métal alcalin ou

alcalino-terreux ou un ammonium quaternaire $NR'_3$, avec **R'** identiques ou différents, représentant H ou alkyle ayant de 1 à 4 atomes de carbone ;

- **R3** représentent indépendamment un groupe alkyle ayant de 1 à 20 atomes de carbone ; un groupement hydroxy ; un groupe R4-COOM avec **R4** représentant un groupe alkylène linéaire ou ramifié, ayant de 1 à 20 atomes de carbone, de préférence de 4 à 16 atomes de carbone et **M** représentant un atome d'hydrogène ; un métal alcalin ou alcalino-terreux ou un ammonium quaternaire $NR'_3$, avec **R'** identiques ou différents, représentant H ou alkyle ayant de 1 à 4 atomes de carbone ; un groupe alcoxy ayant de 1 à 20 atomes de carbone ; un groupe aryle ayant de 6 à 12 atomes de carbone ou un groupe $R_a$-$(OR_b)_x$-COOM avec $\mathbf{R_a}$ représentant un groupe alkylène, linéaire ou ramifié, ayant de 1 à 4 atomes de carbone, $\mathbf{R_b}$ représentant un groupe alkyle ayant de 1 à 4 atomes de carbone, x étant un nombre entier allant de 1 à 200; et **M** représentant un atome d'hydrogène ; un métal alcalin ou alcalino-terreux ou un ammonium quaternaire $NR'_3$, avec **R'** identiques ou différents, représentant H ou alkyle ayant de 1 à 4 atomes de carbone;
- **n** désigne un nombre entier allant de 1 à 1000 ;
- **p** désigné un nombre entier allant de 0 à 1000 ;

étant entendu qu'au moins un des radicaux R2 et/ou R3 comprend un groupement carboxylique COOH ou COOM avec **M** représentant un métal alcalin ou alcalino-terreux ou un ammonium quaternaire $NR'_3$, avec **R'** identiques ou différents, représentant H ou un alkyle ayant de 1 à 4 atomes de carbone .

**17.** Procédé selon l'une quelconque des revendications 15 ou 16, **caractérisé en ce que** le ou les composés siliconés comprenant au moins un groupement carboxylique sont choisis parmi les organosiloxanes de formule (XXVIII) suivante :

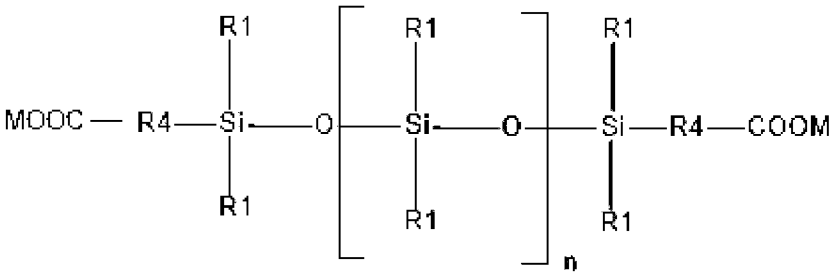

(XXVIII)

dans laquelle :

- **R1** représente indépendamment un groupe alkyl, linéaire ou ramifié, ayant de 1 à 20 atomes de carbone, de préférence de 1 à 10 atomes de carbone encore mieux de 1 à 6 atomes de carbone ; préférentiellement méthyl ;
- **R4** représente indépendamment un groupe alkylène linéaire ou ramifié, ayant de 1 à 20 atomes de carbone, de préférence de 4 à 16 atomes de carbone ; ou bien un groupe divalent $R_a$-$(OR_b)_x$- avec $\mathbf{R_a}$ représentant un groupe alkylène, linéaire ou ramifié, ayant de 1 à 4 atomes de carbone, $\mathbf{R_b}$ représentant un groupe alkylène ayant de 1 à 4 atomes de carbone, et x étant un nombre entier allant de 1 à 200;
- **M** représente indépendamment un atome d'hydrogène, un métal alcalin ou alcalino-terreux ou un ammonium quaternaire $NR'_3$, avec **R'** identiques ou différents, représentant H ou un alkyle ayant de 1 à 4 atomes de carbone;
- **n** désigne un nombre entier allant de 1 à 1000.

**18.** Procédé selon l'une quelconque des revendications 15 ou 16, **caractérisé en ce que** le ou les composés siliconés comprenant au moins un groupement carboxylique sont choisis parmi les organosiloxanes de formule (XXIX) suivante :

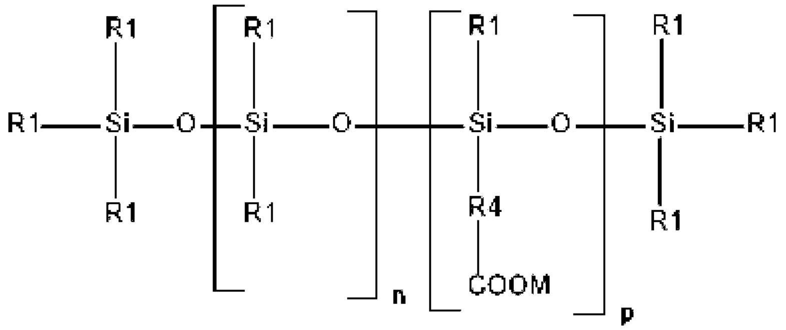

(XXIX)

dans laquelle:

- **R1** représente indépendamment un groupe alkyle ayant de 1 à 10 atomes de carbone, de préférence de 1 à 6 atomes de carbone, plus préférentiellement un méthyl;
- **R4** représente un groupe alkylène linéaire ou ramifié, saturé ou insaturé, ayant de 1 à 20 atomes de carbone, de préférence de 4 à 16 atomes de carbone ; ou bien un groupe divalent $R_a$-$(OR_b)_x$- avec $\mathbf{R_a}$ représentant un groupe alkylène, linéaire ou ramifié, ayant de 1 à 4 atomes de carbone, $\mathbf{R_b}$ représentant un groupe alkylène ayant de 1 à 4 atomes de carbone, et x étant un nombre entier allant de 1 à 200;
- **M** représente un atome d'hydrogène ; un métal alcalin ou alcalino-terreux ou un ammonium quaternaire $NR'_3$, avec **R'** identiques ou différents, représentant H ou un alkyle ayant de 1 à 4 atomes de carbone;
- **p** désigne un entier allant de 1 à 1000 ;
- **n** désigne un entier allant de 1 à 1000 .

**19.** Procédé selon l'une quelconque des revendications 15 à 18, **caractérisé en ce que** la quantité totale du ou des composés siliconés comprenant au moins un groupement carboxylique présents dans la composition (D) va de 0,01% à 20% en poids, plus préférentiellement de 0,1% à 15% en poids, et mieux encore de 0,5% à 10% en poids, par rapport au poids total de la composition (D).

**20.** Procédé selon l'une quelconque des revendications 15 à 19, **caractérisé en ce que** la composition (D) comprend une ou plusieurs huile(s), de préférence choisie(s) parmi les alcanes en $C_8$-$C_{16}$, plus préférentiellement parmi l'isododécane, l'isohexadécane, le tetradécane et/ou leurs mélanges.

**21.** Procédé selon l'une quelconque des revendications 15 à 20, **caractérisé en ce que** la composition (C) et la composition (D) sont appliquées simultanément sur les cheveux.

**22.** Procédé selon l'une quelconque des revendications 15 à 20, **caractérisé en ce que** la composition (D) est appliquée sur les cheveux après l'application de la composition (C) sur les cheveux.

**23.** Dispositif de traitement des cheveux comprenant un ou plusieurs compartiments contenant :

- dans un premier compartiment (E1), une composition (C) telle que définie selon l'une quelconque des revendications 1 à 14; et
- optionnellement, dans un deuxième compartiment (E2), une composition (D) telle que définie selon l'une quelconque des revendications 15 à 20.

**24.** Dispositif de traitement des cheveux comprenant au moins deux compartiments contenant :

- dans un premier compartiment (F1), une composition (C1) comprenant au moins un composé (poly)carbodiimide tel que défini selon l'une quelconque des revendications 1 à 8 et
- dans un deuxième compartiment (F2), une composition (C2) comprenant au moins une dispersion aqueuse de particules de polymère(s) choisi(s) parmi les polyuréthanes, les polymères acryliques, et leurs mélanges telle que définie selon l'une quelconque des revendications 10 ou 11,

la composition (C1) et/ou la composition (C2) comprenant au moins un agent colorant choisi parmi les pigments, les colorants directs, et leurs mélanges, et comprenant au moins une silicone telle que définie selon la revendication 13, et

- optionnellement, dans un troisième compartiment (F3), une composition (D) telle que définie selon l'une quelconque des revendications 15 à 20.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019211050 A1
- US 3412054 A
- US 7445770 B
- US 7452770 B
- US 4957732 A
- EP 186507 A
- EP 342834 A
- US 4185087 A
- EP 0530974 A
- FR 2679771
- EP 1184426 A
- JP 09188830 A
- JP 10158450 A
- JP 10158541 A
- JP 07258460 A
- JP 05017710 A
- US 4578266 A
- WO 9515144 A
- WO 9501772 A
- EP 714954 A
- WO 2008155059 A
- WO 2007068371 A
- US 4284730 A
- CA 2509861


### Non-patent literature cited in the description


- Walter Noll's Chemistry and Technology of Silicones. Academic Press, 1968
- Volatile Silicone Fluids for Cosmetics. Cosmetics and Toiletries. Todd & Byers, vol. 91, 27-32
- *Cosmetics and Toiletries*, February 1990, vol. 105, 53-64
- Sciences of Synthesis - Houben - Weyl Methods of Molecular Transformations. Georg Thiem Verlag Kg, 2005